(19) Europäisches Patentamt
European Patent Office
Office européen des brevets

(11) EP 1 794 292 B1

(12) # EUROPÄISCHE PATENTSCHRIFT

(45) Veröffentlichungstag und Bekanntmachung des Hinweises auf die Patenterteilung:
**30.12.2015 Patentblatt 2015/53**

(21) Anmeldenummer: **05788794.5**

(22) Anmeldetag: **22.09.2005**

(51) Int Cl.:
***C12N 9/28*** *(2006.01)* ***C11D 3/386*** *(2006.01)*

(86) Internationale Anmeldenummer:
**PCT/EP2005/010259**

(87) Internationale Veröffentlichungsnummer:
**WO 2006/037483 (13.04.2006 Gazette 2006/15)**

(54) **GEGEN DI- UND/ODER MULTIMERISIERUNG STABILISIERTE ALPHA-AMYLASE-VARIANTEN, VERFAHREN ZU DEREN HERSTELLUNG SOWIE WASCH- UND REINIGUNGSMITTEL MIT DIESEN ALPHA-AMYLASE-VARIANTEN**

ALPHA-AMYLASE VARIANTS STABILIZED AGAINST DIMERIZATION AND/OR MULTIMERIZATION, METHOD FOR THE PRODUCTION THEREOF, AND DETERGENTS AND CLEANSERS CONTAINING THESE ALPHA-AMYLASE VARIANTS

VARIANTES D'ALPHA-AMYLASE STABILISEES PAR RAPPORT A LA DIMERISATION ET/OU LA MULTIMERISATION, PROCEDE PERMETTANT DE LES PRODUIRE, DETERGENTS ET NETTOYANTS COMPRENANT LESDITES VARIANTES D'ALPHA-AMYLASE

(84) Benannte Vertragsstaaten:
**AT BE BG CH CY CZ DE DK EE ES FI FR GB GR HU IE IS IT LI LT LU LV MC NL PL PT RO SE SI SK TR**

(30) Priorität: **01.10.2004 DE 102004047776**

(43) Veröffentlichungstag der Anmeldung:
**13.06.2007 Patentblatt 2007/24**

(73) Patentinhaber: **BASF SE**
**67056 Ludwigshafen (DE)**

(72) Erfinder:
- **BESSLER, Cornelius**
  **40597 Düsseldorf (DE)**
- **WIELAND, Susanne**
  **41541 Dormagen-Zons (DE)**
- **MAURER, Karl-Heinz**
  **40699 Erkrath (DE)**

(56) Entgegenhaltungen:
**EP-A- 1 199 356** **WO-A-00/22103**
**WO-A-01/66712** **WO-A-02/10356**

EP 1 794 292 B1

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

## Beschreibung

Die vorliegende Erfindung betrifft α-Amylase-Varianten, die durch Punktmutagenese von Oberflächenaminosäuren gegenüber einer über elektrostatische Wechselwirkungen vermittelten Di- und/oder Multimerisierung stabilisiert sind, Verfahren zu deren Herstellung über Mutagenese von Aminosäureresten mit einem Beitrag zum elektrostatischen Potential des Moleküls sowie Wasch- und Reinigungsmittel mit diesen α-Amylase-Varianten.

α-Amylasen (E.C. 3.2.1.1) hydrolysieren interne α-1,4-glycosidische Bindungen von Stärke und stärkeähnlichen Polymeren unter Bildung von Dextrinen und β-1,6-verzweigten Oligosacchariden. Sie gehören zu den technisch wichtigsten Enzymen überhaupt. So werden α-Amylasen beispielsweise bei der Herstellung von Glucosesirup, zur Behandlung von Rohmaterialien in der Textilherstellung, zur Herstellung von Klebstoffen oder zur Herstellung von zuckerhaltigen Lebensmitteln oder Lebensmittelbestandteilen eingesetzt. Ein anderes wichtiges Einsatzgebiet ist die als aktive Komponente in Wasch- und Reinigungsmitteln.

Weil Wasch- und Reinigungsmittel überwiegend alkalische pH-Werte aufweisen, werden hierfür insbesondere α-Amylasen eingesetzt, die im alkalischen Medium aktiv sind. Solche werden von Mikroorganismen, das heißt Pilzen oder Bakterien, vor allem denen der Gattungen *Aspergillus* und *Bacillus* produziert und sekretiert. Ausgehend von diesen natürlichen Enzymen steht inzwischen eine nahezu unüberschaubare Fülle von Varianten zur Verfügung, die über Mutagenese abgeleitet worden sind und je nach Einsatzgebiet spezifische Vorteile aufweisen.

Beispiele hierfür sind die α-Amylasen aus *Bacillus licheniformis,* aus *B. amyloliquefaciens* und aus *B. stearothermophilus* sowie deren für den Einsatz in Wasch- und Reinigungsmitteln verbesserte Weiterentwicklungen. Das Enzym aus *B. licheniformis* ist von der Firma Novozymes unter dem Namen Termamyl® und von der Firma Genencor unter dem Namen Purastar®ST erhältlich. Weiterentwicklungsprodukte dieser α-Amylase sind von der Firma Novozymes unter den Handelsnamen Duramyl® und Termamyl®ultra, von der Firma Genencor unter dem Namen Purastar®OxAm und von der Firma Daiwa Seiko Inc., Tokyo, Japan, als Keistase® erhältlich. Die α-Amylase von *B. amyloliquefaciens* wird von der Firma Novozymes unter dem Namen BAN® vertrieben, und abgeleitete Varianten von der α-Amylase aus *B. stearothermophilus* unter den Namen BSG® und Novamyl®, ebenfalls von der Firma Novozymes.

Beispiele für α-Amylasen aus anderen Organismen sind die unter den Handelsnamen Fungamyl® von der Firma Novozymes erhältlichen Weiterentwicklungen der α-Amylase aus *Aspergillus niger* und A. *oryzae*. Ein weiteres Handelsprodukt ist beispielsweise die Amylase-LT®.

Ferner sei auf die in der Anmeldung WO 02/10356 A2 offenbarte α-Amylase aus *Bacillus sp.* A 7-7 (DSM 12368) und die in der Anmeldung WO 02/44350 A2 beschriebene Cyclodextrin-Glucanotransferase (CGTase) aus *B. agaradherens* (DSM 9948) hingewiesen. Zusätzlich werden beispielsweise in den Anmeldungen WO 03/002711 A2 und WO 03/054177 A2 Sequenzräume von α-Amylasen definiert, die prinzipiell alle für entsprechende Anwendungen geeignet sein könnten.

Punktmutationen zur Verbesserung der Aktivität dieser Enzyme im alkalischen Medium werden beispielsweise in der nicht vorveröffentlichten Anmeldung DE 10309803.8 beschrieben. Dieser Anmeldung zufolge eignen sich hierfür Aminosäureaustausche in den Positionen 13, 32, 194, 197, 203, 230, 297, 356, 406, 414 und/oder 474 gemäß der Zählung der nicht prozessierten α-Amylase aus *B. amyloliquefaciens.* - Das sind in der Zählung der unprozessierten α-Amylase aus *Bacillus sp.* A 7-7 (DSM 12368; WO 02/10356 A2) die Positionen L13, T36, W198. S201, 1208, A235, D302, D361, H408, K416 beziehungsweise N476, mit folgenden besonders wirkungsvollen Austauschen: 13P, 32A, 194R, 197P, 203L, 230V, 297D, 356D, 406R, 414S und 474Q.

Ein weiteres Beispiel zur Punktmutagenese an α-Amylasen ist die Anmeldung WO 00/22103 A1. Darin werden Polypeptide, unter anderem auch α-Amylase-Varianten mit mutagenisierten Oberflächenaminosäuren offenbart. Der Zweck dieser Mutagenese hat darin bestanden, die von diesen Molekülen ausgehende Immunogenizität und/oder Allergenizität zu senken.

Ebenso sind Fusionsprodukte von α-Amylasen für den Einsatz in Wasch- und Reinigungsmitteln beschrieben. So werden beispielsweise in der Anmeldung WO 96/23874 A1 Hybride aus den α-Amylasen aus *Bacillus licheniformis, B. amyloliquefaciens* und *B. stearothermophilus* offenbart. Derartige Hybrid-Amylasen können nach der Lehre dieser Anmeldung zur Ermittlung der dreidimensionalen Struktur dieser Amylasen hergestellt werden, um darüber für die enzymatische Aktivität wichtige Positionen zu erkennen. Diesbezügliche Weiterentwicklungen stellen die Anmeldungen WO 97/41213 A1 und WO 00/60059 A2 dar, aus welchen zahlreiche α-Amylase-Varianten hervorgehen, die jeweils hinsichtlich ihrer Leistung verbessert sind. Spezielle Hybrid-Amylasen aus *B. licheniformis* und *B. amyloliquefaciens* offenbart die Anmeldung WO 03/014358 A2.

Einen weiteren wichtigen Stand der Technik bilden die drei Patentanmeldungen WO96/23873 A1, WO00/60060 A2 und WO01/66712 A2, welche die Grundlage für das Handelsprodukt Stainzyme® der Fa. Novozymes darstellen. All die darin jeweils ausgeführten durch Punktmutagenese erhältlichen Varianten besitzen geänderte enzymatische Eigenschaften und werden somit für den Einsatz in Wasch- und Reinigungsmitteln beansprucht beziehungsweise beschrieben. WO 96/23873 A1 nennt zum Teil jeweils mehrere Punktmutationen in mehr als 30 verschiedenen Positionen in vier verschiedenen Wildtypamylasen. Sie sollen geänderte enzymatische Eigenschaften hinsichtlich der Thermostabilität,

der Oxidationsstabilität und der Calciumabhängigkeit aufweisen. Darunter sind Punktmutationen in folgenden Positionen, die jeweils unter Bezug auf die α-Amylase aus *Bacillus* sp. NCIB 12512 angegeben sind: Ersatz oxidierbarer Aminosäuren in den Positionen M9, M10, M105, M202, M208, M261, M309, M382, M430 oder M440, vorzugsweise M91L; M10L; M105L; M202L,T,F,I,V; M208L; M261L; M309L; M382L; M430L und M440L; Deletionen von F180, R181, G182, T183, G184 und/oder K185; sowie zusätzlich die Austausche K269R; P260E; R124P; M105F,I,L,V; M208F,W,Y; L217I; V206I,L,F; Y243F; K108R; K179R; K239R; K242R; K269R; D163N; D188N; D192N; D199N; D205N; D207N; D209N; E190Q; E194Q oder N106D.

Die Anmeldung WO00/60060 A2 benennt ebenfalls eine Vielzahl an möglichen Aminosäureaustauschen in 10 verschiedenen Positionen, und zwar anhand von zwei sehr ähnlichen α-Amylasen aus zwei verschiedenen Mikroorganismen gleicher Zählung (die α-Amylasen AA349 und AA4560). Das sind folgende Sequenzvariationen: R181*, G182*, D183*, G184*; N195A,R,D,C,E,Q,G,H,I,L,K,M,F,P,S,T,W,Y,V; 1206A,R,D,N,C,E,Q,G,H,L,K,M,F,P,S,T,W,Y,V; E212A, R,D,N,C,Q,G,H,I,L,K,M,F,P,S,T,W,Y,V; E216A,R,D,N,C,Q,G,H,I,L,K,M,F,P,S,T,W,Y,V; K269A,R,D,N, C,E,Q, G,H,I, L, M,F,P,S,T,W,Y,V; und/oder R181A,N,D,C,E,Q,G,H,I,L,K,M,F,P,S,T,W,Y,V. Auch diese Entwicklung steht vor dem Hintergrund, über Mutationen eine Leistungsverbesserung zu erreichen.

WO 01/66712 A2, schließlich, bezeichnet 31 verschiedene, zum Teil mit den zuvor genannten identische Aminosäurepositionen, die in einer der beiden in der Anmeldung WO 00/60060 A2 genannten α-Amylasen mutiert worden sind und sowohl Leistungs- als auch Stabilitätsaspekte verbessern sollen. Das sind Punktmutationen in folgenden Positionen: R28, R118, N174; R181, G182, D183, G184, G186, W189, N195, M202, Y298, N299, K302, S303, N306, R310, N314; R320, H324,E345, Y396, R400, W439, R444, N445, K446, Q449, R458, N471 und N484, wiederum definiert über die α-Amylase AA560, das heißt auch gemäß deren Zählung. Hierunter seien besonders folgende Varianten vorteilhaft: Delta G184; Delta (R181-G182); Delta (D183-G184); R28N,K; S94K; R118K; N125A,R,K; N174D; R181Q,E,K; G186R; W189R,K; N195F; M202L,T; Y298H,F; N299A; K302R, S303Q, N306G,D,R,K; R310A, K,Q,E,H,D,N; N314D; R320K; H324K; E345R,D,K,N; Y396F; R400T,K; W439R; R444K; N445K.Q; K446N; Q449E; R458K; N471E und N484Q.

Ferner wird in dieser zuletzt genannten Anmeldung beschrieben, Polypeptidkristalle, insbesondere solche von Enzymen könnten dadurch hinsichtlich ihres Auflösungsvermögens verbessert werden, daß in den Molekülen, die im betreffenden Kristall neben den eigentlich interessierenden Molekülen liegen und mit diesem interagieren, solche Aminosäuren mutiert werden könnten, die innerhalb eines Abstands von 6,0 A zum interessierenden Polypeptid liegen. Dies gelte insbesondere für Termamyl-ähnliche Enzyme, die in einem Kristall neben anderen oder gleichen Termamyl-ähnlichen Enzymen liegen; hier besonders für einen Abstand von weniger als 3,5 A. Dies beträfe die Positionen 19, 20, 21, 22, 25, 28, 29, 53, 76, 84, 87, 90, 93, 94, 124, 125, 126, 128, 142, 144, 156, 157, 158, 159, 160, 161, 170, 171, 172, 173, 174, 175, 183, 184, 185, 186, 187, 188, 189, 190, 193, 195, 196, 197, 209, 212, 226, 229, 256, 257, 258, 259, 280, 281, 298, 299, 300, 302, 303, 304, 305, 306, 310, 311, 314, 319, 320, 321, 322, 341, 345, 405, 406, 408, 444, 447, 448, 449, 463, 464, 465, 466 und 467; beziehungsweise die Positionen 22, 25, 28, 76, 94, 125, 128, 158, 160, 171, 173, 174, 184, 189, 209, 226, 229, 298, 299, 302, 306, 310, 314, 320, 345, 405, 447 und 466 für den geringeren Abstand.

All diesen Anmeldungen zur Punktmutagenese ist gemeinsam, daß auch der Stabilitätsgesichtspunkt unter dem Aspekt einer guten Leistung im entsprechenden Einsatzgebiet bewertet wird. Denn die während der Lagerung und dem Einsatz der α-Amylasen beispielsweise im Rahmen von Waschmittelrezepturen aufrechterhaltene Stabilität führt zu einer hohen beziehungsweise möglichst gleichbleibend hohen Leistung bei entsprechendem Einsatz. Eine Stabilitätserhöhung, insbesondere gegenüber einer Aggregatbildung, vor allem im Zuge der Aufarbeitung wird nicht beschrieben.

Den Zweck der Stabilitätserhöhung verfolgen zahlreiche Anmeldungen, die spezielle Enzymstabilisatoren beschreiben. Diese zusätzlichen Inhaltsstoffe bewirken, daß ein in entsprechenden Mitteln enthaltenes Protein und/oder Enzym besonders während der Lagerung gegen Schädigungen wie beispielsweise Inaktivierung, Denaturierung oder Zerfall geschützt wird. So bilden reversible Proteaseinhibitoren eine Gruppe von Stabilisatoren. Andere, beispielsweise Polyole, stabilisieren gegenüber physikalischen Einflüssen wie beispielsweise dem Einfrieren. Andere polymere Verbindungen wie Acryl-Polymere und/oder Polyamide stabilisieren die Enzym-Präparation unter anderem gegenüber pH-Wert-Schwankungen. Reduktionsmittel und Antioxidantien erhöhen die Stabilität der Enzyme gegenüber oxidativem Zerfall.

Derartige Verbindungen werden den Enzymen sowohl bei der Anwendung als auch im Zuge ihrer Aufarbeitung zugegeben, was insbesondere dann wichtig ist, wenn in einem Teilschritt der Aufarbeitung, etwa einer Fällung, ein zuvor enthaltener Stabilisator zusammen mit den übrigen Verunreinigungen abgetrennt worden ist.

Der Stand der Technik zur Stabilitätsverbesserung von α-Amylasen läßt sich folgendermaßen zusammenfassen: Es sind über Punktmutagenese eine Vielzahl von α-Amylase-Varianten entwickelt worden, wobei das Ziel dieser Entwicklungen überwiegend darin bestanden hat, diese hinsichtlich ihrer Leistung zu verbessern. In diese Kategorie sind auch jene Varianten zu zählen, die bezüglich denaturierender Agentien wie Bleichmitteln oder Tensiden stabilisiert sind, weil bei ihnen jeweils auf die gewünschte Leistung des Enzyms optimiert wird. Ansonsten wird zur Erhöhung der Stabilität oder der Aufrechterhaltung der physikalisch-chemischen Konformation überwiegend auf zusätzliche Verbindungen zurückgegriffen, die in ihrer Gesamtheit als Stabilisatoren bezeichnet werden.

Ein bislang wenig beachteter Aspekt in der Enzymentwicklung besteht darin, die Moleküle an sich so zu stabilisieren, daß sie bereits während ihrer Aufarbeitung eine gegenüber dem Wildtypmolekül erhöhte Stabilität aufweisen. Eine vorteilhafte zusätzliche Wirkung davon dürfte darin bestehen, daß diese gesteigerte Stabilität auch dem später beabsichtigten Einsatz des betreffenden Enzyms zugute kommen sollte.

Die Notwendigkeit hierfür zeigt sich insbesondere bei α-Amylasen. Zumindest einige davon neigen vor allem während des Produktions- und Aufarbeitungsprozesses dazu, Multimere, und zwar in der Form amorpher Aggregate zu bilden, welche irreversibel präzipitieren. Dadurch gehen die betreffenden Aktivitäten bereits bei der Aufarbeitung verloren. Unter dem Aufarbeitungsprozeß sind alle Schritte der großtechnischen Herstellung zu verstehen, von der Isolierung des betreffenden Enzyms, insbesondere den für die biotechnologische Gewinnung üblichen Fermentationsmedien, über die folgenden Wasch- und Separationsschritte (beispielsweise durch Fällung) und die Konzentrierung bis hin zur Konfektionierung, etwa der Granulation. Kritisch sind hier insbesondere solche Teilschritte, in denen das Enzym in Lösungen mit vergleichsweise hohen Konzentrationen vorliegt, weil es hier statistisch gesehen zu häufigeren Kontakten zwischen den Molekülen als bei niedrigeren Konzentrationen kommt. Die Aggregatbildung kann aber auch bei der Lagerung von α-Amylase-haltigen Mitteln oder während der Anwendung auftreten, etwa bei Einsatz als aktiver Inhaltsstoff in Wasch- oder Reinigungsprozessen.

Diese Problematik kann soweit führen, daß sich einzelne α-Amylasen zwar im Labormaßstab gewinnen und untersuchen lassen, sich jedoch einer großtechnischen Herstellung mithilfe allgemein üblicher Methoden widersetzen. Man spricht dann auch davon, daß diese Enzyme eine niedrige Prozeßstabilität besitzen, womit die verschiedensten Verarbeitungs- und Einsatzmöglichkeiten gemeint sind. Beispielsweise die α-Amylase aus *Bacillus sp.* A 7-7 (DSM 12368) zeigt eine höhere Neigung zur Multimerisierung als die native α-Amylase aus *B. licheniformis.* Ansätze zur Behebung dieser Art der Instabilität, das heißt zur Verringerung der Neigung zur Multimerisierung, würden derartige Enzyme einer großtechnischen Produktion und damit den vielfältigen Einsatzgebieten überhaupt erst in technisch relevanten Mengen zugänglich machen.

Es stellte sich somit die Aufgabe, α-Amylasen, und insbesondere die aus *Bacillus sp.* A 7-7 (DSM 12368) an sich so zu stabilisieren, daß sie eine im Vergleich zum Ausgangsmolekül erhöhte Stabilität gegenüber einer Aggregatbildung aufweisen.

Unter einem Teilaspekt dieser Aufgabe mußte zunächst eine mögliche, in der Struktur der betreffenden Enzyme begründete Ursache für deren Neigung zur Aggregatbildung ermittelt werden. Anschließend galt es, dieser mit geeigneten Strukturveränderungen entgegenzutreten.

Diese Strukturverändemgen würden sich zum einen vorteilhaft auf die Aufarbeitung, das heißt die großtechnische Gewinnung dieser Enzyme auswirken. Zum anderen sollten sie auch für den Einsatz der α-Amylasen, etwa in Wasch- und Reinigungsmitteln vorteilhaft sein, weil damit zusätzlich eine gleichbleibend hohe Aktivität während des Einsatzes einhergehen sollte.

Als besonders vorteilhafte Lösungen dieser Aufgabe wären somit solche α-Amylasen anzusehen, die neben der genannten Stabilität gegenüber einer Aggregatbildung weitere positive Eigenschaften hinsichtlich ihres beabsichtigten Einsatzes, insbesondere hinsichtlich ihres Einsatzes in Wasch- und Reinigungsmitteln zeigen.

Dies galt vor allem für die α-Amylase aus *Bacillus sp.* A 7-7 (DSM 12368). Gleichwohl sind solche Lösungen bevorzugt, die sich auf andere α-Amylasen übertragen lassen.

Als Grund für das insbesondere bei α-Amylasen, insbesondere bei bestimmten α-Amylasen beobachtete Phänomen der Aggregatbildung wurde auf dem Weg zur Lösung der genannten Aufgabe das Vorhandensein von Bereichen mit unterschiedlichem elektrostatischem Potential auf der Oberfläche dieser Moleküle in ihrer nativen, korrekt gefalteten globulären Struktur in Erwägung gezogen. So sind beispielsweise große Bereiche der Oberfläche der α-Amylase aus *Bacillus sp.* A 7-7 (DSM 12368) negativ geladen, während andere zum Teil großflächige Bereiche ein positives oder neutrales elektrostatisches Potential besitzen. Somit kann es über elektrostatische Wechselwirkungen zwischen den unterschiedlich polarisierten oder geladenen Bereichen mehrerer Moleküle zu deren Aneinanderlagerung und damit zu einer Dimerisierung bis hin zur Ausbildung von Multimeren kommen. Diese Wechselwirkung läßt sich durch zugesetzte denaturierende Agentien vermutlich nur unter Beeinträchtigung der globulären Struktur, das heißt unter dem Risiko der irreversiblen Inaktivierung wieder auflösen. Zum anderen kann diese Wechselwirkung an sich, insbesondere wenn die gegenseitigen Anziehungskräfte stark genug sind, um die Struktur des Moleküls zu beeinflussen, auch ohne von außen einzugreifen zu irreversibel inaktiven Aggregaten führen.

Zur Lösung der gestellten Aufgabe wird ein molekularbiologischer Ansatz verfolgt. Denn hierdurch wird auf die durch die bloße Aminosäuresequenz vermittelten enzymatischen Eigenschaften Einfluß genommen. Dabei wurde überraschenderweise festgestellt, daß Modifikationen, das heißt Punktmutationen, die das Ladungsmuster auf der Oberfläche dieser Moleküle einander angleichen, einer Aggregatbildung entgegenwirken.

Welche Aminosäurereste "auf der Oberfläche dieser Moleküle" liegen, kann über die 3D-Struktur des globulären Enzymproteins, über die sogenannte Conolly-Oberfläche oder über den Wert der sogenannten Akzessibilität, das heißt der Lösungsmittelzugänglichkeit (siehe unten) definiert werden. Der beobachtete positive, negative oder neutrale Beitrag

zum elektrostatischen Potential des Moleküls ergibt sich über die chemischen Eigenschaften der jeweiligen Aminosäurereste unter dem Einfluß der jeweils benachbarten, insbesondere unter der Oberfläche liegenden benachbarten Aminosäurereste und kann wie unten dargestellt berechnet werden. Die beobachtete Aggregation scheint insbesondere dann aufzutreten, wenn mehrere auf der Oberfläche unmittelbar benachbarte Reste die gleiche Polarität beziehungsweise Ladung aufweisen.

Ohne an diese Theorie gebunden sein zu wollen, kann man annehmen, daß die Bildung von α-Amylase-Aggregaten zumindest zu einem signifikanten Anteil auf eine Di- und/oder Multimerisierung über unterschiedlich polare und/oder unterschiedliche geladene Oberflächenbereiche dieser Proteinmoleküle zurückzuführen ist. Hierdurch neigen die Moleküle dazu, sich ähnlich wie Magneten, in regelmäßiger Orientierung zueinander auszurichten. Ein Durchbrechen der Flächenhaftigkeit der positiv polarisierten oder geladenen Bereiche zugunsten der vorherrschenden negativen Ladung stabilisiert die α-Amylasen somit gegenüber einer auf Multimerisierung beruhenden Aggregatbildung. Dies kommt sowohl der Gewinnung (etwa bei einem Fällungsschritt) und der Lagerung (etwa in hydrophoben Lösungsmitteln, die eine Aggregation über polare Regionen fördern) als auch dem Einsatz zugute.

Diese Annahme wird dadurch untermauert, daß die α-Amylase aus *B. licheniformis* mit einer vergleichweise geringen Neigung zur Multimerisierung ein weitgehend negatives Ladungspotential auf ihrer Oberfläche aufweist.

Eine neben der Vermeidung der Di- und/oder Multimerisierung weitere vorteilhafte Wirkung besteht darin, daß die Enzyme statistisch betrachtet vermehrt in ihrer nativen Konformation vorliegen und nicht über die genannten elektrostatischen Wechselwirkungen deformiert werden, wodurch sich sonst den denaturierenden Agentien Angriffsflächen auftun. Dieser Effekt erhöht insgesamt ihre Stabilität, beispielsweise gegenüber organischen Lösungsmitteln und Tensiden, welche sich über die hydrophoben, normalerweise dem Lösungsmittel wenig zugänglichen Bereiche anlagern und diese zu solubilisieren versuchen, oder gegenüber Proteasen, die die intern gelegenen Säureamidbindungen des Rückgrats angreifen. Auch intern gelegene oxidierbare Aminosäurereste sind über diesen Effekt besser geschützt, beispielsweise gegen Luftsauerstoff oder Bleichmittel.

Die gestellte Aufgabe wird demzufolge durch α-Amylase-Varianten mit mindestens einem Aminosäureaustausch gegenüber dem Ausgangsmolekül gelöst, wodurch ein auf der Oberfläche des Moleküls gelegener und einen neutralen oder positiv polaren oder geladenen Beitrag zum elektrostatischen Potential des Moleküls ausübender Aminosäurerest des Ausgangsmoleküls zu einem stärker negativ polaren oder negativ geladenen Aminosäurerest ausgetauscht worden ist, wobei folgende Aminosäureaustausche möglich sind:

| Ausgangsaminosäure | zu |
|---|---|
| Arg (R) | K, Y, C, H, G, A, V, L, I, M, F, W, P, S, T, N, Q, E oder D |
| Lys (K) | Y, C, H, G, A, V, L, I, M, F, W, P, S, T, N, Q, E oder D |
| Tyr (Y) | C, H, G, A, V, L, I, M, F, W, P, S, T, N, Q, E oder D |
| Cys (C) | H, G, A, V, L, I, M, F, W, P, S, T, N, Q, E oder D |
| His (H) | G, A, V, L, I, M, F, W, P, S, T, N, Q, E oder D |
| Gly (G) | A, V, L, I, M, F, W, P, S, T, N, Q, E oder D |
| Ala (A) | V, L, I, M, F, W, P, S, T, N, Q, E oder D |
| Val (V) | L, I, M, F, W, P, S, T, N, Q, E oder D |
| Leu (L) | I, M, F, W, P, S, T, N, Q, E oder D |
| Ile (I) | M, F, W, P, S, T, N, Q, E oder D |
| Met (M) | F, W, P, S, T, N, Q, E oder D |
| Phe (F) | W, P, S, T, N, Q, E oder D |
| Trp (W) | P, S, T, N, Q, E oder D |
| Pro (P) | S, T, N, Q, E oder D |
| Ser (S) | T, N, Q, E oder D |
| Thr(T) | N, Q, E oder D |
| Asn (N) | Q, E oder D |
| Gln (Q) | E oder D |
| Glu (E) | D |

und wobei der genannte Aminosäurerest in einer Position liegt, die einer der beiden folgenden Gruppen angehört:

(A) 5, 6, 7, 19, 22, 25, 26, 28, 29, 32, 33, 35, 37, 53, 72, 75, 76, 81, 82, 83, 84, 86, 87, 90, 91, 93, 94, 95, 96, 98, 118, 136, 142, 149, 150, 151, 152, 154, 156, 158, 160, 171, 172, 181, 227, 229, 247, 251, 254, 259, 260, 281, 283, 394, 395, 399, 400, 417, 418, 419, 420, 421 und 422; oder
(B) 435, 436, 439, 444, 445, 449, 450, 452, 458, 459, 460, 461, 463, 465, 466, 471, 473, 475, 476 und 484,

jeweils angegeben in der Zählung des maturen Proteins gemäß SEQ ID NO. 2, wobei es sich bei dem Ausgangsmolekül um die α-Amylase aus *Bacillus sp.* A 7-7 (DSM 12368) handelt, deren Aminosäuresequenz zu der in SEQ ID NO. 2 angegebenen Aminosäuresequenz in den Positionen +1 bis 484 100% identisch ist.

Unter α-Amylasen im Sinne der vorliegenden Anmeldung sind wie eingangs erläutert die Enzyme der Klasse E.C. 3.2.1.1 zu verstehen, die interne α-1,4-glycosidische Bindungen von Stärke und stärkeähnlichen Polymeren unter Bildung von Dextrinen und β-1,6-verzweigten Oligosaccharide hydrolysieren.

α-Amylase-Varianten sind solche α-Amylasen, die durch an sich bekannte gentechnische Modifizierungen aus einem Vorgängermolekül abgleitet worden sind. "Variante" ist auf der Ebene der Proteine der zu "Mutante" entsprechende Begriff auf der Ebene der Nukleinsäuren. Bei den Vorgänger- oder Ausgangsmolekülen kann es sich um Wildtypenzyme handeln, das heißt solche, die aus natürlichen Quellen erhältlich sind. Solche sind einleitend beispielhaft vorgestellt worden. Es kann sich auch um Enzyme handeln, die an sich bereits Varianten darstellen, das heißt gegenüber den Wildtypmolekülen bereits verändert worden sind. Darunter sind beispielsweise Punktmutanten, solche mit Änderungen der Aminosäuresequenz, über mehrere Positionen oder längere zusammenhängende Bereiche, oder auch Hybridmoleküle zu verstehen, die aus einander ergänzenden Abschnitten verschiedener Wildtyp-α-Amylasen zusammengesetzt sind. Sowohl Wildtypenzyme als auch Mutanten und Hybridenzyme sind einleitend beispielhaft vorgestellt worden.

Unter Aminosäureaustauschen sind Substitutionen einer Aminosäure gegen eine andere Aminosäure zu verstehen. Erfindungsgemäß werden solche Substitutionen unter Bezeichnung der Positionen, in der der Austausch erfolgt, gegebenenfalls kombiniert mit den betreffenden Aminosäuren im international gebräuchlichen Einbuchstabencode angegeben. "Austausch in Position 83" bedeutet beispielsweise, daß eine Variante in der Position, die in der Sequenz eines Referenzproteins die Position 83 aufweist, eine andere Aminosäure aufweist. Üblicherweise werden solche Austausche auf der DNA-Ebene über Mutationen einzelner Basenpaare durchgeführt (siehe oben). "N83D" bedeutet beispielsweise, daß das Referenzenzym an der Position 83 die Aminosäure Asparagin aufweist, während die betrachtete Variante an der hiermit homologisierbaren Position über die Aminosäure Asparaginsäure verfügt. "83D" bedeutet, daß jede beliebige, das heißt in der Regel eine natürlicherweise vorgegebene Aminosäure an einer Position, die der Position 83 entspricht, gegen eine Asparaginsäure ersetzt ist; und "N83X" bedeutet, daß die Aminosäure Asparagin in Position 83 gegen eine prinzipiell beliebige andere Aminosäure ersetzt ist.

Grundsätzlich sind die mit der vorliegenden Anmeldung bezeichneten erfindungsgemäßen Aminosäureaustausche nicht darauf beschränkt, daß sie die einzigen Austausche sind, in denen sich die betreffende Variante von dem Wildtypmolekül unterscheidet. Es ist im Stand der Technik bekannt, daß sich die vorteilhaften Eigenschaften einzelner Punktmutationen einander ergänzen können. Eine hinsichtlich bestimmter Eigenschaften wie etwa Calcium-Bindung oder Stabilität gegenüber Tensiden optimierte α-Amylase kann erfindungsgemäß zusätzlich über die hier vorgestellten Substitutionen weiterentwickelt werden. Somit umfassen Ausführungsformen der vorliegenden Erfindung alle Varianten, die neben anderen Austauschen gegenüber dem Wildtypmolekül auch die erfindungsgemäßen Austausche aufweisen.

Als Referenzenzym hinsichtlich der Nummerierung der Positionen ist erfindungsgemäß die α-Amylase aus *Bacillus sp.* A 7-7 (DSM 12368) anzusehen, deren Nukleotidsequenz in SEQ ID NO. 1 angegeben ist, gefolgt von der zugehörigen Aminosäuresequenz in SEQ ID NO. 2. Diese Sequenzangaben sind, wie in Beispiel 1 der vorliegenden Anmeldung erläutert wird, gegenüber der Beschreibung in WO 02/10356 A2 in jeweils zwei Positionen korrigiert und entsprechen in dieser Form nach dem derzeitigen Kenntnisstand exakt den aus dem hinterlegten und in WO 02/10356 A2 beschriebenen Stamm DSM 12368 erhältlichen Sequenzdaten.

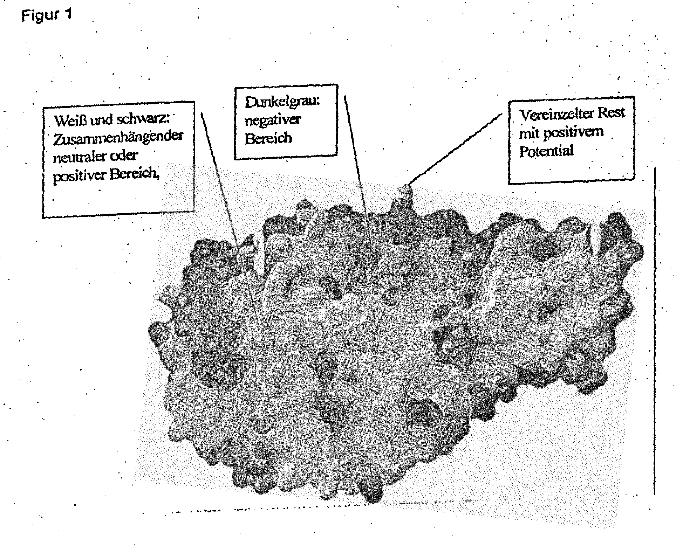

Diese korrekte Aminosäuresequenz geht auch aus der ersten Zeilen des in Figur 1 gezeigten Alignments hervor, welches lediglich für die maturen Teile der jeweiligen Enzyme erstellt worden ist. Das ist dadurch gerechtfertigt, daß *in vivo* allein der mature Teil (das heißt der mit der positiven Zählung in SEQ ID NO. 2) als α-Amylase wirksam wird. Für manche Enzyme wie beispielsweise AA349 und AA560 werden in der Patentliteratur (WO 00/60060 A2) ohnehin lediglich die maturen Sequenzteile angegeben.

Die Oberfläche des Enzyms umfaßt all diejenigen Aminosäuren des nativ gefalteten Enzyms, die dem Lösungsmittel zugewandt sind. Das sind beispielsweise in der α-Amylase aus *Bacillus sp.* A 7-7 (DSM 12368) die 407 Aminosäuren, die im Einzelnen in Beispiel 2 aufgeführt werden.

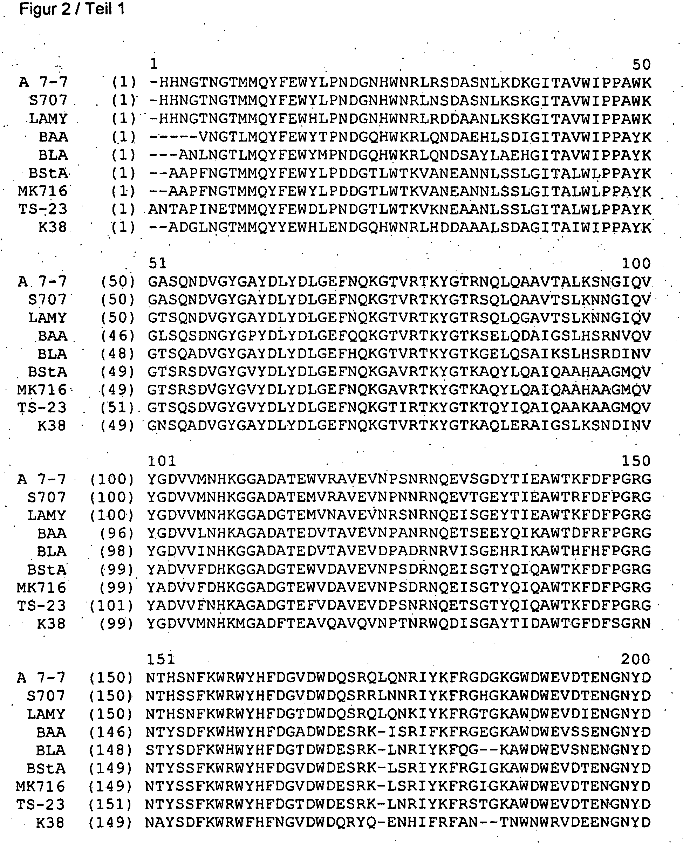

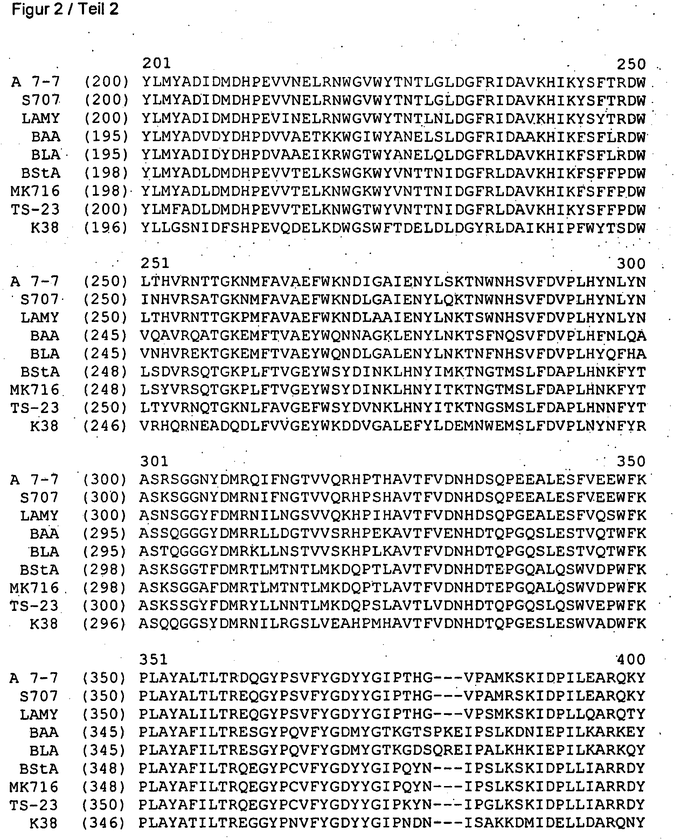

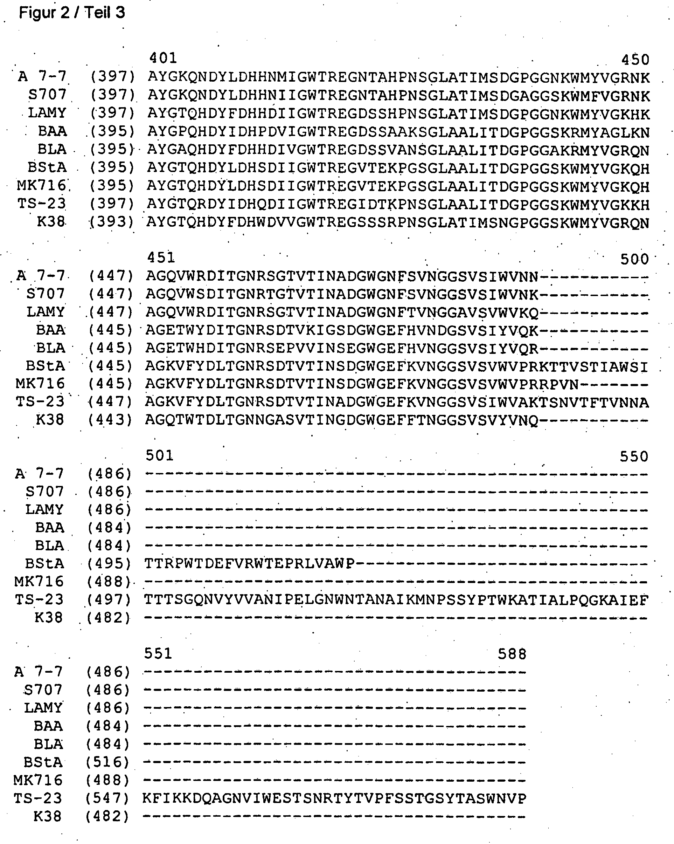

Die Oberflächenaminosäuren anderer α-Amylasen können prinzipiell unter Heranziehen des Alignments der Figur 2 erschlossen werden. Dies gilt jedoch nur näherungsweise. In hochkonservierten und strukturell gesicherten Bereichen wie α-Helices oder β-Faltblättern ergibt sich i.d.R. eine gute Übereinstimmung; in flexibleren Bereichen, insbesondere Loops ist der auf dem Alignment, das heißt der Primärstruktur beruhende Vergleich mit Unsicherheiten

behaftet. Ausschlaggebend ist in allen Fällen (sofern bekannt) die gesicherte Röntgenstruktur, wie sie tatsächlich für die meisten kommerziell wichtigen α-Amylasen inzwischen in allgemein zugänglichen Datenbanken hinterlegt ist. Das sind beispielsweise die Datenbanken GenBank (National Center for Biotechnology Information NCBI, National Institutes of Health, Bethesda, MD, USA) und Swiss-Prot (Geneva Bioinformatics (GeneBio) S.A., Genf, Schweiz; http://www.genebio.com/sprot.html).

Sollte für ein interessierendes Molekül die 3D- (oder Tertiär-)Struktur noch nicht bestimmt worden sein, so kann diese über ein Homologiemodelling erschlossen werden. Bei dieser Methode zur Strukturvorhersage von Proteinen, von denen noch keine Kristallstrukturen gelöst wurden, wird davon ausgegangen, daß Proteine mit ähnlicher Primärstruktur auch eine ähnliche Sekundär- und Tertiärstruktur besitzen. Die Ähnlichkeit zwischen zwei Proteinsequenzen läßt sich über einen geeigneten Algorithmus bestimmen, beispielsweise BLAST, FASTA oder CLUSTAL. Solche Algorithmen stehen über allgemein zugängliche Protein-Datenbanken ebenfalls zur Verfügung; so verfügen beispielsweise GenBank und Swiss-Prot über entsprechende Verknüpfungen. Die RSCB-Protein-Databank (zugänglich über das Max-Delbrück-Zentrum in Berlin, Deutschland, über die Internet-Adresse http://www.pdb.mdc-berlin.de/pdb; 24.8.2004) bietet dem Benutzer die Möglichkeit, über eine FASTA-Suche zu einer bestimmten Sequenz Kristallstrukturen verwandter Proteine zu finden.

Die darauf aufbauenden, beispielsweise mithilfe des sogenannten Swiss-Pdb-Viewer möglichen Berechnungen werden in der Publikation "SWISS-Model and the Swiss-PdbViewer: An environment for comparative protein modeling" (1997) von N. Guex und M. C. Peitsch in Electrophoresis, Band 18, Seiten 2714 bis 2723, beschrieben. Dieser Viewer ist über die Internetadresse http://us.expasy.org/spdbv/ (24.8.2004) der Organisation Swiss Institute of Bioinformatics (Central Administration, Bätiment Ecole de Pharmacieroom 3041, Universite de Lausanne, 1015 Lausanne, Switzerland) kostenlos zugänglich. Das zugehörige Handbuch (SwissPDB-Viewer-Manual) steht unter der Internetadresse http://us.expasy.org/spdbv/text/selmenu.htm (24.8.2004) zur Verfügung. Durch Übereinanderlagerung (Superimposition) dieser Strukturen kann darüber eine Leitstruktur erstellt werden, auf die dann die Proteinsequenz der interessierenden α-Amylase aufmodelliert wird. Die einzelnen Schritte hierfür sind dem genannten Benutzerhandbuch zu entnehmen.

Allen hier diskutierten Darstellungen der Oberfläche eines Enzyms, das heißt der speziellen Aufzählung der Oberflächenaminosäuren der α-Amylase aus *Bacillus sp.* A 7-7 (DSM 12368; vgl. Figur 1) als auch den dargestellten Modelling-Ansätzen liegt eine Berechnung zugrunde. Dabei wird kalkulatorisch eine Sonde der Größe von 1,4 A über die Oberfläche des Proteins gerollt. Alle Positionen, die davon berührt werden, werden zur Kontaktfläche mit dieser Sonde und damit zur Oberfläche gezählt; engere, theoretisch nach außen offene Spalten werden dabei nicht mehr als Oberfläche angesehen sondern dem Molekülinneren zugerechnet. Nach diesem Ansatz erhält man die sogenannte Conolly-Oberfläche, die erstmals von M.L. Connolly in dem Aufsatz "Solvent-Accessible Surfaces of Proteins and Nucleic Acids" (1983) in Science, Band 221, 709-713, beschrieben worden ist. Diese anerkannte Methode ist dem Fachmann vertraut und dient erfindungsgemäß zur Definition der Oberfläche der α-Amylasen.

Für die α-Amylase aus *Bacillus sp.* A 7-7 (DSM 12368) ergaben sich auf diese Weise die erwähnten 407 Oberflächenaminosäuren, die im einzelnen in Beispiel 2 aufgeführt werden.

Die auf der Oberfläche der betrachteten α-Amylasen gelegenen und erfindungsrelevanten Aminosäurereste sind diejenigen, die einen neutralen oder positiv polaren oder geladenen Beitrag zum elektrostatischen Potential des Moleküls bei einem pH-Wert von 7 ausüben. Solch ein Beitrag zum elektrostatischen Potential des Moleküls ist als der Anteil der einzelnen Aminosäure mit Ladungen größer/gleich Null definiert.

Das elektrostatische Potential an einem bestimmten Punkt der Oberfläche wird unter Rückgriff auf die oben erwähnte Conolly-Oberfläche als das Potential definiert, das auf die benannte Sonde einwirkt. Dieses theoretisch an jedem Ort der Oberfläche herrschende elektrostatische Potential kann durch geeignete Algorithmen berechnet werden, wozu erfindungsgemäß folgende Überlegung angestellt wird:

Das elektrische Feld ist grundsätzlich als die Summe der durch die Einzelladungen erzeugten elektrischen Teilfelder anzusehen. Für eine brauchbare Berechnung werden die Einzelladungen in erster Näherung als Punktladungen angesehen. Das elektrische Feld $E_i$ einer Punktladung $Q_i$ läßt sich im Vakuum an der Stelle $r_i$ über folgende Gleichung (1) ermitteln, wobei $\varepsilon_0$ die Dielelektrizitätskonstante ist:

$$(1) \qquad \vec{E}_i = \frac{1}{4\pi\varepsilon_0}\frac{Q_i}{r_i^2}\hat{r}_i$$

Ein Ensemble an N Ladungen ergibt somit folgendes elektrische Feld E:

$$(2) \qquad \vec{E} = \sum_{i=1}^{N}\frac{1}{4\pi\varepsilon_0}\frac{Q_i}{r_i^2}\hat{r}_i$$

Das daraus erzeugte Coulomb-Potential $V_c(P_1)$ am Punkt $P_1$ im elektrischen Feld E ist dann folgendermaßen definiert, wobei L für den Koordinaten-Raum steht:

$$(3) \qquad V_c(P_1) = \int_{\infty}^{P_1} \bar{E} \cdot d\bar{L}$$

Diese Gleichung läßt sich in Worten etwa folgendermaßen interpretieren: Das am Punkt $P_1$ herrschende elektrostatische Potential ist als der Potentialunterschied zwischen einem unendlich entfernten Punkt und dem Punkt $P_1$ definiert. L ist ein Ortsvektor, der in der Praxis die Integrationsvariable des Raumes repräsentiert. Der Punkt $P_1$ und dementsprechend auch jeder weitere relevante, auf die gleiche Weise in diese Berechnung eingebrachte Punkt liegt dabei auf der Proteinoberfläche. Mit dieser Formel können Computer mit geeigneten Computerprogrammen für jeden Punkt auf der Proteinoberfläche das jeweilige lokale elektrostatische Potential ermitteln. Hierdurch kann jedem Aminosäurerest eine entsprechende Ladung oder Polarität zugewiesen werden, was in einer visuellen Darstellung beispielsweise wie in Figur 1 veranschaulicht werden kann.

Die Berechnung mit dieser Gleichung (3) ergibt eine nicht exakte aber erfindungsgemäß hinreichende Näherung des elektrostatischen Potentials. In erster Näherung muß die Dielektrizitätskonstante $\varepsilon_0$ durch die tatsächliche Dielelektrizitätskonstante $\varepsilon = \varepsilon_0 {*} \varepsilon_r$ als der Dielektrizitätskonstante des Mediums ersetzt werden. Dieser Ansatz wird "Screened Coulomb Potential" genannt und ist von S. A. Hassa et al. (2002) im Aufsatz "A Critical Analysis of Continuum Electrostatics: The Screened Coulomb Potential-Implicit Solvent Model and the Study of the Alanine Dipeptide and Discrimination of Misfolded Structures of Proteins", in Proteins, Band 45, Seite 47 beschrieben. Für weitere genauere Algorithmen finden sich in der Literatur mehrere Beispiele, wie etwa E. L. Mehler et al. (1991), "Electrostatic effects in proteins: comparison of dielectric and charge models", Protein Eng., Band 8, Seiten 903 - 910, und A. Jakalian et al. (2002) "Fast, efficient generation of high-quality atomic charges. AM1-BCC model: II. Parameterization and validation" in J. Comput. Chem., Band 16, Seiten 1623 - 1641. Für die hier beschriebene Anwendung sind diese jedoch nicht erforderlich, da letztendlich eine semiquantitative Aussage (positiv, neutral, negativ) die Lösung der Aufgabenstellung zuläßt.

Die betreffenden Aminosäuren können beispielsweise über einen Computer-Algorithmus berechnet werden, der als weiteres Modul des bereits erwähnten SwissPDB-Viewer zur Verfügung steht. Darin kann über die Punkte "Tools", "Compute Molecular Surface", und "Electrostatic potential" die Oberflächenladungsverteilung des untersuchten Moleküls berechnet werden, wobei unter Standardparametern die Partialladungen der Atome jeder Aminosäure mit Ausnahme der Wasserstoffatome berücksichtigt werden.

Hierdurch wurden, wie in Beispiel 2 dargestellt ist, für die $\alpha$-Amylase aus *Bacillus sp.* A 7-7 (DSM 12368) als Teilmenge der zuvor bestimmten 407 Oberflächenaminosäurereste 118 Reste ermittelt, die einen positiven oder neutralen Beitrag zum elektrostatischen Potential der Oberfläche ausüben.

Zur Bezeichnung der jeweils erlaubten Austausche wird betrachtet, welche Polaritätswerte oder Ladungen die verschiedenen Aminosäureseitenketten an sich besitzen. Dadurch ergibt sich folgende Reihenfolge: R, als die am stärksten basische und in der Regel positiv geladene Aminosäureseitenkette, gefolgt von K, Y, C, H, G, A, V, L, I, M, F, W, P, S, T, N, Q, E und D, wobei D die am stärksten saure Aminosäureseitenkette darstellt und bei neutralem pH-Wert eine negative Ladung trägt. Hieraus erklärt sich die oben bezeichnete Abstufung, wobei erfindungsgemäß davon ausgegangen werden kann, daß bei Austausch einzelner Oberflächenaminosäuren die Struktur des Moleküls und die weiteren Ladungseffekte praktisch keine Rolle spielen. Sollte eine einzelne dieser Aminosäureseitenketten also einen leicht negativeren oder leicht positiveren Ladungsanteil tragen, als aufgrund der für die freie Aminosäure allgemein bekannten Werte zu erwarten wäre, so wird erfindungsgemäß davon ausgegangen, daß eine in dieser Reihenfolge später genannte Aminosäureseitenkette in derselben Molekülumgebung einen dementsprechend leicht negativeren oder leicht positiveren Ladungsanteil trägt und die hier genannte Reihenfolge der für den Austausch erlaubten Aminosäuren insgesamt nicht verändert wird.

Die Darstellung in Figur 1 zeigt das gemäß Beispiel 2 errechnete Muster an unterschiedlichen Polaritäten und Ladungen auf einer Oberfläche der $\alpha$-Amylase aus *Bacillus sp.* A 7-7 (DSM 12368). Die dort nicht zu sehende Rückseite des Moleküls, die auch das aktive Zentrum enthält, weist ein durchgehend negatives Ladungsmuster auf. Hieraus wird verständlich, daß sich relativ leicht Dimere bilden können, bei denen sich ein Molekül mit einer in Figur 1 zu sehenden positiv polarisierten oder geladenen Fläche an die rückwärtig gelegene, negativ geladene/polarisierte Fläche eines anderen Moleküls anlagert und dadurch dessen aktives Zentrum blockiert. Derartige Dimere sollten an einem doppelt so hohen Molekulargewicht und/oder einer im Verhältnis zur Gesamt-Proteinkonzentration halb so hohen Amylase-Aktivität zu erkennen sein, jeweils im Vergleich zu einer entsprechenden Lösung mit Monomeren. Mit zunehmender Anlagerung weiterer Moleküle in der gleichen Orientierung sollte die Aktivität entsprechender Lösungen weiter abfallen, bis schließlich ein weit genug aggregierter Anteil der Enzyme als amorpher Niederschlag ausfällt und endgültig denaturiert und unbrauchbar geworden ist.

Diesen Prozeß sollten erfindungsgemäße α-Amylase-Varianten, der Zahl und Lokalisation der durchgeführten erfindungsgemäßen Austausche entsprechend, statistisch gesehen weniger durchlaufen.

In einer bevorzugten Ausführungsform handelt es sich bei erfindungsgemäßen α-Amylase-Varianten um solche, in denen der genannte Aminosäurerest vor dem Aminosäureaustausch eine Akzessibilität von mindestens 10%, vorzugsweise mindestens 20%, besonders bevorzugt mindestens 30% aufweist, wobei die Akzessibilität des betreffenden Aminosäurerests über eine Skala von 0% (nicht dem Solvens zugänglich) bis 100% (in einem hypothetischen Pentapeptid GGXGG enthalten) berechnet wird.

Unter der Akzessibilität eines Aminosäurerests ist erfindungsgemäß zu verstehen, wie gut er in natürlicher Konformation des Moleküls dem umgebenden Lösungsmittel (zumeist Wasser) zugänglich ist. Zur Ermittlung dieser physikalisch-chemischen Eigenschaft wird eine Skala von 0 bis 100% zugrundegelegt, wobei ein Wert von 0% Akzessibilität bedeutet, daß der betreffende Rest dem Solvens nicht zugänglich ist, und 100% für die in einem hypothetischen Pentapeptid GGXGG mögliche Zugänglichkeit steht.

Erfindungsgemäß spiegelt sich diese Moleküleigenschaft darin wieder, daß ein exponierter, sich aus der Oberfläche abhebender Aminosäurerest leichter mit anderen Molekülen in Berührung kommt als einer, der dem Lösungsmittel weniger zugänglich ist. Somit stellen solche Reste bevorzugte Angriffspunkte für die Umsetzung der vorliegenden Erfindung dar.

Über den bereits genannten SwissPDB-Viewer besteht die Möglichkeit, auch diese Werte für jede Oberflächen-Aminosäure eines globulären Proteins zu berechnen. Die Berechnung der Akzessibilität der Aminosäuren geschieht dort über den Menüpunkt "Select --> Accessible aa", gefolgt von der Eingabe der Zugänglichkeit in Prozent. Danach sind die entsprechenden Aminosäuren ausgewählt und können durch Drücken der "Return"-Taste angezeigt werden. Es ist dabei zusätzlich möglich, die Berechnung über ein selbst erstellbares Programm zu automatisieren, was umso empfehlenswerter ist, je mehr Aminosäuren in die Berechnung einbezogen werden sollen.

Wie in Beispiel 2 dargestellt ist, wurden für die α-Amylase von *Bacillus sp.* A 7-7 (DSM 12368) folgende 97 Aminosäurereste ermittelt, die einen positiven oder neutralen Beitrag zum elektrostatischen Potential der Oberfläche leisten und gleichzeitig einen Wert für die Akzessibilität von mindestens 10% aufweisen, wobei die Werte für die Solvenszugänglichkeit jeweils in % in Klammern hinter den genannten Positionen angegeben sind:

> T5 (39), N6 (12), G7 (13), N19 (28), N22 (28), N25 (16), R26 (27), R28 (39), S29 (38), S32 (28), N33 (28), K35 (37), K37 (18), Q53 (12), K72 (27), V75 (30), R76 (24), T81 (16), R82 (22), N83 (44), Q84 (24), Q86 (18), A87 (18), T90 (30), A91 (11), K93 (32), S94 (50), N95 (19), G96 (25), Q98 (29), R118 (41), T136 (22), K142 (30), G149 (14), N150 (39), T151 (22), H152 (27), N154 (41), K156 (30), R158 (33), Y160 (20), R171 (32), Q172 (53), R176 (41), R181 (34), R218 (18), T227 (19), G229 (14), K242 (15), R247 (20), T251 (23), R254 (15), K259 (26), N260 (49), K281 (33), N283 (40), R302 (50), R310 (31), R320 (52), T323 (49), R359 (13), Y368 (12), Y372 (37), T376 (56), K383 (19), K385 (37), Q394 (20), K395 (38), G399 (16), K400 (44), Y404 (11), G417 (11), N418 (25), T419 (59), A420 (37), H421 (16), P422 (46), G435 (25), G436 (17), W439 (47), R444 (49), N445 (41), Q449 (31), V450 (24), R452 (33), R458 (24), S459 (52), G460 (32), T461 (35), T463 (40), N465 (22), A466 (37), N471 (20), S473 (10), N475 (25), G476 (31), N484 (12).

Die für dieses Molekül hinsichtlich der Akzessibilität weiter bevorzugten Ausführungsformen können anhand des angegebenen Prozentwerts für die Akzessibilität ausgewählt werden.

Eine Übertragung auf andere α-Amylasen ist näherungsweise über ein Alignment wie in Figur 2 möglich. Ausschlaggebend ist wie bereits erläutert jedoch die tatsächliche dreidimensionale Struktur, anhand derer wie im Beispiel für die α-Amylase aus *Bacillus sp.* A 7-7 (DSM 12368) demonstriert die entsprechenden Berechnungen zur tatsächlichen Ladungsverteilung und Lösungsmittelzugänglichkeit vorgenommen werden können.

In einer weiter bevorzugten Ausführungsform handelt es sich bei erfindungsgemäßen α-Amylase-Varianten um solche α-Amylase-Varianten, wobei der genannte Aminosäurerest in einem neutralen oder positiv polarisierten oder geladenen Bereich aus mindestens zwei auf der Oberfläche unmittelbar benachbarten Aminosäureresten liegt.

Hierbei wird also die oben erläuterte Berechnung durchgeführt und zur erfindungsgemäßen Mutagenese solch eine Aminosäure ausgewählt, die zusätzlich zu den oben genannten Eigenschaften einem ebensolchen Rest auf der Oberfläche unmittelbar benachbart ist, das heißt das nicht ausschließlich von solchen Aminosäureresten umgeben ist, an denen nicht auch ein erfindungsgemäßer Austausch vorgenommen werden könnte.

Hiermit wird also solch eine Aminosäure einer erfindungsgemäßen Mutation unterzogen, die nicht isoliert auftritt, sondern Teil einer neutralen oder positiv polaren oder geladenen Oberfläche des Moleküls ist. Dadurch wird die flächenhafte Anordnung durchbrochen, die wie oben erläutert besonders als Ursache dafür angesehen werden kann, daß α-Amylasen über elektrostatische Wechselwirkungen aggregieren und Multimere bilden. Eine isolierte positive Ladung oder Polarität sollte statistisch gesehen einen geringeren Beitrag zur Multimerisierung ausüben, da sie durch zunehmend mehr negativ geladene Nachbaraminosäuren weniger Wirkung auf andere Moleküle ausüben dürfte. Deshalb werden bevorzugt Austausche in zusammenhängenden, neutral oder positiv erscheinenden Bereichen vorgenommen. Solche

sind am Beispiel der α-Amylase aus *Bacillus sp.* A 7-7 (DSM 12368) in Figur 1 schwarz oder weiß dargestellt.

Bei den erfindungsgemäßen α-Amylase-Varianten handelt es sich um solche α-Amylase-Varianten, wobei der erfindungsgemäß zu mutierende Aminosäurerest in einer Position liegt, die einer der beiden folgenden Gruppen angehört:

(A) 5, 6, 7, 19, 22, 25, 26, 28, 29, 32, 33, 35, 37, 53, 72, 75, 76, 81, 82, 83, 84, 86, 87, 90, 91, 93, 94, 95, 96, 98, 118, 136, 142, 149, 150, 151, 152, 154, 156, 158, 160, 171, 172, 181, 227, 229, 247, 251, 254, 259, 260, 281, 283, 394, 395, 399, 400, 417, 418, 419, 420, 421 und 422; oder

(B) 435, 436, 439, 444, 445, 449, 450, 452, 458, 459, 460, 461, 463, 465, 466, 471, 473, 475, 476 und 484,

jeweils angegeben in der Zählung des maturen Proteins gemäß SEQ ID NO. 2.

Wie in Beispiel 3 beschrieben ist, konnte die Oberflächenladungsverteilung für die α-Amylase aus *Bacillus sp.* A 7-7 (DSM 12368) genau modelliert werden. Dabei wurde festgestellt, daß die 97 auf der Oberfläche des Moleküls gelegenen und einen neutralen oder positiv polaren oder geladenen Beitrag zum elektrostatischen Potential des Moleküls ausübenden und zusätzlich eine Akzessibilität von mehr als 10% aufweisenden Aminosäurereste dieses Moleküls nach ihrer Lage drei Gruppen zugeordnet werden können. Hierbei stellen die Reste der Gruppen A und B jeweils zusammenhängende Bereiche neutraler oder positiver Polarität oder Ladung dar.

Als Gruppe A werden folgende 63 Aminosäurepositionen bezeichnet, wobei jeweils die in der α-Amylase aus *Bacillus sp.* A 7-7 (DSM 12368) dort vorhandene Aminosäure angegeben ist:

T5, N6, G7, N19, N22, N25, R26, R28, S29, S32, N33, K35, K37, Q53, K72, V75, R76, T81, R82, N83, Q84, Q86, A87, T90, A91, K93, S94, N95, G96, Q98, R118, T136, K142, G149, N150, T151, H152, N154, K156, R158, Y160, R171, Q172, R181, T227, G229, R247, T251, R254, K259, N260, K281, N283, Q394, K395, G399, K400, G417, N418, T419, A420, H421, P422.

Zur Gruppe B gehören folgende 20 Positionen:

G435, G436, W439, R444, N445, Q449, V450, R452, R458, S459, G460, T461, T463, N465, A466, N471, S473, N475, G476, N484.

Die verbleibenden 14 Aminosäurereste, die zur Gruppe C zusammengefaßt werden, können als neutrale oder positive Inseln innerhalb ansonsten negative polarisierter oder geladener Bereiche angesehen werden:

R176 (41), R218 (18), K242 (15), R302 (50), R310 (31), R320 (52), T323 (49), R359 (13), Y368 (12), Y372 (37), T376 (56), K383 (19), K385 (37), Y404 (11).

Bei den zuletzt genannten Aminosäureresten kann man von einem eher geringen Beitrag zur Aggregationsneigung des Gesamtmoleküls ausgehen. Umso mehr dürften die zusammenhängenden Bereiche A und B die Multimerisierung herbeiführen, weil darüber ein stärkerer die Anordnung mehrerer Moleküle herbeiführender elektrostatischer Effekt ausgeübt werden dürfte, welcher wie erfindungsgemäß angenommen zu der beobachteten Tendenz zur Aggregation führt. Deshalb werden die erfindungsgemäßen Aminosäureaustausche in diesen zusammenhängenden Bereichen A und/oder B durchgeführt.

Für prinzipiell jede andere im Stand der Technik etablierte α-Amylase können dieselben Überlegungen und Berechnungen wie im Beispiel für die α-Amylase aus *Bacillus sp.* A 7-7 (DSM 12368) vorgenommen werden. Insbesondere bei diesen konkreten Positionen sei auf den einfacheren Vergleich der zugehörigen Aminosäuresequenzen über ein Alignment hingewiesen. Die jeweiligen α-Amylasen können mit der α-Amylase aus *Bacillus sp.* A 7-7 (DSM 12368) homologisiert werden. Solch ein Alignment ist beispielsweise in Figur 2 für die wichtigsten im Stand der Technik etabierten α-Amylasen gezeigt; die Zählung des maturen Proteins gemäß SEQ ID NO. 2 entspricht Zeile 1 in Figur 2. Über solch ein Alignment können die hier aufgeführten, für die Austausche bevorzugten Positionen für das jeweils interessierende Enzym abgeleitet werden, die zumindest näherungsweise mit gleichem Erfolg wie bei dem hier betrachteten Enzym verändert werden können. Wie bereits erläutert ist letztlich jedoch die jeweils im betreffenden Enzym konkret vorhandene Situation ausschlaggebend.

In einer weiter bevorzugten Ausführungsform handelt es sich bei erfindungsgemäßen α-Amylase-Varianten um solche α-Amylase-Varianten, wobei mehrere der genannten Aminosäureaustausche durchgeführt worden sind, vorzugsweise mindestens 2, 3, 4, 5, 6, 7, 8, 9, 10, 11, 12, 13, 14, 15, 16, 17, 18, 19, 20, 21, 22, 23, 24, 25, 26, 27, 28, 29 oder 30, besonders bevorzugt zwischen 10 und 30 und ganz besonders bevorzugt zwischen 15 und 25.

Denn der Lösung der vorliegenden Aufgabe hat die Beobachtung zugrundegelegen, daß manche α-Amylasen amorphe Aggregate bilden, und als mögliche Erklärung hierfür wurde die Existenz größerer unterschiedlich geladener oder polarisiererter flächiger Bereiche angesehen. Gleichzeitig sind aus dem Stand der Technik α-Amylasen bekannt,

die überwiegend negative Oberflächenladungen aufweisen. Deshalb ist es vorteilhaft, auch mehr als lediglich einen Rest erfindungsgemäß in einen stärker negativ polaren oder negativ geladenen Aminosäurerest zu überführen.

Diesem Vorgehen ist der Natur der Enzyme entsprechend eine individuelle Obergrenze gesetzt, die gegebenenfalls experimentell ermittelt werden muß. So dürfte ein Molekül, das mit zu vielen negativen Ladungen übersät ist, zu stark vom Substrat abgestoßen werden und dadurch an Aktivität verlieren. Zudem können sich zu viele Ladungen auch ungünstig auf die Faltung auswirken, so daß ab einem kritischen Wert zu viele fehlgefaltete und deshalb nicht aktive Moleküle gebildet würden.

Zumindest für die α-Amylase aus *Bacillus sp.* A 7-7 (DSM 12368) hat es sich daher als vorteilhaft herausgestellt, die Zahl der erfindungsgemäßen Austausche auf weniger als 30 zu beschränken, wobei mehr als 10 durchaus als vorteilhaft anzusehen sind. Für die hiermit homologisierbaren α-Amylasen, zum Beispiel denen aus Figur 2 und insbesondere denen, die an den homologen Positionen ebenfalls über neutrale oder positiv geladene oder polarisierte Aminosäuren tragen, ist ein ähnliches Ergebnis zu erwarten.

In einer weiter bevorzugten Ausführungsform handelt es sich bei erfindungsgemäßen α-Amylase-Varianten um solche α-Amylase-Varianten, bei denen an mindestens einer bis höchstens genauso vielen anderen Positionen ein oder mehrere andere auf der Oberfläche des Moleküls gelegene Aminosäurereste des Ausgangsmoleküls zu weniger negativ polaren oder negativ geladenen Aminosäureresten ausgetauscht worden ist/sind, wobei jeweils folgende Aminosäureaustausche möglich sind:

| Ausgangsaminosäure | zu |
|---|---|
| K, Y, C, H, G, A, V, L, I, M, F, W, P, S, T, N, Q, E oder D | Arg (R) |
| Y, C, H, G, A, V, L, I, M, F, W, P, S, T, N, Q, E oder D | I ys (K) |
| C, H, G, A, V, L, I, M, F, W, P, S, T, N, Q, E oder D | Tyr (Y) |
| H, G, A, V, L, I, M, F, W, P, S, T, N, Q, E oder D | Cys (C) |
| G, A, V, L, I, M, F, W, P, S, T, N, Q, E oder D | His (H) |
| A, V, L, I, M, F, W, P, S, T, N, Q, E oder D | Gly (G) |
| V, L, I, M, F, W, P, S, T, N, Q, E oder D | Ala (A) |
| L, I, M, F, W, P, S, T, N, Q, E oder D | Val (V) |
| I, M, F, W, P, S, T, N, Q, E oder D | Leu (L) |
| M, F, W, P, S, T, N, Q, E oder D | Ile (I) |
| F, W, P, S, T, N, Q, E oder D | Met (M) |
| W, P, S, T, N, Q, E oder D | Phe (F) |
| P, S, T, N, Q, E oder D | Trp (W) |
| S, T, N, Q, E oder D | Pro (P) |
| T, N, Q, E oder D | Ser (S) |
| N, Q, E oder D | Thr (T) |
| Q, E oder D | Asn (N) |
| E oder D | Gln (Q) |
| D | Glu (E) |

Durch diese der eigentlichen Erfindung entgegengesetzte Mutation wird der erfindungsgemäß eingeführte Ladungseffekt wieder abgeschwächt. Dadurch werden zweierlei Ziele verfolgt: Zum einen kann wie oben erwähnt, insbesondere wenn mehrere erfindungsgemäße Mutationen durchgeführt werden (erfindungsgemäß werden bis zu 30 als besonders sinnvoll angesehen), das α-Amylase-Molekül eine insgesamt zu starke negative Ladung erhalten, so daß Stabilität und Reaktivität beeinträchtigt werden könnten. Insofern kann es angebracht sein, insbesondere wenn mehrere Mutationen durchgeführt werden, deren Ladungseffekt in der Summe wieder abzuschwächen.

Zum anderen wird dadurch die Flächenhaftigkeit der Ladungsverteilung auf der Oberfläche des Enzyms durchbrochen. Diese Flächenhaftigkeit wurde, wie oben erläutert, als ein wesentlicher Grund für die Multimerisierung angesehen. Wenn nun bei insgesamt gleichbleibender oder nur gering veränderter Gesamtladung eine Änderung des Ladungsmusters hin zu einem aufgelockerteren Muster erreicht wird, ist ebenfalls mit einer Verringerung der Aggregationsneigung zu rechnen, weil sich die Moleküle nicht mehr automatisch wie Magnete in regelmäßiger Orientierung zueinander anordnen.

Zur Bezeichnung der jeweils erlaubten Austausche wird auf die oben bereits erläuterte Betrachtung zurückgegriffen, welche Polaritätswerte oder Ladungen die verschiedenen Aminosäureseitenketten an sich besitzen. Dadurch ergibt sich genau die umgekehrte Reihenfolge, weil R, als die stärksten basische und in der Regel positiv geladene Aminosäureseitenkette zur Herbeiführung einer eher positiven Polarität oder Ladung durch keine andere ersetzt werden kann, selbst aber an die Stelle jeder anderen treten kann. Dies gilt in entsprechender Abstufung für K, Y, C, H, G, A, V, L, I, M, F, W, P, S, T, N, Q, E und D, wobei D die am stärksten saure Aminosäureseitenkette darstellt, bei neutralem pH-Wert in der Regel eine negative Ladung trägt und unter diesem Erfindungsaspekt durch jede andere, einschließlich E ersetzt werden kann.

In einer weiter bevorzugten Ausführungsform handelt es sich bei erfindungsgemäßen α-Amylase-Varianten um solche α-Amylase-Varianten, wobei der beziehungsweise die zur Verringerung der Gesamtladungsänderung durchgeführte(n) Austausch(e) an Aminsäureposition(en) durchgeführt worden ist/sind, der/die nicht zu den folgenden gehört/en:

(A) 5, 6, 7, 19, 22, 25, 26, 28, 29, 32, 33, 35, 37, 53, 72, 75, 76, 81, 82, 83, 84, 86, 87, 90, 91, 93, 94, 95, 96, 98, 118, 136, 142, 149, 150, 151, 152, 154, 156, 158, 160, 171, 172, 181, 227, 229, 247, 251, 254, 259, 260, 281, 283, 394, 395, 399, 400, 417, 418, 419, 420, 421 und 422 oder
(B) 435, 436, 439, 444, 445, 449, 450, 452, 458, 459, 460, 461, 463, 465, 466, 471, 473, 475, 476 und 484 oder
(C) 176, 218, 242, 302, 310, 320, 323, 359, 368, 372, 376, 383, 385 und 404,

jeweils angegeben in der Zählung des maturen Proteins gemäß SEQ ID NO. 2.

In dieser bevorzugten Ausführungsform wird die an der α-Amylase aus *Bacillus sp.* A 7-7 (DSM 12368) gewonnene Erkenntnis aufgegriffen, wonach diese, den Bereichen A und B zuzuordnenden Aminosäurereste großflächige neutral oder positiv polarisierte oder geladene Bereiche ausbilden und die unter (C) genannten Reste neutral oder positiv polarisierte oder geladene Inseln innerhalb einer überwiegend negativen Umgebung bilden. Da wie oben erläutert unter dem hier betrachteten Erfindungsaspekt gerade die Flächenhaftigkeit der Ladungsverteilung durchbrochen werden soll, ist es besonders sinnvoll, für die ladungsausgleichende Rückmutation andere Positionen als die hier bezeichneten auszuwählen. Denn dadurch würde man wieder die genannten neutral oder positiv polarisierten oder geladenen Regionen vergrößern.

In einer weiter bevorzugten Ausführungsform sind erfindungsgemäße α-Amylase-Varianten solche α-Amylase-Varianten, wobei es sich bei dem Ausgangsmolekül um die folgende α-Amylase handelt: α-Amylase aus *Bacillus sp.* A 7-7 (DSM 12368).

Wie einleitend dargestellt sind im Stand der Technik zahlreiche α-Amylasen für den Einsatz zu verschiedenen technischen Zwecken etabliert. Hierzu zählen beispielsweise pilzliche und bakterielle Enzyme.

Für technische Zwecke sind solche α-Amylasen aus grampositiven Bakterien, insbesondere der Gattung *Bacillus,* die an ein alkalisches Milieu angepaßt sind, besonders gebräuchlich. Denn sie weisen von vornherein günstige Eigenschaften für diese Einsatzgebiete auf. Dementsprechend ist die vorliegende Erfindung auf die α-Amylase aus *Bacillus sp.* A 7-7 (DSM 12368), bekannt aus WO 02/10356 A2 und der vorliegenden Anmeldung gerichtet.

Zusätzlich sind im Stand der Technik zahlreiche durch Mutagenese einzelner oder mehrerer Aminosäuren an den genannten α-Amylasen abgeleitete α-Amylase-Varianten beschrieben. Auf all diese kann die vorliegende Erfindung ebenfalls angewendet werden, wobei prinzipiell davon auszugehen ist, daß die jeweiligen Effekte additiv sind. So dürfte sich eine α-Amylase-Variante, die aufgrund eines bestimmten Aminosäureaustauschs in einem speziellen Anwendungsgebiet besonders leistungsfähig ist, durch die Vornahme einer erfindungsgemäßen Mutation zusätzlich zu diesem Leistungsaspekt auch in ihrer Tendenz zur Aggregation verbessern lassen. Vorzugsweise handelt es sich also um Aminosäurevariationen, für die bereits ein entsprechender Vorteil beschrieben ist.

Hierbei spielt es prinzipiell keine Rolle, in welcher Reihenfolge die betreffenden Austausche vorgenommen worden sind, das heißt ob eine entsprechende Punktmutante erfindungsgemäß weiterentwickelt wird oder zunächst beispielsweise aus einem Wildtypmolekül eine erfindungsgemäße Variante erzeugt wird, die entsprechend anderer im Stand der Technik zu findender Lehren weiterentwickelt wird. Es können auch gleichzeitig in einem Mutageneseansatz mehrere Austausche vorgenommen werden, etwa erfindungsgemäße und andere zusammen. Diese Situation tritt beispielsweise dann ein, wenn eine α-Amylase mit statistisch wirkenden Mutageneseverfahren wie etwa durch mutagenisierende Agentien oder Shuffling weiterentwickelt wird. Das gilt für jede Art von Modifikation der betreffenden Enzyme, insbesondere für Punktmutationen, die prinzipiell unabhängig voneinander wirken.

Besonders leistungsfähige, durch Punktmutationen erhältliche α-Amylasen gehen beispielsweise aus der Anmeldung WO 00/60060 A2 hervor, die anhand der Amylase AA560 (selbe Zählung wie das mature Protein in SEQ ID NO. 2) zahlreiche Mutationen in den Positionen 181, 182, 183, 184, 195, 206, 212, 216, und 269 beschreibt. Diese

Anmeldung kann als Weiterentwicklung von WO 96/23873 A1 angesehen werden, die zuvor schon die Möglichkeit der Punktmutagenese zur Leistungsverbesserung in den Positionen 180, 181, 182, 183, 184 und 185 beschrieben hatte. Weitere Verbesserungen der darin als Termamyl-ähnliche Amylasen bezeichneten Enzyme benennt die Anmeldung WO 00/60059 A2; demnach seien die Positionen 13, 48, 49, 50, 51, 52, 53, 54, 57, 107, 108, 111, 168 und 197 (in der Zählung der α-Amylase aus *B. licheniformis)* hierfür geeignete Ansatzpunkte. Bei dem Ausgangsmolekül handelt es sich um α-Amylase aus *Bacillus sp.* A 7-7 (DSM 12368).

So wird die hier beschriebene Erfindung in den Beispielen der vorliegenden Anmeldung insbesondere anhand der α-Amylase aus *Bacillus sp.* A 7-7 (DSM 12368) dargestellt, deren aller Wahrscheinlichkeit nach korrekte DNA- und Aminosäuresequenzen im Sequenzprotokoll der vorliegenden Anmeldung gezeigt sind. Dieses Wildtypenzym besitzt gegenüber anderen etablierten α-Amylasen die in der Anmeldung WO 02/10356 A2 dargestellten Vorteile hinsichtlich eines Einsatzes in Wasch- und Reinigungsmitteln. Mit der Cyclodextrin-Glucanotransferase aus *B. agaradherens* (DSM 9948) ist ein weiteres Ausgangsmolekül oben bereits erwähnt worden, welches, wie in der Anmeldung WO 02/44350 A2 beschrieben, gegenüber anderen Enzymen ebenfalls besondere Beiträge zur Gesamtleistung von Wasch- und Reinigungsmitteln leistet.

Hybridamylasen aus den α-Amylasen aus *B. amyloliquefaciens* und *B. licheniformis* werden in der Anmeldung WO 03/014358 A2 beschrieben. Unter diesen haben die mit den Akronymen AL34, AL76, AL112, AL256, ALA34-84, LAL19-153 oder LAL19-433 bezeichneten Moleküle besonders vorteilhafte Leistungen beim Einsatz in Wasch- und Reinigungsmitteln gezeigt. Die jeweiligen Namen bezeichnen die Elemente, aus denen sie jeweils zusammengesetzt sind, vom N-Terminus her betrachtet, wobei A für die α-Amylase aus *B. amyloliquefaciens* und L für die α-Amylase aus *B. licheniformis* stehen und die nachfolgende Nummer den Übergangspunkt der einen Aminosäuresequenz zur anderen angibt. Weitere Details hierzu können der genannten Anmeldung entnommen werden.

Erfindungsgemäße α-Amylase-Varianten sind solche α-Amylase-Varianten, wobei es sich bei dem Ausgangsmolekül um eine α-Amylase handelt, deren Aminosäuresequenz zu der in SEQ ID NO. 2 angegebenen Aminosäuresequenz in den Positionen +1 bis 484 zu 100% identisch ist.

Diese α-Amylase aus *Bacillus sp.* A 7-7 (DSM 12368) wird in der bereits mehrmals erwähnten internationalen Patentanmeldung WO 02/10356 A2 ausführlich beschrieben. Hierzu sei angemerkt, daß die Nukleotidsequenz und die davon abgeleitete Aminosäuresequenz der aus diesem Stamm erhältlichen α-Amylase aller Wahrscheinlichkeit nach die in SEQ ID NO. 1 beziehungsweise 2 der vorliegenden Anmeldung angegebenen Sequenzen aufweisen. Dies ist eine Korrektur gegenüber den in der Anmeldung WO 02/10356 A2 angegebenen Sequenzen. Auch die in jener Anmeldung beschriebenen Versuche sind mit der aus diesem Stamm erhältlichen, nativen α-Amylase durchgeführt worden.

Die Nukleotidsequenz gemäß SEQ ID NO. 1 versetzt den Fachmann unmittelbar in die Lage, erfindungsgemäße Mutationen vorzunehmen. Wer anstatt auf den hinterlegten Mikroorganismus zurückzugreifen, eine α-Amylase-codierende DNA nach dem Sequenzprotokoll von WO 02/10356 A2 hergestellt hat, kann diese mithilfe einfacher Punktmutageneseverfahren, wie sie auch in den Beispielen der vorliegenden Anmeldung genannt werden, in eine Nukleotidsequenz gemäß SEQ ID NO. 1 der vorliegenden Anmeldung umwandeln.

Die Kultivierungsmöglichkeiten des zugehörigen Mikroorganismus, die Aufreinigung und die enzymatische Charakterisierung der Wildtyp-α-Amylase sind aus jener Anmeldung ebenfalls bekannt und werden in Beispiel 1 der vorliegenden Anmeldung noch einmal zusammengefaßt. Erfindungsgemäße Varianten dieses Enzyms lassen sich in prinzipiell gleicher Weise gewinnen und aufreinigen, wobei - beispielsweise bei der Betrachtung des isoelektrischen Punkts (IEP) - die erfindungsgemäß eingeführten Unterschiede zu berücksichtigen sind. Beispielsweise der IEP dürfte etwas in den sauren Bereich verschoben sein.

Weiterhin bevorzugt sind solche erfindungsgemäßen α-Amylase-Varianten, wobei es sich bei dem Ausgangsmolekül um eine α-Amylase-Variante mit zunehmend bevorzugt 1, 2, 3, 4, 5, 6, 7, 8, 9, 10 oder 11 zusätzlichen Punktmutationen handelt.

Die in SEQ ID NO. 1 und 2 angegebenen Sequenzen unterscheiden sich, wie in Beispiel 1 erläutert wird, in jeweils zwei Positionen von den Angaben in WO 02/10356 A2. Sie stellen nach dem heutigen Kenntnisstand die besten Beschreibungen der α-Amylase aus *Bacillus sp.* A 7-7 (DSM 12368) dar und sind somit besonders bevorzugte Ausgangspunkte zum Einführen erfindungsgemäßer Aminosäureaustausche.

Ferner kann man entsprechend dem oben Gesagten davon ausgehen, daß prinzipiell jede α-Amylase mit einzelnen Punktmutationen, das heißt Deletionen, Insertionen oder Substitutionen einzelner Aminosäuren hinsichtlich anderer Aspekte verbessert werden kann. So gehen, wie einleitend bereits dargestellt, aus den Anmeldungen WO 00/22103 A1, WO 96/23873 A1, WO 00/60060 A2 und WO 01/66712 A2 in einzelnen Positionen mutagenisierte α-Amylase-Varianten hervor, die jeweils hinsichtlich spezieller Aspekte verbessert worden sind. Da diese zu dieser in SEQ ID NO. 2 gezeigten Amylase zum Teil hohe Homologiewerte aufweisen, das heißt als hochverwandt bezeichnet werden können, ist zu erwarten, daß sich die darin bezeichneten Austausche mit den gleichen vorteilhaften Auswirkungen auch auf diese Moleküle sowie in analoger Weise auch auf andere α-Amylasen übertragen lassen. Weitere mögliche Modifikationen können dem Stand der Technik entnommen werden, wie er beispielsweise in der Einleitung zur vorliegenden Anmeldung zusammengefaßt ist.

Weiterhin bevorzugte Ausführungsformen der vorliegenden Erfindung sind erfindungsgemäße α-Amylase-Varianten mit den zusätzlichen Punktmutationen in einer oder mehreren der folgenden Positionen: 5, 167, 170, 177, 202, 204, 271, 330, 377, 385 und 445, in der Zählung des maturen Proteins gemäß SEQ ID NO. 2.

So geht aus der nicht vorveröffentlichten deutschen Patentanmeldung DE 10309803.8 hervor, daß in den Positionen -19, 5, 167, 170, 177, 204, 271, 330, 377, 385 und/oder 445 (beziehungsweise 13, 32, 194, 197, 203, 230, 297, 356, 406, 414 und 474 gemäß der Zählung der nicht prozessierten α-Amylase aus *B. amyloliquefaciens)* Punktmutationen zur Verbesserung der Alkaliaktivität von α-Amylasen vorgenommen werden können. Vorzugsweise wird die vorliegende Erfindung deshalb auf diese bereits verbesserten Moleküle angewendet.

Gegenüber Oxidation stabilisierte α-Amylase-Varianten gehen aus der Anmeldung WO 94/18314 A1 hervor, hierunter vor allem in Position 197 (in der Zählung der α-Amylase von *B. licheniformis*), die der Position 202 der α-Amylase gemäß SEQ ID NO. 2 entspricht. Erfindungsgemäße Varianten können durch die darin genannten Austausche, vor allem in Position 202 zusätzlich gegenüber Oxidation stabilisiert werden. Dieser Austausch ist auch deshalb besonders interessant, weil es sich bei dem M202 in der α-Amylase aus *Bacillus sp.* A 7-7 (DSM 12368) um den einzigen Methionin-Rest handelt, der mit einem Akzessibilitätswert von 30% dem Lösungsmittel zu mehr als 10% zugänglich ist (vergleiche Beispiel 2). Hierdurch erklärt sich dessen besondere Oxidationssensitivität sowie der Zusammenhang zur vorliegenden Erfindung.

Bevorzugte Ausführungsformen hiervon stellen solche α-Amylase-Varianten dar, wobei es sich in den Ausgangsenzymen um folgende Punktmutationen handelt: 5A, 167R, 170P, 177L, 202L, 204V, 271 D, 330D, 377R, 385S und/oder 445Q.

So sind die Sequenzvariationen -19P, 5A, 167R, 170P, 177L, 204V, 271 D, 330D, 377R, 385S und 445Q (beziehungsweise in der Zählung der unprozessierten α-Amylase aus *B. licheniformis* die Austausche zu 13P, 32A, 194R, 197P, 203L, 230V, 297D, 356D, 406R, 414S und 474Q) in der erwähnten Anmeldung DE 10309803.8 als bevorzugte Austausche beschrieben.

In WO 94/18314 A1 wird besonders die oxidationsstabilisierende Wirkung des Austauschs M197L dargestellt, der der Aminosäuresubstitution M202L gemäß SEQ ID NO.2 entspricht. Erfindungsgemäße Varianten können somit insbesondere durch diese Punktmutation zusätzlich gegenüber Oxidation stabilisiert werden.

Einen weiteren Gegenstand der vorliegenden Erfindung bilden Verfahren zur Erhöhung der Stabilität einer α-Amylase gegenüber einer über elektrostatische Wechselwirkungen vermittelten Dimerisierung und/oder Multimerisierung, wodurch mindestens ein auf der Oberfläche des Moleküls gelegener und einen neutralen oder positiv polaren oder geladenen Beitrag zum elektrostatischen Potential des Moleküls ausübender Aminosäurerest des Ausgangsmoleküls zu einem stärker negativ polaren oder negativ geladenen Aminosäurerest ausgetauscht wird ist, wobei folgende Aminosäureaustausche möglich sind:

| Ausgangsaminosäure | zu |
|---|---|
| Arg (R) | K, Y, C, H, G, A, V, L, I, M, F, W, P, S, T, N, Q, E oder D |
| Lys (K) | Y, C, H, G, A, V, L, I, M, F, W, P, S, T, N, Q, E oder D |
| Tyr (Y) | C, H, G, A, V, L, I, M, F, W, P, S, T, N, Q, E oder D |
| Cys (C) | H, G, A, V, L, I, M, F, W, P, S, T, N, Q, E oder D |
| His (H) | G, A, V, L, I, M, F, W, P, S, T, N, Q, E oder D |
| Gly (G) | A, V, L, I, M, F, W, P, S, T, N, Q, E oder D |
| Ala (A) | V, L, I, M, F, W, P, S, T, N, Q, E oder D |
| Val (V) | L, I, M, F, W, P, S, T, N, Q, E oder D |
| Leu (L) | I, M, F, W, P, S, T, N, Q, E oder D |
| Ile (I) | M, F, W, P, S, T, N, Q, E oder D |
| Met (M) | F, W, P, S, T, N, Q, E oder D |
| Phe (F) | W, P, S, T, N, Q, E oder D |
| Trp (W) | P, S, T, N, Q, E oder D |
| Pro (P) | S, T, N, Q, E oder D |
| Ser (S) | T, N, Q, E oder D |
| Thr (T) | N, Q, E oder D |

(fortgesetzt)

| Ausgangsaminosäure | zu |
|---|---|
| Asn (N) | Q, E oder D |
| Gln (Q) | E oder D |
| Glu (E) | D |

und wobei der genannte Aminosäurerest in einer Position liegt, die einer der beiden folgenden Gruppen angehört:

(A) 5, 6, 7, 19, 22, 25, 26, 28, 29, 32, 33, 35, 37, 53, 72, 75, 76, 81, 82, 83, 84, 86, 87, 90, 91, 93, 94, 95, 96, 98, 118, 136, 142, 149, 150, 151, 152, 154, 156, 158, 160, 171, 172, 181, 227, 229, 247, 251, 254, 259, 260, 281, 283, 394, 395, 399, 400, 417, 418, 419, 420, 421 und 422; oder

(B) 435, 436, 439, 444, 445, 449, 450, 452, 458, 459, 460, 461, 463, 465, 466, 471, 473, 475, 476 und 484,

jeweils angegeben in der Zählung des maturen Proteins gemäß SEQ ID NO. 2, wobei es sich bei dem Ausgangsmolekül um die α-Amylase aus *Bacillus sp.* A 7-7 (DSM 12368) handelt, deren Aminosäuresequenz zu der in SEQ ID NO.2 angegebenen Aminosäuresequenz in den Positionen +1 bis 484 100% identisch ist.

Erfindungsgemäß wird die Neigung zur Bildung amorpher α-Amylase-Aggregate auf die Bildung von Dimeren, Trimeren oder Aggregate aus mehr einzelnen α-Amylase-Molekülen verstanden. Da hierdurch der betreffenden Enzympräparation, wie einleitend erläutert makroskopisch betrachtet ein beträchtlicher Anteil an Aktivität verlorengeht, wird dies als ein Stabilitätsaspekt verstanden. Jedes Verfahren, das der Aggregation entgegenwirkt, ist somit eines, das die Stabilität der betreffenden Enzympräparation hinsichtlich ihrer Gesamtaktivität erhöht.

Der erfindungsgemäße Ansatz zum Lösen dieses Problems besteht, wie ebenfalls bereits erläutert, darin, die über elektrostatische Wechselwirkungen vermittelte Multimerisierung einzuschränken. Dies geschieht dadurch, daß die auf der Oberfläche der α-Amylase gelegenen Ladungen und Polaritäts-Träger verändert werden, und zwar in Richtung auf weniger neutrale oder weniger positive Polaritäten, das heißt hin zu eher negativen Polaritäten und Ladungen. Hierbei sind, wie bereits erläutert und weiter unten noch einmal aufgegriffen, Austausche in bestimmten Regionen und bestimmten Positionen bevorzugt.

Verfahren zur Herstellung von Enzymvarianten sind einem Biotechnologen an sich bekannt. Er greift dabei insbesondere auf die für die betreffenden Ausgangsenzyme codierenden Nukleinsäuren zurück, die entsprechend der einzuführenden Aminosäure in dem zugehörigen Codon mutiert wird.

Das Prinzip hierzu wird auch aus Beispiel 3 deutlich: Beispielsweise mit Hilfe des QuikChange-Kit (Fa. Stratagene, Kat.-Nr. 200518) werden nach dem zugehörigen Protokoll Primer synthetisiert, die den zu mutierenden Bereich überdecken und die einzuführende Mutation enthalten (Mismatch-Primer). Die Primersequenzen werden anhand der zugehörigen Nukleotidsequenz unter Berücksichtigung des universellen genetischen Codes entworfen. Hierbei sind zur Erzeugung einer weniger neutralen oder positiven Polarität oder Ladung wie oben bereits erläutert die entsprechend abgestuften Aminosäureaustausche möglich. Nach diesem Prinzip wird geeigneterweise ein die α-Amylase-Sequenz enthaltender Expressionsvektor entsprechend mutagenisiert und nach allgemein bekannten Verfahren in einen zur Expression der Amylase geeigenten Expressionsstamm transformiert. Da die Variante im Vergleich zum Ausgangsmolekül in der Regel nur einige Austausche aufweist, kann man sich bei der Wahl des Expressionssystems, der Kultivierungs- und der Aufarbeitungsverfahren an den für das Ausgangsmolekül etablierten Systemen orientieren. Dabei ist zu berücksichtigen, daß erfindungsgemäß die elektrostatischen Eigenschaften der α-Amylasen verändert werden. Eine dementsprechende Änderung des IEP kann beispielsweise mithilfe einer isoelektrischen Fokussierung überprüft werden.

Ansonsten gelten die oben zu den jeweiligen Enzymen gemachten Aussagen entsprechend. Dies gilt entsprechend auch für die bevorzugten Ausführungsformen, das heißt für diejenigen erfindungsgemäßen Verfahren, mit denen die oben bereits als erfindungsgemäß dargestellten Varianten erhalten werden.

Entsprechend dem oben Gesagten handelt es sich bei bevorzugten erfindungsgemäßen Verfahren um solche, wobei der genannte Aminosäurerest vor dem Aminosäureaustausch eine Akzessibilität von mindestens 10%, vorzugsweise mindestens 20%, besonders bevorzugt mindestens 30% aufweist, wobei die Akzessibilität des betreffenden Aminosäurerests über eine Skala von 0% (nicht dem Solvens zugänglich) bis 100% (in einem hypothetischen Pentapeptid GGXGG enthalten) berechnet wird.

Entsprechend dem oben Gesagten handelt es sich bei weiter bevorzugten erfindungsgemäßen Verfahren um solche, wobei der genannte Aminosäurerest in einem neutralen oder positiv polarisierten oder geladenen Bereich aus mindestens zwei auf der Oberfläche unmittelbar benachbarten Aminosäureresten liegt.

Entsprechend dem oben Gesagten handelt es sich bei weiter bevorzugten erfindungsgemäßen Verfahren um solche, wobei mehrere der genannten Aminosäureaustausche durchgeführt werden, vorzugsweise mindestens 2, 3, 4,

5, 6, 7, 8, 9, 10, 11, 12, 13, 14, 15, 16, 17, 18, 19, 20, 21, 22, 23, 24, 25, 26, 27, 28, 29 oder 30, besonders bevorzugt zwischen 10 und 30 und ganz besonders bevorzugt zwischen 15 und 25.

Entsprechend dem oben Gesagten handelt es sich bei weiter bevorzugten erfindungsgemäßen Verfahren um solche, wonach an mindestens einer bis höchstens genauso vielen anderen Positionen ein oder mehrere andere auf der Oberfläche des Moleküls gelegene Aminosäurereste des Ausgangsmoleküls zu weniger negativ polaren oder negativ geladenen Aminosäureresten ausgetauscht wird ist/sind, wobei jeweils folgende Aminosäureaustausche möglich sind:

| Ausgangsaminosäure | zu |
|---|---|
| K, Y, C, H, G, A, V, L, I, M, F, W, P, S, T, N, Q, E oder D | Arg (R) |
| Y, C, H, G, A, V, L, I, M, F, W, P, S, T, N, Q, E oder D | Lys (K) |
| C, H, G, A, V, L, I, M, F, W, P, S, T, N, Q, E oder D | Tyr (Y) |
| H, G, A, V, L, I, M, F, W, P, S, T, N, Q, E oder D | Cys (C) |
| G, A, V, L, I, M, F, W, P, S, T, N, Q, E oder D | His (H) |
| A, V, L, I, M, F, W, P, S, T, N, Q, E oder D | Gly (G) |
| V, L, I, M, F, W, P, S, T, N, Q, E oder D | Ala (A) |
| L, I, M, F, W, P, S, T, N, Q, E oder D | Val (V) |
| I, M, F, W, P, S, T, N, Q, E oder D | Leu (L) |
| M, F, W, P, S, T, N, Q, E oder D | Ile (I) |
| F, W, P, S, T, N, Q, E oder D | Met (M) |
| W, P, S, T, N, Q, E oder D | Phe (F) |
| P, S, T, N, Q, E oder D | Trp (W) |
| S, T, N, Q, E oder D | Pro (P) |
| T, N, Q, E oder D | Ser (S) |
| N, Q, E oder D | Thr (T) |
| Q, E oder D | Asn (N) |
| E oder D | Gln (Q) |
| D | Glu (E) |

Hierbei ist es prinzipiell unerheblich, ob zuerst der erfindungsgemäße Austausch oder zuerst dieser die Ladung ausgleichende Austausch vorgenommen wird.

Hierunter sind entsprechend dem oben Gesagten Verfahren bevorzugt, wobei der beziehungsweise die zur Verringerung der Gesamtladungsänderung durchgeführte(n) Austausch(e) an Aminosäureposition(en) durchgeführt worden ist/sind, der/die nicht zu den folgenden gehört/en:

(A) 5, 6, 7, 19, 22, 25, 26, 28, 29, 32, 33, 35, 37, 53, 72, 75, 76, 81, 82, 83, 84, 86, 87, 90, 91, 93, 94, 95, 96, 98, 118, 136, 142, 149, 150, 151, 152, 154, 156, 158, 160, 171, 172, 181, 227, 229, 247, 251, 254, 259, 260, 281, 283, 394, 395, 399, 400, 417, 418, 419, 420, 421 und 422 oder
(B) 435, 436, 439, 444, 445, 449, 450, 452, 458, 459, 460, 461, 463, 465, 466, 471, 473, 475, 476 und 484 oder
(C) 176, 218, 242, 302, 310, 320, 323, 359, 368, 372, 376, 383, 385 und 404,

jeweils angegeben in der Zählung des maturen Proteins gemäß SEQ ID NO. 2.

Entsprechend dem oben Gesagten handelt es sich bei weiter bevorzugten erfindungsgemäßen Verfahren um solche, wobei es sich bei dem Ausgangsmolekül um α-Amylase aus *Bacillus sp.* A 7-7 (DSM 12368) handelt und/oder um eine hiervon durch Mutagenese einzelner oder mehrerer Aminosäuren abgeleitete α-Amylase.

Hierunter sind entsprechend dem oben Gesagten Verfahren bevorzugt, wobei es sich bei dem Ausgangsmolekül um eine α-Amylase-Variante mit zunehmend bevorzugt 1, 2, 3, 4, 5, 6, 7, 8, 9, 10 oder 11 zusätzlichen Punktmutationen handelt.

Entsprechend dem oben Gesagten handelt es sich bei weiter bevorzugten erfindungsgemäßen Verfahren um

solche, wobei die eingebrachte α-Amylase eine α-Amylase-Variante mit den zusätzlichen Punktmutationen in einer oder mehreren der folgenden Positionen ist: 5, 167, 170, 177, 202, 204, 271, 330, 377, 385 und 445, in der Zählung des maturen Proteins gemäß SEQ ID NO. 2.

Hier sei auch noch einmal darauf hingewiesen, daß es bei Mutanten als erfindungsgemäß einsetzbaren Ausgangsmolekülen prinzipiell keine Rolle spielt, ob die erfindungsgemäße Aminosäuresubstitution nach oder vor Einführen der sonstigen, aus dem Stand der Technik bekannten Austausche erfolgt.

Hierunter sind entsprechend dem oben Gesagten Verfahren bevorzugt, wobei es sich um folgende Punktmutationen handelt: 5A, 167R, 170P, 177L, 202L, 204V, 271 D, 330D, 377R, 385S und/oder 445Q.

Einen weiteren Gegenstand der vorliegenden Erfindung bilden Nukleinsäuren, die für eine der oben beschriebenen erfindungsgemäßen α-Amylase-Varianten codieren.

Hierunter sind sowohl RNA- als auch DNA-Moleküle sowie DNA-Analoga zu verstehen, sowohl der codierende als auch der codogene Strang, und zwar in jedem Leseraster. Denn beispielsweise ist es möglich, die mit der Erfindung verbundene Lehre auszunutzen, um über eine interferierende entsprechende RNA eine Regulation entsprechender α-Amylasen vorzunehmen oder die Lebensdauer der genetischen Information zu erhöhen, indem sie in ein *in vivo* langsamer abbaubares DNA-Analogon überführt wird. Die Nukleinsäuren eröffnen, wie an den weiter unten ausgeführten Erfindungsgegenständen zu erkennen ist, der vorliegenden Erfindung gewissermaßen die molekularbiologische Dimension. Sie sind entsprechend dem oben Gesagten entsprechend bevorzugt.

Vorzugsweise sind hierunter DNA-Moleküle zu verstehen. Denn über diese ist es möglich, die erfindungsgemäßen α-Amylase-Varianten über an sich bekannte molekularbiologische Verfahren herzustellen und/oder gegebenenfalls weiter zu modifizieren. Für die oben genannten, im Stand der Technik etablierten α-Amylasen sind die Nukleotidsequenzen in allgemein bekannten Datenbanken (zum Beispiel Genbank oder Swissprot; siehe oben) hinterlegt oder gehen aus den genannten Publikationen hervor. Diese Sequenzen stellen entsprechend bevorzugte Ausgangspunkte für das Einführen erfindungsgemäßer Punktmutationen dar.

Ausgangspunkte für diese Nukleinsäure können auch die im Sequenzprotokoll angegebenen Sequenzinformationen sein, insbesondere für Derivate der α-Amylase aus *Bacillus sp.* A 7-7 (DSM 12368). Eine weitere Alternative besteht darin, eine Gesamt-DNA-Präparation von bestimmten dafür ins Auge gefaßten Mikroorganismen vorzunehmen und über PCR die endogenen α-Amylasegene zu isolieren. Als Primer können prinzipiell die aus SEQ ID NO. 1 ebenfalls abzulesenden 5'- und 3'-Sequenzabschnitte eingesetzt werden.

Die reinen proteincodierenden Abschnitte können beispielsweise auch über eine PCR mutagenisiert, das heißt erfindungsgemäß in ihrer Sequenz verändert werden. Hierfür werden eine PCR mit einer entsprechenden statistischen Fehlerrate durchgeführt, das PCR-Produkt kloniert und die eingeführten Mutationen durch anschließende Sequenzierung verifiziert.

Bevorzugt ist hierunter eine Nukleinsäure, welche sich von einer Nukleinsäure gemäß SEQ ID NO. 1 ableitet, oder von einer Variante hiervon mit zunehmend bevorzugt 1, 2, 3, 4, 5, 6, 7, 8, 9, 10 oder 11 zusätzlichen Punktmutationen zu solchen Mutationen, die zu einem der oben als erfindungsgemäß definierten Aminosäureaustausche führen, womit diejenigen gemeint sind, wodurch mindestens ein auf der Oberfläche des Moleküls gelegener und einen neutralen oder positiv polaren oder geladenen Beitrag zum elektrostatischen Potential des Moleküls ausübender Aminosäurerest des Ausgangsmoleküls zu einem stärker negativ polaren oder negativ geladenen Aminosäurerest ausgetauscht worden ist, wobei die ausgeführte Rangfolge der erlaubten Aminosäureaustausche gemeint ist, beziehungsweise die beschriebenen ausgleichenden Austausche an mindestens einer bis höchstens genauso vielen anderen Positionen von einem oder mehreren anderen auf der Oberfläche des Moleküls gelegenen Aminosäureresten des Ausgangsmoleküls zu weniger negativ polaren oder negativ geladenen Aminosäureresten, wobei die entsprechend entgegengesetzte Rangfolge der erlaubten Aminosäureaustausche gemeint ist.

Denn wie oben erläutert können in α-Amylase-Moleküle verschiedene Punktmutationen eingeführt werden, die prinzipiell unabhängig voneinander vorteilhafte Wirkungen ausüben können. So können erfindungsgemäße Austausche an solchen Molekülen vorgenommen werden, die insbesondere hinsichtlich spezieller Leistungsaspekte, der Stabilität oder etwa der Allergenizität verbessert worden sind. Dazu gehören insbesondere auch nichterfindungsgemäße Punktmutationen. Dieser Ansatz zur Erzeugung letztlich erfindungsgemäßer Aminosäureaustausche baut auf den entsprechenden Nukleinsäuren auf, wobei es technisch grundsätzlich irrelevant ist, in welcher Reihenfolge diese verschiedenen Mutationen vorgenommen werden.

Einen weiteren Gegenstand der vorliegenden Erfindung bilden Vektoren, die eine zuvor bezeichnete erfindungsgemäße Nukleinsäure enthalten.

Denn um mit den erfindungsrelevanten Nukleinsäuren umzugehen, und damit insbesondere die erfindungsgemäßen Verfahren durchzuführen und Produktion erfindungsgemäßer Proteine vorzubereiten, werden sie geeigneterweise in Vektoren ligiert. Solche Vektoren sowie die zugehörigen Arbeitsmethoden sind im Stand der Technik ausführlich beschrieben. Vektoren sind in großer Zahl und Variationsbreite, sowohl für die Klonierung als auch für die Expression kommerziell erhältlich. Dazu gehören beispielsweise Vektoren, die sich von bakteriellen Plasmiden, von Bakteriophagen oder von Viren ableiten, oder überwiegend synthetische Vektoren. Ferner werden sie nach der Art der Zelltypen, in

denen sie sich zu etablieren vermögen, beispielsweise nach Vektoren für gramnegative, für grampositive Bakterien, für Hefen oder für höhere Eukaryonten unterschieden. Sie bilden geeignete Ausgangspunkte beispielsweise für molekularbiologische und biochemische Untersuchungen, für die Mutagenese der enthaltenen Nukleinsäureabschnitte sowie für die Expression des betreffenden Gens oder zugehörigen Proteins. Insbesondere zur Herstellung von Konstrukten zur Deletion oder Verstärkung der Expression sind sie - wie aus dem hierfür einschlägigen Stand der Technik hervorgeht - praktisch unerläßlich.

Vektoren sind besonders geeignet, um erfindungsgemäße Sequenzvariationen vorzunehmen, beispielsweise über ortsgerichtete Mutagenese, wie dies in Beispiel 3 dargestellt ist.

In einer Ausführungsform handelt es sich bei erfindungsgemäßen Vektoren um Klonierungsvektoren.

Denn Klonierungsvektoren eignen sich neben der Lagerung, der biologischen Amplifizierung oder der Selektion des interessierenden Gens für dessen molekularbiologische Charakterisierung. Gleichzeitig stellen sie transportierbare und lagerfähige Formen der beanspruchten Nukleinsäuren dar und sind auch Ausgangspunkte für molekularbiologische Techniken, die nicht an Zellen gebunden sind, wie beispielsweise die PCR oder *In-vitro*-Mutagenese-Verfahren.

Beispielsweise kann ein Klonierungsvektor, welcher das Gen für eine im Stand der Technik beschriebene α-Amylase trägt, durch Durchführung einer erfindungsgemäßen Mutation in einen erfindungsgemäßen Klonierungsvektor umgewandelt werden.

Vorzugsweise handelt es sich bei erfindungsgemäßen Vektoren um Expressionsvektoren.

Denn derartige Expressionsvektoren sind die Basis dafür, die entsprechenden Nukleinsäuren in biologischen Produktionssystemen zu realisieren und damit die zugehörigen Proteine zu produzieren, da sie *in vivo* die Transkription und Translation, das heißt die Synthese des betreffenden Genprodukts ermöglichen. Bevorzugte Ausführungsformen dieses Erfindungsgegenstands sind Expressionsvektoren, die die zur Expression notwendigen genetischen Elemente tragen, beispielsweise den natürlichen, ursprünglich vor diesem Gen lokalisierten Promotor oder einen Promotor aus einem anderen Organismus. Diese Elemente können beispielsweise in Form einer sogenannten Expressionskassette angeordnet sein. Alternativ können einzelne oder alle Regulationselemente auch von der jeweiligen Wirtszelle bereitgestellt werden. Besonders bevorzugt sind die Expressionsvektoren hinsichtlich weiterer Eigenschaften, wie beispielsweise die optimale Kopienzahl, auf das gewählte Expressionssystem, insbesondere die Wirtszelle (siehe unten) abgestimmt.

In der Regel ist die Bereitstellung eines Expressionsvektors die beste Möglichkeit, ein erfindungsgemäßes Protein vorzugsweise in Kombination mit damit zusammenwirkenden erfindungsgemäßen Proteinen verstärkt zu bilden und somit die betreffende Aktivität zu erhöhen beziehungsweise einer quantitativen Herstellung zugänglich zu machen.

Einen eigenen Erfindungsgegenstand bilden Zellen, die nach gentechnischer Modifizierung eine der zuvor bezeichneten erfindungsgemäßen Nukleinsäuren enthalten.

Denn diese Zellen enthalten die genetische Information zur Synthese eines erfindungsgemäßen Proteins und kommen dann in erfindungsgemäßen Verfahren zum Einsatz, wenn die betreffenden α-Amylasen biotechnologisch gewonnen werden. Hierunter sind insbesondere diejenigen Zellen gemeint, die nach an sich bekannten Verfahren mit den erfindungsgemäßen Nukleinsäuren versehen worden sind, beziehungsweise die sich von solchen Zellen ableiten. Dafür werden geeigneterweise solche Wirtszellen ausgewählt, die sich vergleichsweise einfach kultivieren lassen und/oder hohe Produktausbeuten liefern.

Sie ermöglichen beispielsweise die Amplifizierung der entsprechenden Gene, aber auch deren Mutagenese, wenn ein *In-vivo*-Mutagenese-Verfahren vorgenommen wird, oder Transkription und Translation und letztlich die biotechnologische Produktion der betreffenden Proteine. Diese genetische Information kann entweder extrachromosomal als eigenes genetisches Element, das heißt bei Bakterien in plasmidaler Lokalisation vorliegen oder in ein Chromosom integriert sein. Die Wahl eines geeigneten Systems hängt von Fragestellungen, wie beispielsweise die Art und Dauer der Lagerung des Gens, beziehungsweise des Organismus oder die Art der Mutagenese oder Selektion ab. Derartige Realisierungsmöglichkeiten sind an sich dem Molekularbiologen bekannt.

Daraus erklärt sich auch die bevorzugte Ausführungsform, nach welcher in einer solchen Zelle die genannte Nukleinsäure Teil eines Vektors ist, insbesondere eines zuvor beschriebenen, erfindungsgemäßen Vektors.

Denn damit sind die oben beschriebenen Vorteile bei der Handhabung, Lagerung, Expression etc. der betreffenden Nukleinsäure verbunden.

Bevorzugt ist unter diesen Zellen jeweils eine Wirtszelle, bei der es sich um ein Bakterium handelt, insbesondere eines, das die gebildete α-Amylase-Variante ins umgebende Medium sekretiert.

Denn Bakterien zeichnen sich durch kurze Generationszeiten und geringe Ansprüche an die Kultivierungsbedingungen aus. Dadurch können kostengünstige Verfahren etabliert werden. Zudem verfügt man bei Bakterien in der Fermentationstechnik über einen reichhaltigen Erfahrungsschatz. Für eine spezielle Produktion können aus verschiedensten, im Einzelfall experimentell zu ermittelnden Gründen wie Nährstoffquellen, Produktbildungsrate, Zeitbedarf etc. gramnegative oder grampositive Bakterien geeignet sein.

Eine zusätzlich vorteilhafte Ausführungsform nimmt darauf Bezug, daß die meisten industriell wichtigen α-Amylasen ursprünglich als Enzyme gefunden worden sind, die von den betreffenden Mikroorganismen, insbesondere

Bakterien ins umgebende Medium sekretiert werden. Denn *in vivo* handelt es sich zumeist um Verdauungsenzyme. Dementsprechend ist es vorteilhaft, wenn die industriell hergestellten erfindungsgemäßen Enzyme ebenfalls ins umgebende Medium sekretiert werden. Denn aus diesem können sie ohne Zellaufschluß und damit vergleichsweise leicht aufgearbeitet werden. Dies kann beispielsweise dadurch erreicht werden, daß die betreffenden Gene um entsprechende Sequenzen ergänzt werden - sofern diese nicht ohnehin vorhanden sind - die für ein Leader-Peptid codieren, welches die betreffende Zelle dazu veranlaßt sie auszuschleusen. Eine Alternative bei gramnegativen Bakterien besteht beispielsweise darin, die äußere Membran zur Abgabe von Proteinen partiell zu öffnen, wie dies beispielsweise in der Anmeldung WO 01/81597 A1 beschrieben ist.

In einer bevorzugten Ausführungsform handelt es sich um ein gramnegatives Bakterium, insbesondere eines der Gattungen *Escherichia coli, Klebsiella, Pseudomonas* oder *Xanthomonas,* insbesondere um Stämme von *E. coli* K12, *E. coli* B oder *Klebsiella planticola,* und ganz besonders um Derivate der Stämme *Escherichia coli* BL21 (DE3), E. *coli* RV308, *E. coli* DH5α, E.*coli* JM109, *E. coli* XL-1 oder *Klebsiella planticola* (Rf).

Denn diese Spezies und Stämme sind im Stand der Technik für molekularbiologische Arbeitsschritte und biotechnologische Herstellprozesse etabliert. Sie stehen zudem über allgemein zugängliche Stammsammlungen wie der Deutschen Sammlung von Mikroorganismen und Zellkulturen GmbH, Mascheroder Weg 1b, 38124 Braunschweig (http://www.dsmz.de) oder aus kommerziellen Quellen zur Verfügung. Sie sind darüber hinaus im Zusammenspiel mit ebenfalls in großer Zahl zur Verfügung stehenden übrigen Komponenten wie etwa Vektoren auf spezifische Herstellbedingungen hin optimierbar.

Bei gramnegativen Bakterien, wie beispielsweise *E. coli,* werden eine Vielzahl von Proteinen in den periplasmatischen Raum sekretiert. Dies kann für spezielle Anwendungen vorteilhaft sein. Wie erwähnt sind auch Verfahren bekannt, nach welchen auch gramnegative Bakterien die exprimierten Proteine ausschleusen.

In einer alternativen, nicht minder bevorzugten Ausführungsform handelt es sich um ein grampositives Bakterium, insbesondere eines der Gattungen *Bacillus, Staphylococcus* oder *Corynebakterium,* ganz besonders der Species *Bacillus lentus, B. licheniformis, B. amyloliquefaciens, B. subtilis, B. globigii* oder *B. alcalophilus, Staphylococcus carnosus* oder *Corynebacterium glutamicum,* und hierunter wiederum ganz besonders bevorzugt um ein Derivat von *B. licheniformis* DSM 13.

Denn grampositive Bakterien besitzen den gramnegativen gegenüber den grundsätzlichen Unterschied, sekretierte Proteine sogleich in das die Zellen umgebende Nährmedium abzugeben, aus welchem sich, wenn das gewünscht ist, die exprimierten erfindungsgemäßen Proteine direkt aufreinigen lassen. Zudem sind sie mit den meisten Herkunftsorganismen für technisch wichtige Enzyme verwandt oder identisch und bilden meist selbst vergleichbare Enzyme, so daß sie über eine ähnliche Codon-Usage verfügen und ihr Protein-Syntheseapparat naturgemäß entsprechend ausgerichtet ist. Der eine ganz besonders bevorzugte Ausführungsform kennzeichnende *B. licheniformis* DSM 13 ist ebenfalls im Stand der Technik als biotechnologischer Produktionsstamm weit verbreitet.

Eine weitere Ausführungsform der vorliegenden Erfindung bilden Verfahren zur Herstellung einer oben beschriebenen α-Amylase-Variante.

Die im engeren Sinne erfindungsgemäßen Verfahren zur Erhöhung der Stabilität einer α-Amylase gegenüber einer über elektrostatische Wechselwirkungen vermittelte Multimerisierung sind bereits oben beschrieben worden. Sie bezeichnen die in erster Linie molekularbiologischen Schritte, um derartige Varianten überhaupt erst einmal zu erzeugen. Die an dieser Stelle definierten, im weiteren Sinne ebenfalls erfindungsgemäßen Verfahren sind diejenigen, die technisch notwendig sind, um die erfindungsgemäßen α-Amylase-Varianten in quantitiven Mengen herzustellen. Es sind also - abgesehen von der eher nur theoretisch relevanten chemischen Synthese der Enzyme - in erster Linie biotechnologische Produktionsverfahren erfindungsgemäßer Varianten gemeint. In diese werden üblicherweise Mikroorganismenstämme eingebracht, die unter Anwendung an sich bekannter molekularbiologischer Techniken in die Lage versetzt worden sind, α-Amylase-Varianten zu produzieren, die im Laufe der vorliegenden Erfindung als erfindungsgemäß vorteilhaft erkannt worden sind.

Das oben ausgeführte erfindungsgemäße, insbesondere auf einer Mutagenese beruhende Verfahren kann somit als kennzeichnender Verfahrensabschnitt in ein etabliertes biotechnologisches Verfahren zur Herstellung von α-Amylasen eingebracht, das heißt mit einem solchen kombiniert werden.

Als Nachweis für die Bildung eines betreffenden Proteins in einem für die Herstellung eingesetzten Stamm dient ein enzymatischer Nachweis der betreffenden Enzymaktivitäten über an sich bekannte Nachweisreaktionen für α-Amylase-Aktivität. Als Nachweis auf molekularbiologischer Ebene können die im vorliegenden Sequenzprotokoll dargestellten Proteine nach üblichen Methoden synthetisiert und hiergegen Antikörper gebildet werden. Diese Proteine sind dann beispielsweise über entsprechende Western-Blots nachweisbar. Besonders bevorzugt reagieren diese Antikörper auf jene Oberflächenbereiche, die erfindungsgemäß modifiziert worden sind, weil darüber eine Unterscheidbarkeit gegenüber den nichterfindungsgemäßen Varianten gegeben ist.

Das Ziel dieser Verfahren besteht darin, die betreffenden α-Amylasen ihren jeweiligen Anwendungen zuzuführen. Hierfür kann auf alle in der Biotechnologie etablierten Aufarbeitungs-, Reinigungs- und Konfektionierungsschritte zurückgegriffen werden. Dazu gehört beispielsweise das in der Anmeldung WO 2004/067557 A1 beschriebene, auf

einer Chromatographie basierende Verfahren zur Veredelung konzentrierter Enzymlösungen, welches sich demzufolge auch erfolgreich auf Amylase-Präparationen anwenden läßt. Hierauf baut die nicht vorveröffentlichte Anmeldung DE 10360841.9 auf, nach welcher die betreffenden, durch unter anderem einen Chromatographie-Schritt erhaltenen Lösungen zu Enzymgranulaten weiterverarbeitet werden können. Weitere ebenfalls vorteilhaft mit der vorliegenden Erfindung zu kombinierende Verfahrensaspekte gehen aus den ebenfalls nicht vorveröffentlichten Anmeldungen DE 102004021384.4 und DE 102004019528 hervor, wonach die Lagerstabilität und Abriebfestigkeit derartiger Granulate durch Einarbeitung von Glycerin oder 1,2-Propandiol beziehungsweise durch eine spezielle Beschichtung erhöht werden können.

Diese Verfahren werden, insbesondere in den Teilschritten, zu denen die Enzympräparation noch in flüssiger Form vorliegt, erfindungsgemäß dadurch verbessert, daß die enthaltene erfindungsgemäße $\alpha$-Amylase-Variante weniger zur Aggregation über elektrostatische Wechselwirkungen neigt und somit ein geringerer Verlust an Gesamtaktivität eintritt. Dadurch können beispielsweise $\alpha$-Amylase-Granulate produziert werden, die einen höheren Anteil an Aktivsubstanz enthalten.

Vorzugsweise handelt es sich bei den Herstellverfahren um Verfahren, die unter Einsatz einer oben bezeichneten, erfindungsgemäßen Nukleinsäure erfolgen, vorzugsweise unter Einsatz eines zuvor bezeichneten erfindungsgemäßen Vektors und besonders bevorzugt unter Einsatz einer zuvor bezeichneten erfindungsgemäßen Zelle.

Denn durch die genannten Nukleinsäuren, insbesondere den konkreten im Sequenzprotokoll angegebenen Nukleinsäuren wird die entsprechend bevorzugte genetische Information in mikrobiologisch verwertbarer Form, das heißt für gentechnische Produktionsverfahren zur Verfügung gestellt. Zunehmend bevorzugt ist die Bereitstellung auf einem von der Wirtszelle besonders erfolgreich verwertbaren Vektor beziehungsweise von solchen Zellen selbst. Die betreffenden Produktionsverfahren sind dem Fachmann an sich bekannt.

Ausführungsformen der vorliegenden Erfindung können auf der Grundlage der zugehörigen Nukleinsäuresequenzen auch zellfreie Expressionssysteme sein, bei denen die Proteinbiosynthese *in vitro* nachvollzogen wird. Alle bereits oben ausgeführten Elemente können auch zu neuen Verfahren kombiniert werden, um erfindungsgemäße Proteine herzustellen. Es ist dabei für jedes erfindungsgemäße Protein eine Vielzahl von Kombinationsmöglichkeiten an Verfahrensschritten denkbar, so daß optimale Verfahren für jeden konkreten Einzelfall experimentell ermittelt werden müssen.

Weiterhin bevorzugt sind solche derartigen Verfahren, bei denen die Nukleotidsequenz in einem, vorzugsweise mehreren und besonders bevorzugt allen Codons an die Codon-Usage des Wirtsstamms angepaßt worden ist. Denn die Übertragung eines der genannten Gene in eine weniger verwandte Spezies kann dann besonders erfolgreich zur Synthese des betreffenden Proteins genutzt werden, wenn sie hinsichtlich des Gebrauchs der Codons entsprechend optimiert ist.

Einen eigenen Erfindungsgegenstand stellen Mittel dar, die eine oben beschriebene erfindungsgemäße $\alpha$-Amylase-Variante enthalten.

Hiermit ist jedes Mittel gemeint, in dem die betreffende $\alpha$-Amylase in irgendeiner Form zur Anwendung, das heißt in den erwünschten hydrolytischen Kontakt zu ihrem Substrat Stärke oder Stärke-ähnliches Polysaccharid gebracht oder hierfür vorbereitet wird. Der gewünschte Kontakt erfolgt in der Regel in einem wäßrigen Milieu, das günstigerweise auf einen entsprechenden pH-Wert gepuffert ist und gegebenenfalls weitere begünstigende Faktoren enthält. Hierzu gehören beispielsweise weitere Enzyme, die die unmittelbaren Reaktionsprodukte weiter umsetzen, etwa in Hinblick auf Stärkeverflüssigung für die Nahrungsmittel- oder Tierfutterherstellung oder für eine Ethanolproduktion. Ferner gehören hierzu niedermolekulare Verbindungen, die beispielsweise in entstehende Oligosaccharide wie Cyclodextrine eingeschlossen werden, oder niedermolekulare Verbindungen, die die Spaltungsprodukte weiter solubilisieren oder eine den Gesamtprozeß begünstigende Wirkung aufweisen, wie etwa Tenside im Rahmen einer Waschmittelrezeptur. Es kann sich auch um Mittel handeln, in denen die erwünschte amylolytische Aktivität erst mit einer großen Zeitverzögerung induziert werden soll, wie beispielsweise bei temporären Klebeverfahren, nach welchen die $\alpha$-Amylase-Variante dem betreffenden Klebstoff schon frühzeitig zugesetzt aber erst nach langer Zeit durch Erhöhung des Wassergehalts tatsächlich aktiv wird. Dies trifft beispielsweise auch auf Wasch- und Reinigungsmittel zu, die erst nach einer Phase der Lagerung im Augenblick der Verdünnung in der Waschflotte gegenüber dem Substrat aktiv werden sollen.

Eine Ausführungsform dieses Erfindungsgegenstands sind derartige Mittel, wobei es sich um ein Wasch- oder Reinigungsmittel handelt.

Die erfindungsgemäßen Eigenschaften und wichtigen Inhaltsstoffe für derartige Wasch- und Reinigungsmittel werden weiter unten ausführlich dargestellt. An dieser Stelle erfolgt zunächst eine Übersicht über die wichtigsten und deshalb erfindungsgemäß besonders bevorzugten Ausführungsformen derartiger Wasch- und Reinigungsmittel:

- Ein erfindungsgemäßes Wasch- oder Reinigungsmittel, enthaltend 0,000001 GewichtsProzent bis 5 Gew.-%, insbesondere 0,00001 bis 3 Gew.-% der $\alpha$-Amylase-Variante;
- ein erfindungsgemäßes Wasch- oder Reinigungsmittel, welches zusätzlich andere Enzyme enthält, insbesondere hydrolytische Enzyme oder Oxidoreduktasen, besonders bevorzugt weitere Amylasen, Proteasen, Lipasen, Cutinasen, Hemicellulasen, Cellulasen, β-Glucanasen, Oxidasen, Peroxidasen, Perhydrolasen und/oder Laccasen;

- ein erfindungsgemäßes Wasch- oder Reinigungsmittel, wobei die α-Amylase-Variante durch einen der sonstigen Bestandteile des Mittels stabilisiert und/oder in ihrem Beitrag zur Wasch-, beziehungsweise Reinigungsleistung des Mittels gesteigert wird;
- ein erfindungsgemäßes Wasch- oder Reinigungsmittel, welches insgesamt fest ist, vorzugsweise nach einem Kompaktierungsschritt für mindestens eine der enthaltenen Komponenten, besonders bevorzugt, daß es insgesamt kompaktiert ist;
- ein erfindungsgemäßes Wasch- oder Reinigungsmittel, welches insgesamt flüssig, gelförmig oder pastös ist, vorzugsweise unter Verkapselung für mindestens eine der enthaltenen Komponenten, besonders bevorzugt unter Verkapselung mindestens eines der enthaltenen Enzyme, ganz besonders bevorzugt unter Verkapselung der α-Amylase-Variante.

Ein wichtiges Einsatzgebiet für Amylasen ist das als aktive Komponenten in Wasch- oder Reinigungsmitteln zur Reinigung von Textilien oder von festen Oberflächen, wie beispielsweise Geschirr, Fußböden oder Arbeitsgeräten. In diesen Anwendungen dient die amylolytische Aktivität dazu, kohlenhydrathaltige Verunreinigungen und insbesondere solche auf Stärkebasis hydrolytisch aufzulösen oder vom Untergrund abzulösen. Dabei können sie allein, in geeigneten Medien oder auch in Wasch- oder Reinigungsmitteln zur Anwendung gebracht werden. Die hierfür zu wählenden Bedingungen, wie beispielsweise Temperatur, pH-Wert, Ionenstärke, Redox-Verhältnisse oder mechanische Einflüsse sollten für das jeweilige Reinigungsproblem optimiert sein, also in Bezug auf die Anschmutzung und auf das Trägermaterial. So liegen übliche Temperaturen für Wasch- und Reinigungsmittel in Bereichen von 10°C bei manuellen Mitteln über 40°C und 60°C bis hin zu 95° bei maschinellen Mitteln oder bei technischen Anwendungen. Da bei modernen Wasch- und Spülmaschinen die Temperatur meist stufenlos einstellbar ist, sind auch alle Zwischenstufen der Temperatur eingeschlossen. Vorzugsweise werden die Inhaltsstoffe der betreffenden Mittel aufeinander abgestimmt. Die übrigen Bedingungen können über die übrigen, unten ausgeführten Bestandteile der Mittel ebenfalls sehr spezifisch auf den jeweiligen Reinigungszweck ausgelegt werden.

Bevorzugte erfindungsgemäße Wasch- und Reinigungsmittel zeichnen sich dadurch aus, daß unter irgendwelchen der auf diese Weise definierbaren Bedingungen die Wasch- oder Reinigungsleistung dieses Mittels durch Zugabe einer erfindungsgemäßen α-Amylase-Variante gegenüber der Rezeptur ohne diese Variante verbessert wird. Bevorzugt sind Synergien hinsichtlich der Reinigungsleistung.

Eine erfindungsgemäße α-Amylase-Variante kann sowohl in Mitteln für Großverbraucher oder technische Anwender als auch in Produkten für den Privatverbraucher Anwendung finden, wobei alle im Stand der Technik etablierten und/oder zweckmäßigen Darreichungsformen auch Ausführungsformen der vorliegenden Erfindung darstellen. Mit den erfindungsgemäßen Wasch- oder Reinigungsmitteln sind somit alle denkbaren Reinigungsmittelarten gemeint, sowohl Konzentrate, als auch unverdünnt anzuwendende Mittel; zum Einsatz im kommerziellen Maßstab, in der Waschmaschine oder bei der Hand-Wäsche, beziehungsweise -Reinigung. Dazu gehören beispielsweise Waschmittel für Textilien, Teppiche, oder Naturfasern, für die nach der vorliegenden Erfindung die Bezeichnung Waschmittel verwendet wird. Dazu gehören beispielsweise auch Geschirrspülmittel für Geschirrspülmaschinen oder manuelle Geschirrspülmittel oder Reiniger für harte Oberflächen wie Metall, Glas, Porzellan, Keramik, Kacheln, Stein, lackierte Oberflächen, Kunststoffe, Holz oder Leder; für solche wird nach der vorliegenden Erfindung die Bezeichnung Reinigungsmittel verwendet. Jegliche Wasch- oder Reinigungsmittelart stellt eine Ausführungsform der vorliegenden Erfindung dar, sofern sie um eine erfindungsgemäße Amylase bereichert ist.

Ausführungsformen der vorliegenden Erfindung umfassen alle nach den Stand der Technik etablierten und/oder alle zweckmäßigen Darreichungsformen der erfindungsgemäßen Mittel. Dazu zählen beispielsweise feste, pulverförmige, flüssige, gelförmige oder pastöse Mittel, gegebenenfalls auch aus mehreren Phasen, komprimiert oder nicht komprimiert; ferner gehören beispielsweise dazu: Extrudate, Granulate, Tabletten oder Pouches, sowohl in Großgebinden als auch portionsweise abgepackt.

α-Amylasen, werden in erfindungsgemäßen Mitteln beispielsweise mit einzelnen oder mehreren der folgenden Inhaltsstoffe kombiniert: nichtionische, anionische und/oder kationische Tenside, Bleichmittel, Bleichaktivatoren, Bleichkatalysatoren, Builder und/oder Cobuilder, Lösungsmittel, Verdicker, Sequestrierungsmittel, Elektrolyte, optische Aufheller, Vergrauungsinhibitoren, Korrosionsinhibitoren, insbesondere Silberschutzmittel, Soil-Release-Wirkstoffe, Farbtransfer(oder -Übertragungs) -Inhibitoren, Schauminhibitoren, Abrasivstoffe, Farbstoffe, Duftstoffe, antimikrobielle Wirkstoffe, UV-Schutzmittel, Enzyme wie beispielsweise Proteasen, (gegebenenfalls andere) Amylasen, Lipasen, Cellulasen, Hemicellulasen oder Oxidasen, Stabilisatoren, insbesondere Enzymstabilisatoren, und andere Komponenten, die aus dem Stand der Technik bekannt sind.

Als nichtionische Tenside werden vorzugsweise alkoxylierte, vorteilhafterweise ethoxylierte, insbesondere primäre Alkohole mit vorzugsweise 8 bis 18 C-Atomen und durchschnittlich 1 bis 12 Mol Ethylenoxid (EO) pro Mol Alkohol eingesetzt, in denen der Alkoholrest linear oder bevorzugt in 2-Stellung methylverzweigt sein kann, beziehungsweise lineare und methylverzweigte Reste im Gemisch enthalten kann, so wie sie üblicherweise in Oxoalkoholresten vorliegen.

Insbesondere sind jedoch Alkoholethoxylate mit linearen Resten aus Alkoholen nativen Ursprungs mit 12 bis 18 C-Atomen, zum Beispiel aus Kokos-, Palm-, Talgfett- oder Oleylalkohol, und durchschnittlich 2 bis 8 EO pro Mol Alkohol bevorzugt. Zu den bevorzugten ethoxylierten Alkoholen gehören beispielsweise $C_{12-14}$-Alkohole mit 3 EO oder 4 EO, $C_{9-11}$-Alkohol mit 7 EO, $C_{13-15}$-Alkohole mit 3 EO, 5 EO, 7 EO oder 8 EO, $C_{12-18}$-Alkohole mit 3 EO, 5 EO oder 7 EO und Mischungen aus diesen, wie Mischungen aus $C_{12-14}$-Alkohol mit 3 EO und $C_{12-18}$-Alkohol mit 5 EO. Die angegebenen Ethoxylierungsgrade stellen statistische Mittelwerte dar, die für ein spezielles Produkt eine ganze oder eine gebrochene Zahl sein können. Bevorzugte Alkoholethoxylate weisen eine eingeengte Homologenverteilung auf (narrow range ethoxylates, NRE). Zusätzlich zu diesen nichtionischen Tensiden können auch Fettalkohole mit mehr als 12 EO eingesetzt werden. Beispiele hierfür sind Talgfettalkohol mit 14 EO, 25 EO, 30 EO oder 40 EO.

Eine weitere Klasse bevorzugt eingesetzter nichtionischer Tenside, die entweder als alleiniges nichtionisches Tensid oder in Kombination mit anderen nichtionischen Tensiden eingesetzt werden, sind alkoxylierte, vorzugsweise ethoxylierte oder ethoxylierte und propoxylierte Fettsäurealkylester, vorzugsweise mit 1 bis 4 Kohlenstoffatomen in der Alkylkette, insbesondere Fettsäuremethylester.

Aus der nicht vorveröffentlichten Anmeldung DE 102004020430.6 gehen Reinigungsmittel, insbesondere maschinelle Geschirrspülmittel hervor, die (a) ein oder mehrere nichtionische Tenside mit folgender allgemeinen Formel enthalten:

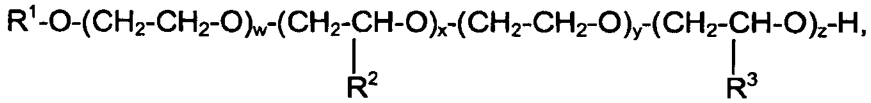

in der $R^1$ für einen $C_{6-24}$-Alkyl- oder -Alkenylrest, die Gruppen $R^2$ und $R^3$ jeweils für bestimmte Kohlenwasserstoffreste und die Indizes w, x, y, z jeweils für ganze Zahlen bis 6 stehen, oder ein Tensidsystem aus mindestens einem nichtionischen Tensid F der allgemeinen Formel:

$$R^1\text{-}CH(OH)CH_2O\text{-}(AO)_w\text{-}(A'O)_x\text{-}(A''O)_y\text{-}(A'''O)_z\text{-}R^2$$

und mindestens einem nichtionischen Tensid G der allgemeinen Formel:

$$A^1\text{-}O\text{-}(AO)_w\text{-}(A'O)_xA''O)_y\text{-}(A'''O)_z\text{-}R^2$$

in denen $R^1$ für einen $C_{6-24}$-Alkyl- oder -Alkenylrest, $R^2$ für einen Kohlenwasserstoffrest mit 2 bis 26 Kohlenstoffatomen, A, A', A" und A"' jeweils für bestimmte Kohlenwasserstoffreste und w, x, y und z jeweils für Werte bis zu 25 stehen, wobei dieses Tensidsystem die Tenside F und G in einem Gewichtsverhältnis zwischen 1:4 und 100:1 aufweist, und als Komponente (b) eine spezielle $\alpha$-Amylase-Variante.

Diese Tenside sind insbesondere in maschinellen Geschirrspülmitteln auch mit $\alpha$-Amylasen kombinierbar, die der vorliegenden Erfindung entsprechen, insbesondere wenn sie neben den erfindungsgemäßen Austauschen solche aufweisen, die einer oder mehrerer der Anmeldungen WO 96/23873 A1, WO 00/60060 A2 und WO 01/66712 A2 zu entnehmen sind. Dies gilt insbesondere für den Fall, daß das unter diese Anmeldungen fallende Handelsprodukt Stainzyme® der Fa. Novozymes in weiteren Positionen verbessert und zusätzlich mit mindestens einem erfindungsgemäßen Autausch versehen wird. Denn prinzipiell muß von einer additiven Wirkung der verschiedenen Modifikationen ausgegangen werden.

Eine weitere Klasse von nichtionischen Tensiden, die vorteilhafterweise eingesetzt werden können, sind die Alkylpolyglycoside (APG). Einsetzbare Alkypolyglycoside genügen der allgemeinen Formel $RO(G)_z$, in der R für einen linearen oder verzweigten, insbesondere in 2-Stellung methylverzweigten, gesättigten oder ungesättigten, aliphatischen Rest mit 8 bis 22, vorzugsweise 12 bis 18 C-Atomen bedeutet und G das Symbol ist, das für eine Glykoseeinheit mit 5 oder 6 C-Atomen, vorzugsweise für Glucose, steht. Der Glycosylierungsgrad z liegt dabei zwischen 1,0 und 4,0, vorzugsweise zwischen 1,0 und 2,0 und insbesondere zwischen 1,1 und 1,4. Bevorzugt eingesetzt werden lineare Alkylpolyglucoside, also Alkylpolyglycoside, in denen der Polyglycosylrest ein Glucoserest und der Alkylrest ein n-Alkylrest ist.

Auch nichtionische Tenside vom Typ der Aminoxide, beispielsweise N-Kokosalkyl-N,N-dimethylaminoxid und N-Talgalkyl-N,N-dihydroxyethylaminoxid, und der Fettsäurealkanolamide können geeignet sein. Der Anteil dieser nichtionischen Tenside liegt vorzugsweise nicht über dem der ethoxylierten Fettalkohole, insbesondere bei nicht mehr als der Hälfte davon.

Weitere geeignete Tenside sind Polyhydroxyfettsäureamide der Formel (II),

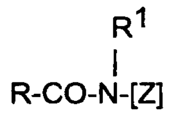

(II)

in der RCO für einen aliphatischen Acylrest mit 6 bis 22 Kohlenstoffatomen, $R^1$ für Wasserstoff, einen Alkyl- oder Hydroxyalkylrest mit 1 bis 4 Kohlenstoffatomen und [Z] für einen linearen oder verzweigten Polyhydroxyalkylrest mit 3 bis 10 Kohlenstoffatomen und 3 bis 10 Hydroxylgruppen steht. Bei den Polyhydroxyfettsäureamiden handelt es sich um bekannte Stoffe, die üblicherweise durch reduktive Aminierung eines reduzierenden Zuckers mit Ammoniak, einem Alkylamin oder einem Alkanolamin und nachfolgende Acylierung mit einer Fettsäure, einem Fettsäurealkylester oder einem Fettsäurechlorid erhalten werden können.

Zur Gruppe der Polyhydroxyfettsäureamide gehören auch Verbindungen der Formel (III),

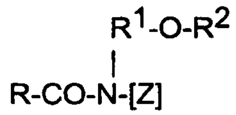

(III)

in der R für einen linearen oder verzweigten Alkyl- oder Alkenylrest mit 7 bis 12 Kohlenstoffatomen, $R^1$ für einen linearen, verzweigten oder cyclischen Alkylrest oder einen Arylrest mit 2 bis 8 Kohlenstoffatomen und $R^2$ für einen linearen, verzweigten oder cyclischen Alkylrest oder einen Arylrest oder einen Oxy-Alkylrest mit 1 bis 8 Kohlenstoffatomen steht, wobei $C_{1-4}$-Alkyl- oder Phenylreste bevorzugt sind und [Z] für einen linearen Polyhydroxyalkylrest steht, dessen Alkylkette mit mindestens zwei Hydroxylgruppen substituiert ist, oder alkoxylierte, vorzugsweise ethoxylierte oder propoxylierte Derivate dieses Restes.

[Z] wird vorzugsweise durch reduktive Aminierung eines reduzierenden Zuckers erhalten, beispielsweise Glucose, Fructose, Maltose, Lactose, Galactose, Mannose oder Xylose. Die N-Alkoxy- oder N-Aryloxy-substituierten Verbindungen können beispielsweise durch Umsetzung mit Fettsäuremethylestern in Gegenwart eines Alkoxids als Katalysator in die gewünschten Polyhydroxyfettsäureamide überführt werden.

Als anionische Tenside werden beispielsweise solche vom Typ der Sulfonate und Sulfate eingesetzt. Als Tenside vom Sulfonat-Typ kommen dabei vorzugsweise $C_{9-13}$-Alkylbenzolsulfonate, Olefinsulfonate, das heißt Gemische aus Alken- und Hydroxyalkansulfonaten sowie Disulfonaten, wie man sie beispielsweise aus $C_{12-18}$-Monoolefinen mit end- oder innenständiger Doppelbindung durch Sulfonieren mit gasförmigem Schwefeltrioxid und anschließende alkalische oder saure Hydrolyse der Sulfonierungsprodukte erhält, in Betracht. Geeignet sind auch Alkansulfonate, die aus $C_{12-18}$-Alkanen beispielsweise durch Sulfochlorierung oder Sulfoxidation mit anschließender Hydrolyse beziehungsweise Neutralisation gewonnen werden. Ebenso sind auch die Ester von $\alpha$-Sulfofettsäuren (Estersulfonate), zum Beispiel die $\alpha$-sulfonierten Methylester der hydrierten Kokos-, Palmkern- oder Talgfettsäuren geeignet.

Weitere geeignete Aniontenside sind sulfierte Fettsäureglycerinester. Unter Fettsäureglycerinestern sind die Mono-, Di- und Triester sowie deren Gemische zu verstehen, wie sie bei der Herstellung durch Veresterung von einem Monoglycerin mit 1 bis 3 Mol Fettsäure oder bei der Umesterung von Triglyceriden mit 0,3 bis 2 Mol Glycerin erhalten werden. Bevorzugte sulfierte Fettsäureglycerinester sind dabei die Sulfierprodukte von gesättigten Fettsäuren mit 6 bis 22 Kohlenstoffatomen, beispielsweise der Capronsäure, Caprylsäure, Caprinsäure, Myristinsäure, Laurinsäure, Palmitinsäure, Stearinsäure oder Behensäure.

Als Alk(en)ylsulfate werden die Alkali- und insbesondere die Natriumsalze der Schwefelsäurehalbester der $C_{12}$-$C_{18}$-Fettalkohole, beispielsweise aus Kokosfettalkohol, Talgfettalkohol, Lauryl-, Myristyl-, Cetyl- oder Stearylalkohol oder der $C_{10}$-$C_{20}$-Oxoalkohole und diejenigen Halbester sekundärer Alkohole dieser Kettenlängen bevorzugt. Weiterhin bevorzugt sind Alk(en)ylsulfate der genannten Kettenlänge, welche einen synthetischen, auf petrochemischer Basis hergestellten geradkettigen Alkylrest enthalten, die ein analoges Abbauverhalten besitzen wie die adäquaten Verbindungen auf der Basis von fettchemischen Rohstoffen. Aus waschtechnischem Interesse sind die $C_{12}$-$C_{16}$-Alkylsulfate und $C_{12}$-$C_{15}$-Alkylsulfate sowie $C_{14}$-$C_{15}$-Alkylsulfate bevorzugt. Auch 2,3-Alkylsulfate sind geeignete Aniontenside.

Auch die Schwefelsäuremonoester der mit 1 bis 6 Mol Ethylenoxid ethoxylierten geradkettigen oder verzweigten $C_{7-21}$-Alkohole, wie 2-Methyl-verzweigte $C_{9-11}$-Alkohole mit im Durchschnitt 3,5 Mol Ethylenoxid (EO) oder $C_{12-18}$-Fettalkohole mit 1 bis 4 EO, sind geeignet. Sie werden in Reinigungsmitteln aufgrund ihres hohen Schaumverhaltens nur in relativ geringen Mengen, beispielsweise in Mengen bis 5 Gew.-%, üblicherweise von 1 bis 5 Gew.-%, eingesetzt.

Weitere geeignete Aniontenside sind auch die Salze der Alkylsulfobernsteinsäure, die auch als Sulfosuccinate oder als Sulfobernsteinsäureester bezeichnet werden und die Monoester und/oder Diester der Sulfobernsteinsäure mit Alkoholen, vorzugsweise Fettalkoholen und insbesondere ethoxylierten Fettalkoholen darstellen. Bevorzugte Sulfosuccinate enthalten $C_{8-18}$-Fettalkoholreste oder Mischungen aus diesen. Insbesondere bevorzugte Sulfosuccinate enthalten einen Fettalkoholrest, der sich von ethoxylierten Fettalkoholen ableitet, die für sich betrachtet nichtionische Tenside

darstellen (Beschreibung siehe unten). Dabei sind wiederum Sulfosuccinate, deren Fettalkohol-Reste sich von ethoxylierten Fettalkoholen mit eingeengter Homologenverteilung ableiten, besonders bevorzugt. Ebenso ist es auch möglich, Alk(en)ylbernsteinsäure mit vorzugsweise 8 bis 18 Kohlenstoffatomen in der Alk(en)ylkette oder deren Salze einzusetzen.

Als weitere anionische Tenside kommen insbesondere Seifen in Betracht. Geeignet sind gesättigte Fettsäureseifen, wie die Salze der Laurinsäure, Myristinsäure, Palmitinsäure, Stearinsäure, hydrierte Erucasäure und Behensäure sowie insbesondere aus natürlichen Fettsäuren, zum Beispiel Kokos-, Palmkern- oder Talgfettsäuren, abgeleitete Seifengemische.

Die anionischen Tenside einschließlich der Seifen können in Form ihrer Natrium-, Kalium- oder Ammoniumsalze sowie als lösliche Salze organischer Basen, wie Mono-, Di- oder Triethanolamin, vorliegen. Vorzugsweise liegen die anionischen Tenside in Form ihrer Natrium- oder Kaliumsalze, insbesondere in Form der Natriumsalze vor.

Die Tenside können in den erfindungsgemäßen Reinigungs- oder Waschmitteln insgesamt in einer Menge von vorzugsweise 5 Gew.-% bis 50 Gew.-%, insbesondere von 8 Gew.-% bis 30 Gew.-%, bezogen auf das fertige Mittel, enthalten sein.

Erfindungsgemäße Mittel können Bleichmittel enthalten. Unter den als Bleichmittel dienenden, in Wasser $H_2O_2$ liefernden Verbindungen haben das Natriumpercarbonat, das Natriumperborattetrahydrat und das Natriumperboratmonohydrat besondere Bedeutung. Weitere brauchbare Bleichmittel sind beispielsweise Peroxopyrophosphate, Citratperhydrate sowie $H_2O_2$ liefernde persaure Salze oder Persäuren, wie Persulfate beziehungsweise Perschwefelsäure. Brauchbar ist auch das Harnstoffperoxohydrat Percarbamid, das durch die Formel $H_2N\text{-}CO\text{-}NH_2 \cdot H_2O_2$ beschrieben werden kann. Insbesondere beim Einsatz der Mittel für das Reinigen harter Oberflächen, zum Beispiel beim maschinellen Geschirrspülen, können sie gewünschtenfalls auch Bleichmittel aus der Gruppe der organischen Bleichmittel enthalten, obwohl deren Einsatz prinzipiell auch bei Mitteln für die Textilwäsche möglich ist. Typische organische Bleichmittel sind die Diacylperoxide, wie zum Beispiel Dibenzoylperoxid. Weitere typische organische Bleichmittel sind die Peroxysäuren, wobei als Beispiele besonders die Alkylperoxysäuren und die Arylperoxysäuren genannt werden. Bevorzugte Vertreter sind die Peroxybenzoesäure und ihre ringsubstituierten Derivate, wie Alkylperoxybenzoesäuren, aber auch Peroxy-$\alpha$-Naphthoesäure und Magnesium-monoperphthalat, die aliphatischen oder substituiert aliphatischen Peroxysäuren, wie Peroxylaurinsäure, Peroxystearinsäure, $\varepsilon$-Phthalimidoperoxycapronsäure (Phthalimidoperoxyhexansäure, PAP), o-Carboxybenzamidoperoxycapronsäure, N-Nonenylamidoperadipinsäure und N-Nonenylamidopersuccinate, und aliphatische und araliphatische Peroxydicarbonsäuren, wie 1,12-Diperoxycarbonsäure, 1,9-Diperoxyazelainsäure, Diperoxysebacinsäure, Diperoxybrassylsäure, die Diperoxyphthalsäuren, 2-Decyldiperoxybutan-1,4-disäure, N,N-Terephthaloyldi(6-aminopercapronsäure) können eingesetzt werden.

Der Gehalt der Mittel an Bleichmittel kann 1 bis 40 Gew.-% und insbesondere 10 bis 20 Gew.-%, betragen, wobei vorteilhafterweise Perboratmonohydrat oder Percarbonat eingesetzt wird. Eine synergistische Verwendung von Amylase mit Percarbonat oder von Amylase mit Percarbonsäure wird mit den Anmeldungen WO 99/63036, beziehungsweise WO 99/63037 offenbart.

Um beim Waschen bei Temperaturen von 60°C und darunter, und insbesondere bei der Wäschevorbehandlung eine verbesserte Bleichwirkung zu erreichen, können die Mittel auch Bleichaktivatoren enthalten. Als Bleichaktivatoren können Verbindungen, die unter Perhydrolysebedingungen aliphatische Peroxocarbonsäuren mit vorzugsweise 1 bis 10 C-Atomen, insbesondere 2 bis 4 C-Atomen, und/oder gegebenenfalls substituierte Perbenzoesäure ergeben, eingesetzt werden. Geeignet sind Substanzen, die O- und/oder N-Acylgruppen der genannten C-Atomzahl und/oder gegebenenfalls substituierte Benzoylgruppen tragen. Bevorzugt sind mehrfach acylierte Alkylendiamine, insbesondere Tetraacetylethylendiamin (TAED), acylierte Triazinderivate, insbesondere 1,5-Diacetyl-2,4-dioxohexahydro-1,3,5-triazin (DADHT), acylierte Glycolurile, insbesondere 1,3,4,6-Tetraacetylglycoluril (TAGU), N-Acylimide, insbesondere N-Nonanoylsuccinimid (NOSI), acylierte Phenolsulfonate, insbesondere n-Nonanoyl- oder Isononanoyloxybenzolsulfonat (n- beziehungsweise iso-NOBS), acylierte Hydroxycarbonsäuren, wie Triethyl-O-acetylcitrat (TEOC), Carbonsäureanhydride, insbesondere Phthalsäureanhydrid, Isatosäureanhydrid und/oder Bernsteinsäureanhydrid, Carbonsäureamide, wie N-Methyldiacetamid, Glycolid, acylierte mehrwertige Alkohole, insbesondere Triacetin, Ethylenglycoldiacetat, Isopropenylacetat, 2,5-Diacetoxy-2,5-dihydrofuran und die aus den deutschen Patentanmeldungen DE 196 16 693 und DE 196 16 767 bekannten Enolester sowie acetyliertes Sorbitol und Mannitol beziehungsweise deren in der europäischen Patentanmeldung EP 0 525 239 beschriebene Mischungen (SORMAN), acylierte Zuckerderivate, insbesondere Pentaacetylglucose (PAG), Pentaacetylfructose, Tetraacetylxylose und Octaacetyllactose sowie acetyliertes, gegebenenfalls N-alkyliertes Glucamin beziehungsweise Gluconolacton, Triazol beziehungsweise Triazolderivate und/oder teilchenförmige Caprolactame und/oder Caprolactamderivate, bevorzugt N-acylierte Lactame, beispielsweise N-Benzoylcaprolactam und N-Acetylcaprolactam. Hydrophil substituierte Acylacetale und Acyllactame werden ebenfalls bevorzugt eingesetzt. Auch Kombinationen konventioneller Bleichaktivatoren können eingesetzt werden. Ebenso können Nitrilderivate wie Cyanopyridine, Nitrilquats, zum Beispiel N-Alkylammoniumacetonitrile, und/oder Cyanamidderivate eingesetzt werden. Bevorzugte Bleichaktivatoren sind Natrium-4-(octanoyloxy)-benzolsulfonat, n-Nonanoyl- oder Isononanoyloxybenzolsulfonat (n- beziehungsweise iso-NOBS), Undecenoyloxybenzolsulfonat (UDOBS), Natriumdodecanoyloxybenzolsulfonat (DOBS), Decanoyloxybenzoesäure (DOBA, OBC 10) und/oder Dodecanoyloxybenzolsulfonat (OBS 12), sowie

N-Methylmorpholinum-acetonitril (MMA). Derartige Bleichaktivatoren können im üblichen Mengenbereich von 0,01 bis 20 Gew.-%, vorzugsweise in Mengen von 0,1 bis 15 Gew.%, insbesondere 1 Gew.-% bis 10 Gew.%, bezogen auf die gesamte Zusammensetzung, enthalten sein.

Zusätzlich zu den konventionellen Bleichaktivatoren oder an deren Stelle können auch sogenannte Bleichkatalysatoren enthalten sein. Bei diesen Stoffen handelt es sich um bleichverstärkende Übergangsmetallsalze beziehungsweise Übergangsmetallkomplexe wie beispielsweise Mn-, Fe-, Co-, Ru - oder Mo-Salenkomplexe oder -carbonylkomplexe. Auch Mn-, Fe-, Co-, Ru-, Mo-, Ti-, V- und Cu-Komplexe mit N-haltigen Tripod-Liganden sowie Co-, Fe-, Cu- und Ru-Aminkomplexe sind als Bleichkatalysatoren geeignet. Gemäß WO 99/63038 vermögen auch Acetonitril-Derivate und gemäß WO 99/63041 bleichaktivierende Übergangsmetallkomplexverbindungen in Kombination mit Amylasen eine bleichaktivierende Wirkung zu entfalten.

Erfindungsgemäße Mittel enthalten in der Regel einen oder mehrere Builder, insbesondere Zeolithe, Silikate, Carbonate, organische Cobuilder und - wo keine ökologischen Gründe gegen ihren Einsatz sprechen - auch die Phosphate. Letztere sind insbesondere in Reinigungsmitteln für das maschinelle Geschirrspülen bevorzugt einzusetzende Gerüststoffe.

Zu nennen sind hier kristalline, schichtförmige Natriumsilikate der allgemeinen Formel $NaMSi_xO_{2x+1} \cdot yH_2O$, wobei M Natrium oder Wasserstoff bedeutet, x eine Zahl von 1,6 bis 4, vorzugsweise 1,9 bis 4,0 und y eine Zahl von 0 bis 20 ist und bevorzugte Werte für x 2, 3 oder 4 sind. Bevorzugte kristalline Schichtsilicate der angegebenen Formel sind solche, in denen M für Natrium steht und x die Werte 2 oder 3 annimmt. Insbesondere sind sowohl β- als auch δ-Natriumdisilicate $Na_2Si_2O_5 \cdot yH_2O$ bevorzugt. Im Handel befinden sich derartige Verbindungen beispielsweise unter der Bezeichnung SKS® (Fa. Clariant). So handelt es sich bei SKS-6® vorwiegend um ein δ-Natriumdisilicat mit der Formel $Na_2Si_2O_5 \cdot yH_2O$, bei SKS-7® vorwiegend um das β-Natriumdisilicat. Durch Reaktion mit Säuren (zum Beispiel Citronensäure oder Kohlensäure) entsteht aus dem δ-Natriumdisilicat Kanemit $NaHSi_2O_5 \cdot yH_2O$, im Handel unter den Bezeichnungen SKS-9® beziehungsweise SKS-10® (Fa. Clariant). Von Vorteil kann es auch sein, chemische Modifikationen dieser Schichtsilicate einzusetzen. So kann beispielsweise die Alkalität der Schichtsilicate geeignet beeinflußt werden. Mit Phosphat beziehungsweise mit Carbonat dotierte Schichtsilicate weisen im Vergleich zu dem δ-Natriumdisilicat veränderte Kristallmorphologien auf, lösen sich schneller und zeigen im Vergleich zu δ-Natriumdisilicat ein erhöhtes Calciumbindevermögen. Besonders zu nennen sind Schichtsilicate der allgemeinen Summenformel x $Na_2O$ • y $SiO_2$ • $P_2O_5$, in der das Verhältnis x zu y einer Zahl 0,35 bis 0,6, das Verhältnis x zu z einer Zahl von 1,75 bis 1200 und das Verhältnis y zu z einer Zahl von 4 bis 2800 entsprechen. Die Löslichkeit der Schichtsilicate kann auch erhöht werden, indem besonders feinteilige Schichtsilicate eingesetzt werden. Auch Compounds aus den kristallinen Schichtsilicaten mit anderen Inhaltsstoffen können eingesetzt werden. Dabei sind insbesondere Compounds mit Cellulosederivaten, die Vorteile in der desintegrierenden Wirkung aufweisen und insbesondere in Waschmitteltabletten eingesetzt werden, sowie Compounds mit Polycarboxylaten, zum Beispiel Citronensäure, beziehungsweise polymeren Polycarboxylaten, zum Beispiel Copolymeren der Acrylsäure, zu nennen.

Einsetzbar sind auch amorphe Natriumsilikate mit einem Modul $Na_2O : SiO_2$ von 1:2 bis 1:3,3, vorzugsweise von 1:2 bis 1:2,8 und insbesondere von 1:2 bis 1:2,6, welche löseverzögert sind und Sekundärwascheigenschaften aufweisen. Die Löseverzögerung gegenüber herkömmlichen amorphen Natriumsilikaten kann dabei auf verschiedene Weise, beispielsweise durch Oberflächenbehandlung, Compoundierung, Kompaktierung/ Verdichtung oder durch Übertrocknung hervorgerufen worden sein. Im Rahmen dieser Erfindung wird unter dem Begriff "amorph" auch "röntgenamorph" verstanden. Dies heißt, daß die Silikate bei Röntgenbeugungsexperimenten keine scharfen Röntgenreflexe liefern, wie sie für kristalline Substanzen typisch sind, sondern allenfalls ein oder mehrere Maxima der gestreuten Röntgenstrahlung, die eine Breite von mehreren Gradeinheiten des Beugungswinkels aufweisen. Es kann jedoch sehr wohl sogar zu besonders guten Buildereigenschaften führen, wenn die Silikatpartikel bei Elektronenbeugungsexperimenten verwaschene oder sogar scharfe Beugungsmaxima liefern. Dies ist so zu interpretieren, daß die Produkte mikrokristalline Bereiche der Größe 10 bis einige Hundert nm aufweisen, wobei Werte bis max. 50 nm und insbesondere bis max. 20 nm bevorzugt sind. Insbesondere bevorzugt sind verdichtete/kompaktierte amorphe Silikate, compoundierte amorphe Silikate und übertrocknete röntgenamorphe Silikate.

Ein gegebenenfalls einsetzbarer, feinkristalliner, synthetischer und gebundenes Wasser enthaltender Zeolith ist vorzugsweise Zeolith A und/oder P. Als Zeolith P wird Zeolith MAP® (Handelsprodukt der Firma Crosfield) besonders bevorzugt. Geeignet sind jedoch auch Zeolith X sowie Mischungen aus A, X und/oder P. Kommerziell erhältlich und im Rahmen der vorliegenden Erfindung bevorzugt einsetzbar ist beispielsweise auch ein Co-Kristallisat aus Zeolith X und Zeolith A (ca. 80 Gew.-% Zeolith X), das von der Firma CONDEA Augusta S.p.A. unter dem Markennamen VEGOBOND AX® vertrieben wird und durch die Formel

$$nNa_2O \cdot (1\text{-}n)K_2O \cdot Al_2O_3\ (2 - 2{,}5)SiO_2\ (3{,}5 - 5{,}5)\ H_2O$$

beschrieben werden kann. Geeignete Zeolithe weisen eine mittlere Teilchengröße von weniger als 10 μm (Volumenverteilung; Meßmethode: Coulter Counter) auf und enthalten vorzugsweise 18 bis 22 Gew.-%, insbesondere 20 bis 22

Gew.-% an gebundenem Wasser.

Selbstverständlich ist auch ein Einsatz der allgemein bekannten Phosphate als Buildersubstanzen möglich, sofern ein derartiger Einsatz nicht aus ökologischen Gründen vermieden werden sollte. Unter der Vielzahl der kommerziell erhältlichen Phosphate haben die Alkalimetallphosphate unter besonderer Bevorzugung von Pentanatriumbeziehungsweise Pentakaliumtriphosphat (Natrium- beziehungsweise Kaliumtripolyphosphat) in der Wasch- und Reinigungsmittel-Industrie die größte Bedeutung.

Alkalimetallphosphate ist dabei die summarische Bezeichnung für die Alkalimetall-(insbesondere Natrium- und Kalium-) -Salze der verschiedenen Phosphorsäuren, bei denen man Metaphosphorsäuren $(HPO_3)_n$ und Orthophosphorsäure $H_3PO_4$ neben höhermolekularen Vertretern unterscheiden kann. Die Phosphate vereinen dabei mehrere Vorteile in sich: Sie wirken als Alkaliträger, verhindern Kalkbeläge auf Maschinenteilen beziehungsweise Kalkinkrustationen in Geweben und tragen überdies zur Reinigungsleistung bei.

Natriumdihydrogenphosphat, $NaH_2PO_4$, existiert als Dihydrat (Dichte 1,91 $gcm^{-3}$, Schmelzpunkt 60°) und als Monohydrat (Dichte 2,04 $gcm^{-3}$). Beide Salze sind weiße, in Wasser sehr leicht lösliche Pulver, die beim Erhitzen das Kristallwasser verlieren und bei 200°C in das schwach saure Diphosphat (Dinatriumhydrogendiphosphat, $Na_2H_2P_2O_7$), bei höherer Temperatur in Natiumtrimetaphosphat ($Na_3P_3O_9$) und Maddrellsches Salz (siehe unten), übergehen. $NaH_2PO_4$ reagiert sauer; es entsteht, wenn Phosphorsäure mit Natronlauge auf einen pH-Wert von 4,5 eingestellt und die Maische versprüht wird. Kaliumdihydrogenphosphat (primäres oder einbasiges Kaliumphosphat, Kaliumbiphosphat, KDP), $KH_2PO_4$, ist ein weißes Salz der Dichte 2,33 $gcm^{-3}$, hat einen Schmelzpunkt 253° [Zersetzung unter Bildung von Kaliumpolyphosphat $(KPO_3)_x$] und ist leicht löslich in Wasser.

Dinatriumhydrogenphosphat (sekundäres Natriumphosphat), $Na_2HPO_4$, ist ein farbloses, sehr leicht wasserlösliches kristallines Salz. Es existiert wasserfrei und mit 2 Mol. (Dichte 2,066 $gcm^{-3}$, Wasserverlust bei 95°), 7 Mol. (Dichte 1,68 $gcm^{-3}$, Schmelzpunkt 48° unter Verlust von 5 $H_2O$) und 12 Mol. Wasser (Dichte 1,52 $gcm^{-3}$, Schmelzpunkt 35° unter Verlust von 5 $H_2O$), wird bei 100° wasserfrei und geht bei stärkerem Erhitzen in das Diphosphat $Na_4P_2O_7$ über. Dinatriumhydrogenphosphat wird durch Neutralisation von Phosphorsäure mit Sodalösung unter Verwendung von Phenolphthalein als Indikator hergestellt. Dikaliumhydrogenphosphat (sekundäres od. zweibasiges Kaliumphosphat), $K_2HPO_4$, ist ein amorphes, weißes Salz, das in Wasser leicht löslich ist.

Trinatriumphosphat, tertiäres Natriumphosphat, $Na_3PO_4$, sind farblose Kristalle, die als Dodecahydrat eine Dichte von 1,62 $gcm^{-3}$ und einen Schmelzpunkt von 73-76°C (Zersetzung), als Decahydrat (entsprechend 19-20% $P_2O_5$) einen Schmelzpunkt von 100°C und in wasserfreier Form (entsprechend 39-40% $P_2O_5$) eine Dichte von 2,536 $gcm^{-3}$ aufweisen. Trinatriumphosphat ist in Wasser unter alkalischer Reaktion leicht löslich und wird durch Eindampfen einer Lösung aus genau 1 Mol Dinatriumphosphat und 1 Mol NaOH hergestellt. Trikaliumphosphat (tertiäres oder dreibasiges Kaliumphosphat), $K_3PO_4$, ist ein weißes, zerfließliches, körniges Pulver der Dichte 2,56 $gcm^{-3}$, hat einen Schmelzpunkt von 1340° und ist in Wasser mit alkalischer Reaktion leicht löslich. Es entsteht zum Beispiel beim Erhitzen von Thomasschlacke mit Kohle und Kaliumsulfat. Trotz des höheren Preises werden in der Reinigungsmittel-Industrie die leichter löslichen, daher hochwirksamen, Kaliumphosphate gegenüber entsprechenden Natrium-Verbindungen vielfach bevorzugt.

Tetranatriumdiphosphat (Natriumpyrophosphat), $Na_4P_2O_7$, existiert in wasserfreier Form (Dichte 2,534 $gcm^{-3}$, Schmelzpunkt 988°, auch 880° angegeben) und als Decahydrat (Dichte 1,815-1,836 $gcm^{-3}$, Schmelzpunkt 94° unter Wasserverlust). Beide Substanzen sind farblose, in Wasser mit alkalischer Reaktion lösliche Kristalle. $Na_4P_2O_7$ entsteht beim Erhitzen von Dinatriumphosphat auf >200°C oder indem man Phosphorsäure mit Soda im stöchiometrischem Verhältnis umsetzt und die Lösung durch Versprühen entwässert. Das Decahydrat komplexiert Schwermetall-Salze und Härtebildner und verringert daher die Härte des Wassers. Kaliumdiphosphat (Kaliumpyrophosphat), $K_4P_2O_7$, existiert in Form des Trihydrats und stellt ein farbloses, hygroskopisches Pulver mit der Dichte 2,33 $gcm^{-3}$ dar, das in Wasser löslich ist, wobei der pH-Wert der 1%igen Lösung bei 25° 10,4 beträgt.

Durch Kondensation des $NaH_2PO_4$ beziehungsweise des $KH_2PO_4$ entstehen höhermolekulare Natrium- und Kaliumphosphate, bei denen man cyclische Vertreter, die Natrium- beziehungsweise Kaliummetaphosphate und kettenförmige Typen, die Natriumbeziehungsweise Kaliumpolyphosphate, unterscheiden kann. Insbesondere für letztere sind eine Vielzahl von Bezeichnungen in Gebrauch: Schmelz- oder Glühphosphate, Grahamsches Salz, Kurrolsches und Maddrellsches Salz. Alle höheren Natrium- und Kaliumphosphate werden gemeinsam als kondensierte Phosphate bezeichnet.

Das technisch wichtige Pentanatriumtriphosphat, $Na_5P_3O_{10}$ (Natriumtripolyphosphat), ist ein wasserfrei oder mit 6 $H_2O$ kristallisierendes, nicht hygroskopisches, weißes, wasserlösliches Salz der allgemeinen Formel $NaO-[P(O)(ONa)-O]_n-Na$ mit n=3. In 100 g Wasser lösen sich bei Zimmertemperatur etwa 17 g, bei 60° ca. 20 g, bei 100° rund 32 g des kristallwasserfreien Salzes; nach zweistündigem Erhitzen der Lösung auf 100° entstehen durch Hydrolyse etwa 8% Orthophosphat und 15% Diphosphat. Bei der Herstellung von Pentanatriumtriphosphat wird Phosphorsäure mit Sodalösung oder Natronlauge im stöchiometrischen Verhältnis zur Reaktion gebracht und die Lösung durch Versprühen entwässert. Ähnlich wie Grahamsches Salz und Natriumdiphosphat löst Pentanatriumtriphosphat viele unlösliche Metall-Verbindungen (auch Kalkseifen usw.). Pentakaliumtriphosphat, $K_5P_3O_{10}$ (Kaliumtripolyphosphat),

kommt beispielsweise in Form einer 50 Gew.-%-igen Lösung (> 23% $P_2O_5$, 25% $K_2O$) in den Handel. Die Kaliumpolyphosphate finden in der Wasch- und Reinigungsmittel-Industrie breite Verwendung. Weiter existieren auch Natriumkaliumtripolyphosphate, welche ebenfalls im Rahmen der vorliegenden Erfindung einsetzbar sind. Diese entstehen beispielsweise, wenn man Natriumtrimetaphosphat mit KOH hydrolysiert:

$$(NaPO_3)_3 + 2\ KOH \rightarrow Na_3K_2P_3O_{10} + H_2O$$

Diese sind erfindungsgemäß genau wie Natriumtripolyphosphat, Kaliumtripolyphosphat oder Mischungen aus diesen beiden einsetzbar; auch Mischungen aus Natriumtripolyphosphat und Natriumkaliumtripolyphosphat oder Mischungen aus Kaliumtripolyphosphat und Natriumkaliumtripolyphosphat oder Gemische aus Natriumtripolyphosphat und Kaliumtripolyphosphat und Natriumkaliumtripolyphosphat sind erfindungsgemäß einsetzbar.

Als organische Cobuilder können in den erfindungsgemäßen Wasch- und Reinigungsmitteln insbesondere Polycarboxylate oder Polycarbonsäuren, polymere Polycarboxylate, Polyasparaginsäure, Polyacetale, gegebenenfalls oxidierte Dextrine, weitere organische Cobuilder (siehe unten) sowie Phosphonate eingesetzt werden. Diese Stoffklassen werden nachfolgend beschrieben.

Brauchbare organische Gerüstsubstanzen sind beispielsweise die in Form ihrer Natriumsalze einsetzbaren Polycarbonsäuren, wobei unter Polycarbonsäuren solche Carbonsäuren verstanden werden, die mehr als eine Säurefunktion tragen. Beispielsweise sind dies Citronensäure, Adipinsäure, Bernsteinsäure, Glutarsäure, Äpfelsäure, Weinsäure, Maleinsäure, Fumarsäure, Zuckersäuren, Aminocarbonsäuren, Nitrilotriessigsäure (NTA), sofern ein derartiger Einsatz aus ökologischen Gründen nicht zu vermeiden ist, sowie Mischungen aus diesen. Bevorzugte Salze sind die Salze der Polycarbonsäuren wie Citronensäure, Adipinsäure, Bernsteinsäure, Glutarsäure, Weinsäure, Zuckersäuren und Mischungen aus diesen.

Auch die Säuren an sich können eingesetzt werden. Sie besitzen neben ihrer Builderwirkung typischerweise auch die Eigenschaft einer Säuerungskomponente und dienen somit auch zur Einstellung eines niedrigeren und milderen pH-Wertes von Wasch- oder Reinigungsmitteln, sofern nicht der sich durch die Mischung der übrigen Komponenten ergebende pH-Wert gewünscht ist. Insbesondere sind hierbei system- unbd umweltverträgliche Säuren wie Citronensäure, Essigsäure, Weinsäure, Äpfelsäure, Milchsäure, Glykolsäure, Bernsteinsäure, Glutarsäure, Adipinsäure, Gluconsäure und beliebige Mischungen aus diesen zu nennen. Aber auch Mineralsäuren, insbesondere Schwefelsäure oder Basen, insbesondere Ammonium- oder Alkalihydroxide können als pH-Regulatoren dienen. Derartige Regulatoren sind in den erfindungemäßen Mitteln in Mengen von vorzugsweise nicht über 20 Gew.-%, insbesondere von 1,2 Gew.-% bis 17 Gew.-%, enthalten.

Als Builder sind weiter polymere Polycarboxylate geeignet, dies sind beispielsweise die Alkalimetallsalze der Polyacrylsäure oder der Polymethacrylsäure, beispielsweise solche mit einer relativen Molekülmasse von 500 bis 70 000 g/mol.

Bei den für polymere Polycarboxylate angegebenen Molmassen handelt es sich im Sinne dieser Schrift um gewichtsmittlere Molmassen $M_w$ der jeweiligen Säureform, die grundsätzlich mittels Gelpermeationschromatographie (GPC) bestimmt wurden, wobei ein UV-Detektor eingesetzt wurde. Die Messung erfolgte dabei gegen einen externen Polyacrylsäure-Standard, der aufgrund seiner strukturellen Verwandtschaft mit den untersuchten Polymeren realistische Molgewichtswerte liefert. Diese Angaben weichen deutlich von den Molgewichtsangaben ab, bei denen Polystyrolsulfonsäuren als Standard eingesetzt werden. Die gegen Polystyrolsulfonsäuren gemessenen Molmassen sind in der Regel deutlich höher als die in dieser Schrift angegebenen Molmassen.

Geeignete Polymere sind insbesondere Polyacrylate, die bevorzugt eine Molekülmasse von 2 000 bis 20 000 g/mol aufweisen. Aufgrund ihrer überlegenen Löslichkeit können aus dieser Gruppe wiederum die kurzkettigen Polyacrylate, die Molmassen von 2 000 bis 10 000 g/mol, und besonders bevorzugt von 3 000 bis 5 000 g/mol, aufweisen, bevorzugt sein.

Geeignet sind weiterhin copolymere Polycarboxylate, insbesondere solche der Acrylsäure mit Methacrylsäure und der Acrylsäure oder Methacrylsäure mit Maleinsäure. Als besonders geeignet haben sich Copolymere der Acrylsäure mit Maleinsäure erwiesen, die 50 bis 90 Gew.-% Acrylsäure und 50 bis 10 Gew.-% Maleinsäure enthalten. Ihre relative Molekülmasse, bezogen auf freie Säuren, beträgt im allgemeinen 2 000 bis 70 000 g/mol, vorzugsweise 20 000 bis 50 000 g/mol und insbesondere 30 000 bis 40 000 g/mol. Die (co-) polymeren Polycarboxylate können entweder als Pulver oder als wässerige Lösung eingesetzt werden. Der Gehalt der Mittel an (co-)polymeren Polycarboxylaten kann von 0,5 bis 20 Gew.-%, insbesondere 1 bis 10 Gew.-%, betragen.

Zur Verbesserung der Wasserlöslichkeit können die Polymere auch Allylsulfonsäuren, wie beispielsweise Allyloxybenzolsulfonsäure und Methallylsulfonsäure, als Monomer enthalten.

Insbesondere bevorzugt sind auch biologisch abbaubare Polymere aus mehr als zwei verschiedenen Monomereinheiten, beispielsweise solche, die als Monomere Salze der Acrylsäure und der Maleinsäure sowie Vinylalkohol beziehungsweise Vinylalkohol-Derivate oder die als Monomere Salze der Acrylsäure und der 2-Alkylallylsulfonsäure sowie Zucker-Derivate enthalten.

Weitere bevorzugte Copolymere sind solche, die als Monomere vorzugsweise Acrolein und Acrylsäure/Acrylsäuresalze beziehungsweise Acrolein und Vinylacetat aufweisen.

Ebenso sind als weitere bevorzugte Buildersubstanzen polymere Aminodicarbonsäuren, deren Salze oder deren Vorläufersubstanzen zu nennen. Besonders bevorzugt sind Polyasparaginsäuren beziehungsweise deren Salze und Derivate.

Weitere geeignete Buildersubstanzen sind Polyacetale, welche durch Umsetzung von Dialdehyden mit Polyolcarbonsäuren, welche 5 bis 7 C-Atome und mindestens 3 Hydroxylgruppen aufweisen, erhalten werden können. Bevorzugte Polyacetale werden aus Dialdehyden wie Glyoxal, Glutaraldehyd, Terephthalaldehyd sowie deren Gemischen und aus Polyolcarbonsäuren wie Gluconsäure und/oder Glucoheptonsäure erhalten.

Weitere geeignete organische Buildersubstanzen sind Dextrine, beispielsweise Oligomere beziehungsweise Polymere von Kohlenhydraten, die durch partielle Hydrolyse von Stärken erhalten werden können. Die Hydrolyse kann nach üblichen, beispielsweise säure- oder enzymkatalysierten Verfahren durchgeführt werden. Vorzugsweise handelt es sich um Hydrolyseprodukte mit mittleren Molmassen im Bereich von 400 bis 500 000 g/mol. Dabei ist ein Polysaccharid mit einem Dextrose-Äquivalent (DE) im Bereich von 0,5 bis 40, insbesondere von 2 bis 30 bevorzugt, wobei DE ein gebräuchliches Maß für die reduzierende Wirkung eines Polysaccharids im Vergleich zu Dextrose ist, welche ein DE von 100 besitzt. Brauchbar sind sowohl Maltodextrine mit einem DE zwischen 3 und 20 und Trockenglucosesirupe mit einem DE zwischen 20 und 37 als auch sogenannte Gelbdextrine und Weißdextrine mit höheren Molmassen im Bereich von 2 000 bis 30 000 g/mol.

Bei den oxidierten Derivaten derartiger Dextrine handelt es sich um deren Umsetzungsprodukte mit Oxidationsmitteln, welche in der Lage sind, mindestens eine Alkoholfunktion des Saccharidrings zur Carbonsäurefunktion zu oxidieren. Besonders bevorzugte organische Builder für erfindungsgemäße Mittel sind oxidierte Stärken, beziehungsweise deren bekannte Derivate.

Auch Oxydisuccinate und andere Derivate von Disuccinaten, vorzugsweise Ethylendiamindisuccinat, sind weitere geeignete Cobuilder. Dabei wird Ethylendiamin-N,N'-disuccinat (EDDS) bevorzugt in Form seiner Natrium- oder Magnesiumsalze verwendet. Weiterhin bevorzugt sind in diesem Zusammenhang auch Glycerindisuccinate und Glycerintrisuccinate. Geeignete Einsatzmengen liegen in zeolithhaltigen und/oder silicathaltigen Formulierungen zwischen 3 und 15 Gew.-%.

Weitere brauchbare organische Cobuilder sind beispielsweise acetylierte Hydroxycarbonsäuren beziehungsweise deren Salze, welche gegebenenfalls auch in Lactonform vorliegen können und welche mindestens 4 Kohlenstoffatome und mindestens eine Hydroxygruppe sowie maximal zwei Säuregruppen enthalten.

Eine weitere Substanzklasse mit Cobuildereigenschaften stellen die Phosphonate dar. Dabei handelt es sich insbesondere um Hydroxyalkan- beziehungsweise Aminoalkanphosphonate. Unter den Hydroxyalkanphosphonaten ist das 1-Hydroxyethan-1,1-diphosphonat (HEDP) von besonderer Bedeutung als Cobuilder. Es wird vorzugsweise als Natriumsalz eingesetzt, wobei das Dinatriumsalz neutral und das Tetranatriumsalz alkalisch (pH 9) reagiert. Als Aminoalkanphosphonate kommen vorzugsweise Ethylendiamintetramethylenphosphonat (EDTMP), Diethylentriaminpentamethylenphosphonat (DTPMP) sowie deren höhere Homologe in Frage. Sie werden vorzugsweise in Form der neutral reagierenden Natriumsalze, zum Beispiel als Hexanatriumsalz der EDTMP beziehungsweise als Hepta- und Octa-Natriumsalz der DTPMP, eingesetzt. Als Builder wird dabei aus der Klasse der Phosphonate bevorzugt HEDP verwendet. Die Aminoalkanphosphonate besitzen zudem ein ausgeprägtes Schwermetallbindevermögen. Dementsprechend kann es, insbesondere wenn die Mittel auch Bleiche enthalten, bevorzugt sein, Aminoalkanphosphonate, insbesondere DTPMP, einzusetzen, oder Mischungen aus den genannten Phosphonaten zu verwenden.

Darüber hinaus können alle Verbindungen, die in der Lage sind, Komplexe mit Erdalkaliionen auszubilden, als Cobuilder eingesetzt werden.

Buildersubstanzen können in den erfindungsgemäßen Mitteln gegebenenfalls in Mengen bis zu 90 Gew.-% enthalten sein. Sie sind vorzugsweise in Mengen bis zu 75 Gew.-% enthalten. Erfindungsgemäße Waschmittel weisen Buildergehalte von insbesondere 5 Gew.-% bis 50 Gew.-% auf. In erfindungsgemäßen Mitteln für die Reinigung harter Oberflächen, insbesondere zur maschinellen Reinigung von Geschirr, beträgt der Gehalt an Buildersubstanzen insbesondere 5 Gew.-% bis 88 Gew.-%, wobei in derartigen Mitteln vorzugsweise keine wasserunlöslichen Buildermaterialien eingesetzt werden. In einer bevorzugten Ausführungsform erfindungsgemäßer Mittel zur insbesondere maschinellen Reinigung von Geschirr sind 20 Gew.-% bis 40 Gew.-% wasserlöslicher organischer Builder, insbesondere Alkalicitrat, 5 Gew.-% bis 15 Gew.-% Alkalicarbonat und 20 Gew.-% bis 40 Gew.-% Alkalidisilikat enthalten.

Lösungsmittel, die in den flüssigen bis gelförmigen Zusammensetzungen von Wasch- und Reinigungsmitteln eingesetzt werden können, stammen beispielsweise aus der Gruppe ein- oder mehrwertigen Alkohole, Alkanolamine oder Glycolether, sofern sie im angegebenen Konzentrationsbereich mit Wasser mischbar sind. Vorzugsweise werden die Lösungsmittel ausgewählt aus Ethanol, n- oder i-Propanol, Butanolen, Ethylenglykolmethylether, Ethylenglykolethylether, Ethylenglykolpropylether, Ethylenglykolmono-n-butylether, Diethylenglykol-methylether, Diethylenglykolethylether, Propylenglykolmethyl-, -ethyl- oder -propyl-ether, Dipropylenglykolmonomethyl-, oder - ethylether, Di-isopropylenglykolmonomethyl-, oder -ethylether, Methoxy-, Ethoxy- oder Butoxytriglykol, 1-Butoxyethoxy-2-propanol, 3-Methyl-3-methoxybutanol, Propylen-glykolt-butylether sowie Mischungen dieser Lösungsmittel.

Lösungsmittel können in den erfindungsgemäßen flüssigen bis gelförmigen Wasch- und Reinigungsmitteln in Mengen zwischen 0,1 und 20 Gew.-%, bevorzugt aber unter 15 Gew.-% und insbesondere unterhalb von 10 Gew.-% eingesetzt werden.

Zur Einstellung der Viskosität können erfindungsgemäßen Zusammensetzungen ein oder mehrere Verdicker, beziehungsweise Verdickungssysteme zugesetzt werden. Diese hochmolekularen Stoffe, die auch Quell(ungs)mittel genannt werden, saugen meist die Flüssigkeiten auf und quellen dabei auf, um schließlich in zähflüssige echte oder kolloide Lösungen überzugehen.

Geeignete Verdicker sind anorganische oder polymere organische Verbindungen. Zu den anorganischen Verdickern zählen beispielsweise Polykieselsäuren, Tonmineralien wie Montmorillonite, Zeolithe, Kieselsäuern und Bentonite. Die organischen Verdicker stammen aus den Gruppen der natürlichen Polymere, der abgewandelten natürlichen Polymere und der vollsynthetischen Polymere. Solche aus der Natur stammenden Polymere sind beispielsweise Agar-Agar, Carrageen, Tragant, Gummi arabicum, Alginate, Pektine, Polyosen, Guar-Mehl, Johannisbrotbaumkernmehl, Stärke, Dextrine, Gelatine und Casein. Abgewandelte Naturstoffe, die als Verdicker verwendet werden, stammen vor allem aus der Gruppe der modifizierten Stärken und Cellulosen. Beispielhaft seien hier Carboxymethylcellulose und andere Celluloseether, Hydroxyethyl- und -propylcellulose sowie Kernmehlether genannt. Vollsynthetische Verdicker sind Polymere wie Polyacryl- und Polymethacryl-Verbindungen, Vinylpolymere, Polycarbonsäuren, Polyether, Polyimine, Polyamide und Polyurethane.

Die Verdicker können in einer Menge bis zu 5 Gew.-%, vorzugsweise von 0,05 bis 2 Gew.-%, und besonders bevorzugt von 0,1 bis 1,5 Gew.-%, bezogen auf die fertige Zusammensetzung, enthalten sein.

Das erfindungsgemäße Wasch- oder Reinigungsmittel kann gegebenenfalls als weitere übliche Inhaltsstoffe Sequestrierungsmittel, Elektrolyte und weitere Hilfsstoffe enthalten.

Erfindungsgemäße Textilwaschmittel können als optische Aufheller Derivate der Diaminostilbendisulfonsäure beziehungsweise deren Alkalimetallsalze enthalten. Geeignet sind zum Beispiel Salze der 4,4'-Bis(2-anilino-4-morpholino-1,3,5-triazinyl-6-amino)stilben-2,2'-disulfonsäure oder gleichartig aufgebaute Verbindungen, die anstelle der Morpholino-Gruppe eine Diethanolaminogruppe, eine Methylaminogruppe, eine Anilinogruppe oder eine 2-Methoxyethylaminogruppe tragen. Weiterhin können Aufheller vom Typ der substituierten Diphenylstyryle anwesend sein, zum Beispiel die Alkalisalze des 4,4'-Bis(2-sulfostyryl)-diphenyls, 4,4'-Bis(4-chlor-3-sulfostyryl)-diphenyls, oder 4-(4-Chlorstyryl)-4'-(2-sulfostyryl)-diphenyls. Auch Gemische der vorgenannten optischen Aufheller können verwendet werden.

Vergrauungsinhibitoren haben die Aufgabe, den von der Textilfaser abgelösten Schmutz in der Flotte suspendiert zu halten. Hierzu sind wasserlösliche Kolloide meist organischer Natur geeignet, beispielsweise Stärke, Leim, Gelatine, Salze von Ethercarbonsäuren oder Ethersulfonsäuren der Stärke oder der Cellulose oder Salze von sauren Schwefelsäureestern der Cellulose oder der Stärke. Auch wasserlösliche, saure Gruppen enthaltende Polyamide sind für diesen Zweck geeignet. Weiterhin lassen sich andere als die obengenannten Stärkederivate verwenden, zum Beispiel Aldehydstärken. Bevorzugt werden Celluloseether, wie Carboxymethylcellulose (Na-Salz), Methylcellulose, Hydroxyalkylcellulose und Mischether, wie Methylhydroxyethylcellulose, Methylhydroxypropylcellulose, Methylcarboxymethylcellulose und deren Gemische, beispielsweise in Mengen von 0,1 bis 5 Gew.-%, bezogen auf die Mittel, eingesetzt.

Um einen Silberkorrosionsschutz zu bewirken, können in erfindungsgemäßen Reinigungsmitteln für Geschirr Silberkorrosionsinhibitoren eingesetzt werden. Solche sind aus dem Stand der Technik bekannt, beispielsweise Benzotriazole, Eisen(III)-chlorid oder $CoSO_4$. Wie bekannt ist, sind für die gemeinsame Verwendung mit Enzymen besonders geeignete Silberkorrosionsinhibitoren Mangan-, Titan-, Zirkonium-, Hafnium-, Vanadium-, Cobalt- oder Cersalze und/oder -komplexe, in denen die genannten Metalle in einer der Oxidationsstufen II, III, IV, V oder VI vorliegen. Beispiele für derartige Verbindungen sind $MnSO_4$, $V_2O_5$, $V_2O_4$, $VO_2$, $TiOSO_4$, $K_2TiF_6$, $K_2ZrF_6$, $Co(NO_3)_2$, $Co(NO_3)_3$, sowie deren Gemische.

"Soil-Release"-Wirkstoffe oder "Soil-Repellents" sind zumeist Polymere, die bei der Verwendung in einem Waschmittel der Wäschefaser schmutzabstoßende Eigenschaften verleihen und/oder das Schmutzablösevermögen der übrigen Waschmittelbestandteile unterstützen. Ein vergleichbarer Effekt kann auch bei deren Einsatz in Reinigungsmitteln für harte Oberflächen beobachtet werden.

Besonders wirksame und seit langer Zeit bekannte Soil-Release-Wirkstoffe sind Copolyester mit Dicarbonsäure-, Alkylenglykol- und Polyalkylenglykoleinheiten. Beispiele dafür sind Copolymere oder Mischpolymere aus Polyethylenterephthalat und Polyoxyethylenglykol und saure Mittel, die unter anderem ein Copolymer aus einer dibasigen Carbonsäure und einem Alkylen- oder Cycloalkylenpolyglykol enthalten. Polymere aus Ethylenterephthalat und Polyethylenoxid-terephthalat sind ebenfalls vorteilhaft einsetzbar, sowie Copolyester aus Ethylenglykol, Polyethylenglykol, aromatischer Dicarbonsäure und sulfonierter aromatischer Dicarbonsäure in bestimmten Molverhältnissen. Ferner sind Methyl- oder Ethylgruppen-endverschlossene Polyester mit Ethylen- und/oder Propylen-terephthalat- und Polyethylenoxid-terephthalat-Einheiten und Waschmitel, die derartiges Soil-release-Polymer enthalten, und Polyester bekannt, die neben Oxyethylen-Gruppen und Terephthalsäureeinheiten auch substituierte Ethyleneinheiten sowie Glycerineinheiten enthalten. Ferner sind Polyester bekannt, die neben Oxyethylen-Gruppen und Terephthalsäureeinheiten 1,2-Propylen-,

1,2-Butylen- und/oder 3-Methoxy-1,2-propylengruppen sowie Glycerineinheiten enthalten und mit $C_1$-bis $C_4$-Alkylgruppen endgruppenverschlossen sind, und zumindest anteilig durch $C_{1-4}$-Alkyl- oder Acylreste endgruppenverschlossene Polyester mit Poly-propylenterephthalat- und Polyoxyethylenterephthalat-Einheiten. Man kannt auch sulfoethylendgruppenverschlossene terephthalathaltige Soil-release-Polyester und durch Sulfonierung ungesättigter Endgruppen hergestellte Soil-Release-Polyester mit Terephthalat-, Alkylenglykol- und Poly-$C_{2-4}$-Glykol-Einheiten. Des weiteren sind saure, aromatische schmutzablösevermögende Polyester und nicht polymere soil-repellent-Wirkstoffe für Materialien aus Baumwolle mit mehreren funktionellen Einheiten bekannt: Eine erste Einheit, die beispielsweise kationisch sein kann, ist zur Adsorption auf die Baumwolloberfläche durch elektrostatische Wechselwirkung befähigt, und eine zweite Einheit, die hydrophob ausgebildet ist, ist verantwortlich für das Verbleiben des Wirkstoffs an der Wasser/ Baumwolle-Grenzfläche.

Zu den für den Einsatz in erfindungsgemäßen Textilwaschmitteln in Frage kommenden Farbübertragungsinhibitoren gehören insbesondere Polyvinylpyrrolidone, Polyvinylimidazole, polymere N-Oxide wie Poly-(vinylpyridin-N-oxid) und Copolymere von Vinylpyrrolidon mit Vinylimidazol.

Beim Einsatz in maschinellen Reinigungsverfahren kann es von Vorteil sein, den Mitteln Schauminhibitoren zuzusetzen. Als Schauminhibitoren eignen sich beispielsweise Seifen natürlicher oder synthetischer Herkunft, die einen hohen Anteil an $C_{18}$-$C_{24}$-Fettsäuren aufweisen. Geeignete nichttensidartige Schauminhibitoren sind beispielsweise Organopolysiloxane und deren Gemische mit mikrofeiner, gegebenenfalls silanierter Kieselsäure sowie Paraffine, Wachse, Mikrokristallinwachse und deren Gemische mit silanierter Kieselsäure oder Bistearylethylendiamid. Mit Vorteilen werden auch Gemische aus verschiedenen Schauminhibitoren verwendet, zum Beispiel solche aus Silikonen, Paraffinen oder Wachsen. Vorzugsweise sind die Schauminhibitoren, insbesondere Silikon- und/oder Paraffin-haltige Schauminhibitoren, an eine granulare, in Wasser lösliche, beziehungsweise dispergierbare Trägersubstanz gebunden. Insbesondere sind dabei Mischungen aus Paraffinen und Bistearylethylendiamiden bevorzugt.

Aus der nicht vorveröffentlichten Anmeldung DE 102004020430.6 gehen Reinigungsmittel, insbesondere maschinelle Geschirrspülmittel hervor, die ein Copolymer aus (i) ungesättigten Carbonsäuren, (ii) Sulfonsäuregruppenhaltigen Monomeren und (iii) optional weiteren ionischen oder nichtionogenen Monomeren und eine spezielle $\alpha$-Amylase-Variante enthalten. Diese Copolymere sind auch mit $\alpha$-Amylasen kombinierbar, die der vorliegenden Erfindung entsprechen, insbesondere wenn sie neben den erfindungsgemäßen Austauschen solche aufweisen, die einer oder mehreren der Anmeldungen WO 96/23873 A1, WO 00/60060 A2 und WO 01/66712 A2 zu entnehmen sind. Dies gilt insbesondere für den Fall, daß das unter diese Anmeldungen fallende Handelsprodukt Stainzyme® der Fa. Novozymes in weiteren Positionen verbessert und zusätzlich mit mindestens einem erfindungsgemäßen Austausch versehen wird. Denn prinzipiell muß von einer additiven Wirkung der verschiedenen Modifikationen ausgegangen werden.

Ein erfindungsgemäßes Reinigungsmittel für harte Oberflächen kann darüber hinaus abrasiv wirkende Bestandteile, insbesondere aus der Gruppe umfassend Quarzmehle, Holzmehle, Kunststoffmehle, Kreiden und Mikroglaskugeln sowie deren Gemische, enthalten. Abrasivstoffe sind in den erfindungsgemäßen Reinigungsmitteln vorzugsweise nicht über 20 Gew.-%, insbesondere von 5 Gew.-% bis 15 Gew.-%, enthalten.

Farb- und Duftstoffe werden Wasch- und Reinigungsmitteln zugesetzt, um den ästhetischen Eindruck der Produkte zu verbessern und dem Verbraucher neben der Wasch- und Reinigungsleistung ein visuell und sensorisch "typisches und unverwechselbares" Produkt zur Verfügung zu stellen. Als Parfümöle beziehungsweise Duftstoffe können einzelne Riechstoffverbindungen, zum Beispiel die synthetischen Produkte vom Typ der Ester, Ether, Aldehyde, Ketone, Alkohole und Kohlenwasserstoffe verwendet werden. Riechstoffverbindungen vom Typ der Ester sind zum Beispiel Benzylacetat, Phenoxyethylisobutyrat, p-tert.-Butylcyclohexylacetat, Linalylacetat, Dimethylbenzyl-carbinylacetat, Phenylethylacetat, Linalylbenzoat, Benzylformiat, Ethylmethylphenyl-glycinat, Allylcyclohexylpropionat, Styrallylpropionat und Benzylsalicylat. Zu den Ethern zählen beispielsweise Benzylethylether, zu den Aldehyden zum Beispiel die linearen Alkanale mit 8-18 C-Atomen, Citral, Citronellal, Citronellyloxyacetaldehyd, Cyclamenaldehyd, Hydroxycitronellal, Lilial und Bourgeonal, zu den Ketonen zum Beispiel die Jonone, $\alpha$-Isomethylionon und Methyl-cedrylketon, zu den Alkoholen Anethol, Citronellol, Eugenol, Geraniol, Linalool, Phenylethylalkohol und Terpineol, zu den Kohlenwasserstoffen gehören hauptsächlich die Terpene wie Limonen und Pinen. Bevorzugt werden jedoch Mischungen verschiedener Riechstoffe verwendet, die gemeinsam eine ansprechende Duftnote erzeugen. Solche Parfümöle können auch natürliche Riechstoffgemische enthalten, wie sie aus pflanzlichen Quellen zugänglich sind, zum Beispiel Pine-, Citrus-, Jasmin-, Patchouly-, Rosen- oder Ylang-Ylang-Öl. Ebenfalls geeignet sind Muskateller, Salbeiöl, Kamillenöl, Nelkenöl, Melissenöl, Minzöl, Zimtblätteröl, Lindenblütenöl, Wacholderbeeröl, Vetiveröl, Olibanumöl, Galbanumöl und Labdanumöl sowie Orangenblütenöl, Neroliol, Orangenschalenöl und Sandelholzöl. Üblicherweise liegt der Gehalt von Wasch- und Reinigungsmitteln an Farbstoffen unter 0,01 Gew.-%, während Duftstoffe bis zu 2 Gew.-% der gesamten Formulierung ausmachen können.

Die Duftstoffe können direkt in die Wasch- und Reinigungsmittel eingearbeitet werden, es kann aber auch vorteilhaft sein, die Duftstoffe auf Träger aufzubringen, die die Haftung des Parfüms auf dem Reinigungsgut verstärken und durch eine langsamere Duftfreisetzung für langanhaltenden Duft, insbesondere von behandelten Textilien sorgen. Als solche Trägermaterialien haben sich beispielsweise Cyclodextrine bewährt, wobei die Cyclodextrin-Parfüm-Komplexe zusätzlich noch mit weiteren Hilfsstoffen beschichtet werden können. Ein weiter bevorzugter Träger für Duftstoffe

ist der beschriebene Zeolith X, der anstelle von oder in Mischung mit Tensiden auch Duftstoffe aufnehmen kann. Bevorzugt sind daher Wasch- und Reinigungsmittel, die den beschriebenen Zeolith X und Duftstoffe, die vorzugsweise zumindest teilweise an dem Zeolithen absorbiert sind, enthalten.

Bevorzugte Farbstoffe, deren Auswahl dem Fachmann keinerlei Schwierigkeit bereitet, besitzen eine hohe Lagerstabilität und Unempfindlichkeit gegenüber den übrigen Inhaltsstoffen der Mittel und gegen Licht sowie keine ausgeprägte Substantivität gegenüber Textilfasern, um diese nicht anzufärben.

Zur Bekämpfung von Mikroorganismen können Wasch- oder Reinigungsmittel antimikrobielle Wirkstoffe enthalten. Hierbei unterscheidet man je nach antimikrobiellem Spektrum und Wirkungsmechanismus zwischen Bakteriostatika und Bakteriziden, Fungistatika und Fungiziden usw. Wichtige Stoffe aus diesen Gruppen sind beispielsweise Benzalkoniumchloride, Alkylarylsulfonate, Halogenphenole und Phenolmercuriacetat. Die Begriffe antimikrobielle Wirkung und antimikrobieller Wirkstoff haben im Rahmen der erfindungsgemäßen Lehre die fachübliche Bedeutung. Geeignete antimikrobielle Wirkstoffe sind vorzugsweise ausgewählt aus den Gruppen der Alkohole, Amine, Aldehyde, antimikrobiellen Säuren beziehungsweise deren Salze, Carbonsäureester, Säureamide, Phenole, Phenolderivate, Diphenyle, Diphenylalkane, Harnstoffderivate, Sauerstoff-, Stickstoff-acetale sowie -formale, Benzamidine, Isothiazoline, Phthalimidderivate, Pyridinderivate, antimikrobiellen oberflächenaktiven Verbindungen, Guanidine, antimikrobiellen amphoteren Verbindungen, Chinoline, 1,2-Dibrom-2,4-dicyanobutan, lodo-2-propyl-butyl-carbamat, Iod, lodophore, Peroxoverbindungen, Halogenverbindungen sowie beliebigen Gemischen der voranstehenden.

Der antimikrobielle Wirkstoff kann dabei ausgewählt sein aus Ethanol, n-Propanol, i-Propanol, 1,3-Butandiol, Phenoxyethanol, 1,2-Propylenglykol, Glycerin, Undecylensäure, Benzoesäure, Salicylsäure, Dihydracetsäure, o-Phenylphenol, N-Methylmorpholinacetonitril (MMA), 2-Benzyl-4-chlorphenol, 2,2'-Methylen-bis-(6-brom-4-chlorphenol), 4,4'-Dichlor-2'-hydroxydiphenylether (Dichlosan), 2,4,4'-Trich(or-2'-hydroxydiphenylether (Trichlosan), Chlorhexidin, N-(4-Chlorphenyl)-N-(3,4-dichlorphenyl)-harnstoff, N,N'-(1,10-decan-diyldi-1-pyridinyl-4-yliden)-bis-(1-octanamin)-dihydrochlorid, N,N'-Bis-(4-chlorphenyl)-3,12-diimino-2,4,11,13-tetraaza-tetradecandiimidamid, Glucoprotaminen, antimikrobiellen oberflächenaktiven quaternären Verbindungen, Guanidinen einschl. den Bi- und Polyguanidinen, wie beispielsweise 1,6-Bis-(2-ethylhexyl-biguanido-hexan)-dihydrochlorid, 1,6-Di-($N_1$,$N_1$'-phenyldiguanido-$N_5$,$N_5$')-hexan-tetrahydochlorid, 1,6-Di-($N_1$,$N_1$'-phenyl-$N_1$,$N_1$-methyldiguanido-$N_5$,$N_5$')-hexan-dihydrochlorid, 1,6-Di-($N_1$,$N_1$'-o-chlorophenyldiguanido- $N_5$,$N_5$')-hexan-dihydrochlorid, 1,6-Di-($N_1$,$N_1$'-2,6-dichlorophenyldiguanido-$N_5$,$N_5$')hexan-dihydrochlorid, 1,6-Di-[$N_1$,$N_1$'-beta-(p-methoxyphenyl) diguanido-$N_5$,$N_5$']-hexane-dihydrochlorid, 1,6-Di-($N_1$,$N_1$'-alpha-methyl-.beta.-phenyldiguanido-$N_5$,$N_5$')-hexan-dihydrochlorid, 1,6-Di-($N_1$,$N_1$'-p-nitrophenyldiguanido-$N_5$,$N_5$')hexan-dihydrochlorid, omega:omega-Di-($N_1$,$N_1$'-phenyldiguanido-$N_5$,$N_5$')-di-n-propylether-dihydrochlorid, omega:omega'-Di-($N_1$,$N_1$'-p-chlorophenyldiguanido-$N_5$,$N_5$')-di-n-propylether-tetrahydrochlorid, 1,6-Di-($N_1$,$N_1$'-2,4-dichlorophenyldiguanido-$N_5$,$N_5$')hexan-tetrahydrochlorid, 1,6-Di-($N_1$,$N_1$'-p-methylphenyldiguanido- $N_5$,$N_5$')hexan-dihydrochlorid, 1,6-Di-($N_1$,$N_1$'-2,4,5-trichlorophenyldiguanido-$N_5$,$N_5$')hexan-tetrahydrochlorid, 1,6-Di-[$N_1$,$N_1$'-alpha-(p-chlorophenyl) ethyldiguanido-$N_5$,$N_5$'] hexan-dihydrochlorid, omega:omega-Di-($N_1$,$N_1$'-p-chlorophenyldiguanido-$N_5$,$N_5$')m-xylene-dihydrochlorid, 1,12-Di-($N_1$,$N_1$'-p-chlorophenyldiguanido-$N_5$,$N_5$') dodecan-dihydrochlorid, 1,10-Di-($N_1$,$N_1$'-phenyldiguanido-$N_5$,$N_5$')-decan-tetrahydrochlorid, 1,12-Di-($N_1$,$N_1$'-pheny)diguanido-$N_5$,$N_5$') dodecantetrahydrochlorid, 1,6-Di-($N_1$,$N_1$'-o-chlorophenyldiguanido-$N_5$,$N_5$') hexan-dihydrochlorid, 1,6-Di-($N_1$,$N_i$'-o-chlorophenyldiguanido- $N_5$,$N_5$') hexan-tetrahydrochlorid, Ethylen-bis-(1-tolyl biguanid), Ethylen-bis-(p-tolyl biguanide), Ethylen-bis-(3,5-dimethylphenylbiguanid), Ethylen-bis-(p-tert-amylphenylbiguanid), Ethylen-bis-(nonylphenylbiguanid), Ethylen-bis-(phenylbiguanid), Ethylen-bis-(N-butylphenylbiguanid), Ethylen-bis (2,5-diethoxyphenylbiguanid), Ethylen-bis (2,4-dimethylphenyl biguanid), Ethylen-bis (o-diphenylbiguanid), Ethylen-bis (mixed amyl naphthylbiguanid), N-Butyl-ethylen-bis-(phenylbiguanid), Trimethylen bis (o-tolylbiguanid), N-Butyl-trimethyle- bis-(phenyl biguanide) und die entsprechenden Salze wie Acetate, Gluconate, Hydrochloride, Hydrobromide, Citrate, Bisulfite, Fluoride, Polymaleate, N-Cocosalkylsarcosinate, Phosphite, Hypophosphite, Perfluorooctanoate, Silicate, Sorbate, Salicylate, Maleate, Tartrate, Fumarate, Ethylendiamintetraacetate, Iminodiacetate, Cinnamate, Thiocyanate, Arginate, Pyromellitate, Tetracarboxybutyrate, Benzoate, Glutarate, Monofluorphosphate, Perfluorpropionate sowie beliebige Mischungen davon. Weiterhin eignen sich halogenierte Xylol- und Kresolderivate, wie p-Chlormetakresol oder p-Chlor-meta-xylol, sowie natürliche antimikrobielle Wirkstoffe pflanzlicher Herkunft (zum Beispiel aus Gewürzen oder Kräutern), tierischer sowie mikrobieller Herkunft. Vorzugsweise können antimikrobiell wirkende oberflächenaktive quaternäre Verbindungen, ein natürlicher antimikrobieller Wirkstoff pflanzlicher Herkunft und/oder ein natürlicher antimikrobieller Wirkstoff tierischer Herkunft, äußerst bevorzugt mindestens ein natürlicher antimikrobieller Wirkstoff pflanzlicher Herkunft aus der Gruppe, umfassend Coffein, Theobromin und Theophyllin sowie ätherische Öle wie Eugenol, Thymol und Geraniol, und/oder mindestens ein natürlicher antimikrobieller Wirkstoff tierischer Herkunft aus der Gruppe, umfassend Enzyme wie Eiweiß aus Milch, Lysozym und Lactoperoxidase, und/oder mindestens eine antimikrobiell wirkende oberflächenaktive quaternäre Verbindung mit einer Ammonium-, Sulfonium-, Phosphonium-, lodonium- oder Arsoniumgruppe, Peroxoverbindungen und Chlorverbindungen eingesetzt werden. Auch Stoffe mikrobieller Herkunft, sogenannte Bakteriozine, können eingesetzt werden.

Die als antimikrobielle Wirkstoffe geeigneten quaternären Ammoniumverbindungen (QAV) weisen die allgemeine Formel $(R^1)(R^2)(R^3)(R^4)\ N^+\ X^-$ auf, in der $R^1$ bis $R^4$ gleiche oder verschiedene $C_1$-$C_{22}$-Alkylreste, $C_7$-$C_{28}$-Aral-

kylreste oder heterozyklische Reste, wobei zwei oder im Falle einer aromatischen Einbindung wie im Pyridin sogar drei Reste gemeinsam mit dem Stickstoffatom den Heterozyklus, zum Beispiel eine Pyridinium- oder Imidazoliniumverbindung, bilden, darstellen und X- Halogenidionen, Sulfationen, Hydroxidionen oder ähnliche Anionen sind. Für eine optimale antimikrobielle Wirkung weist vorzugsweise wenigstens einer der Reste eine Kettenlänge von 8 bis 18, insbesondere12 bis 16, C-Atomen auf.

QAV sind durch Umsetzung tertiärer Amine mit Alkylierungsmitteln, wie zum Beispiel Methylchlorid, Benzylchlorid, Dimethylsulfat, Dodecylbromid, aber auch Ethylenoxid herstellbar. Die Alkylierung von tertiären Aminen mit einem langen Alkyl-Rest und zwei Methyl-Gruppen gelingt besonders leicht, auch die Quaternierung von tertiären Aminen mit zwei langen Resten und einer Methyl-Gruppe kann mit Hilfe von Methylchlorid unter milden Bedingungen durchgeführt werden. Amine, die über drei lange Alkyl-Reste oder Hydroxy-substituierte Alkyl-Reste verfügen, sind wenig reaktiv und werden bevorzugt mit Dimethylsulfat quaterniert.

Geeignete QAV sind beispielweise Benzalkoniumchlorid (N-Alkyl-N,N-dimethyl-benzyl-ammoniumchlorid, CAS No. 8001-54-5), Benzalkon B (m,p-Dichlorbenzyl-dimethyl-C12-alkylammoniumchlorid, CAS No. 58390-78-6), Benzoxoniumchlorid (Benzyl-dodecyl-bis-(2-hydroxyethyl)-ammonium-chlorid), Cetrimoniumbromid (N-Hexadecyl-N,N-trimethyl-ammoniumbromid, CAS No. 57-09-0), Benzetoniumchlorid (N,N-Dimethyl-N-[2-[2-[p-(1,1,3,3-tetramethylbutyl)-pheno-xy]ethoxy]ethyl]-benzylammoniumchlorid, CAS No. 121-54-0), Dialkyldimethylammonium-chloride wie Di-n-decyldimethyl-ammoniumchlorid (CAS No. 7173-51-5-5), Didecyldi-methylammoniumbromid (CAS No. 2390-68-3), Dioctyldimethyl-ammoniumchloric, 1-Cetylpyridiniumchlorid (CAS No. 123-03-5) und Thiazoliniodid (CAS No. 15764-48-1) sowie deren Mischungen. Besonders bevorzugte QAV sind die Benzalkoniumchloride mit $C_8$-$C_{18}$-Alkylresten, insbesondere $C_{12}$-$C_{14}$-Aklyl-benzyl-dimethyl-ammoniumchlorid.

Benzalkoniumhalogenide und/oder substituierte Benzalkoniumhalogenide sind beispielsweise kommerziell erhältlich als Barquat® ex Lonza, Marquat® ex Mason, Variquat® ex Witco/ Sherex und Hyamine® ex Lonza, sowie Bardac® ex Lonza. Weitere kommerziell erhältliche antimikrobielle Wirkstoffe sind N-(3-Chlorallyl)-hexaminiumchlorid wie Dowicide® und Dowicil® ex Dow, Benzethoniumchlorid wie Hyamine® 1622 ex Rohm & Haas, Methylbenzethoniumchlorid wie Hyamine® 10X ex Rohm & Haas, Cetylpyridiniumchlorid wie Cepacolchlorid ex Merrell Labs.

Die antimikrobiellen Wirkstoffe werden in Mengen von 0,0001 Gew.-% bis 1 Gew.-%, bevorzugt von 0,001 Gew.-% bis 0,8 Gew.-%, besonders bevorzugt von 0,005 Gew.-% bis 0,3 Gew.-% und insbesondere von 0,01 bis 0,2 Gew.-% eingesetzt.

Die Mittel können UV-Absorbenzien (UV-Absorber) enthalten, die auf die behandelten Textilien aufziehen und die Lichtbeständigkeit der Fasern und/oder die Lichtbeständigkeit sonstiger Rezepturbestandteile verbessern. Unter UV-Absorber sind organische Substanzen (Lichtschutzfilter) zu verstehen, die in der Lage sind, ultraviolette Strahlen zu absorbieren und die aufgenommene Energie in Form längerwelliger Strahlung, zum Beispiel Wärme wieder abzugeben.

Verbindungen, die diese gewünschten Eigenschaften aufweisen, sind beispielsweise die durch strahlungslose Desaktivierung wirksamen Verbindungen und Derivate des Benzophenons mit Substituenten in 2- und/oder 4-Stellung. Weiterhin sind auch substituierte Benzotriazole, in 3-Stellung Phenylsubstituierte Acrylate (Zimtsäurederivate, gegebenenfalls mit Cyanogruppen in 2-Stellung), Salicylate, organische Ni-Komplexe sowie Naturstoffe wie Umbelliferon und die körpereigene Urocansäure geeignet. Besondere Bedeutung haben Biphenyl- und vor allem Stilbenderivate wie sie beispielsweise kommerziell als Tinosorb® FD oder Tinosorb® FR ex Ciba erhältlich sind. Als UV-B-Absorber sind zu nennen: 3-Benzylidencampher beziehungsweise 3-Benzylidennorcampher und dessen Derivate, zum Beispiel 3-(4-Methylbenzyliden)campher; 4-Aminobenzoesäurederivate, vorzugsweise 4-(Dimethylamino)benzoesäure-2-ethylhexylester, 4-(Dimethylamino)benzoesäure-2-octylester und 4-(Dimethylamino)benzoesäureamylester; Ester der Zimtsäure, vorzugsweise 4-Methoxyzimtsäure-2-ethylhexylester, 4-Methoxyzimtsäurepropylester, 4-Methoxyzimtsäureisoamylester, 2-Cyano-3,3-phenylzimtsäure-2-ethylhexylester (Octocrylene); Ester der Salicylsäure, vorzugsweise Salicylsäure-2-ethylhexylester, Salicylsäure-4-isopropylbenzylester, Salicylsäurehomomenthylester; Derivate des Benzophenons, vorzugsweise 2-Hydroxy-4-methoxybenzophenon, 2-Hydroxy-4-methoxy-4'-methylbenzophenon, 2,2'-Dihydroxy-4-methoxybenzophenon; Ester der Benzalmalonsäure, vorzugsweise 4-Methoxybenzmalonsäuredi-2-ethylhexylester; Triazinderivate, wie zum Beispiel 2,4,6-Trianilino-(p-carbo-2'-ethyl-1'-hexyloxy)-1,3,5-triazin und Octyl Triazon oder Dioctyl Butamido Triazone (Uvasorb® HEB); Propan-1,3-dione, wie zum Beispiel 1-(4-tert.Butylphenyl)-3-(4'methoxyphenyl)propan-1,3-dion; Ketotricyclo(5.2.1.0)decan-Derivate. Weiterhin geeignet sind 2-Phenylbenzimidazol-5-sulfonsäure und deren Alkali-, Erdalkali-, Ammonium-, Alkylammonium-, Alkanolammonium- und Glucammoniumsalze; Sulfonsäurederivate von Benzophenonen, vorzugsweise 2-Hydroxy-4-methoxybenzophenon-5-sulfonsäure und ihre Salze; Sulfonsäurederivate des 3-Benzylidencamphers, wie zum Beispiel 4-(2-Oxo-3-bornylidenmethyl)benzol-sulfonsäure und 2-Methyl-5-(2-oxo-3-bornyliden)sulfonsäure und deren Salze.

Als typische UV-A-Filter kommen insbesondere Derivate des Benzoylmethans in Frage, wie beispielsweise 1-(4'-tert.Butylphenyl)-3-(4'-methoxyphenyl)propan-1,3-dion, 4-tert.-Butyl-4'-methoxydibenzoylmethan (Parsol 1789), 1-Phenyl-3-(4'-isopropylphenyl)-propan-1,3-dion sowie Enaminverbindungen (BASF). Die UV-A und UV-B-Filter können selbstverständlich auch in Mischungen eingesetzt werden. Neben den genannten löslichen Stoffen kommen für diesen

Zweck auch unlösliche Lichtschutzpigmente, nämlich feindisperse, vorzugsweise nanoisierte Metalloxide beziehungsweise Salze in Frage. Beispiele für geeignete Metalloxide sind insbesondere Zinkoxid und Titandioxid und daneben Oxide des Eisens, Zirkoniums, Siliciums, Mangans, Aluminiums und Cers sowie deren Gemische. Als Salze können Silicate (Talk), Bariumsulfat oder Zinkstearat eingesetzt werden. Die Oxide und Salze werden in Form der Pigmente bereits für hautpflegende und hautschützende Emulsionen und dekorative Kosmetik verwendet. Die Partikel sollten dabei einen mittleren Durchmesser von weniger als 100 nm, vorzugsweise zwischen 5 und 50 nm und insbesondere zwischen 15 und 30 nm aufweisen. Sie können eine sphärische Form aufweisen, es können jedoch auch solche Partikel zum Einsatz kommen, die eine ellipsoide oder in sonstiger Weise von der sphärischen Gestalt abweichende Form besitzen. Die Pigmente können auch oberflächenbehandelt, das heißt hydrophilisiert oder hydrophobiert vorliegen. Typische Beispiele sind ummantelte Titandioxide, wie zum Beispiel Titandioxid T 805 (Degussa) oder Eusolex® T2000 (Merck; als hydrophobe Coatingmittel kommen dafür bevorzugt Silicone und besonders bevorzugt Trialkoxyoctylsilane oder Simethicone in Frage. Vorzugsweise wird mikronisiertes Zinkoxid verwendet.

Die UV-Absorbenzien werden üblicherweise in Mengen von 0,01 Gew.-% bis 5 Gew.-%, vorzugsweise von 0,03 Gew.-% bis 1 Gew.-%, eingesetzt.

Erfindungsgemäße Mittel können zur Steigerung der Wasch-, beziehungsweise Reinigungsleistung neben den erfindungsgemäßen α-Amylase-Varianten weitere Enzyme enthalten, wobei prinzipiell alle im Stand der Technik für diese Zwecke etablierten Enzyme einsetzbar sind. Hierzu gehören insbesondere Proteasen, weitere Amylasen, Lipasen, Hemicellulasen, Cellulasen oder Oxidoreduktasen, sowie vorzugsweise deren Gemische. Diese Enzyme sind im Prinzip natürlichen Ursprungs; ausgehend von den natürlichen Molekülen stehen für den Einsatz in Wasch- und Reinigungsmitteln verbesserte Varianten zur Verfügung, die entsprechend bevorzugt eingesetzt werden. Erfindungsgemäße Mittel enthalten Enzyme vorzugsweise in Gesamtmengen von $1 \times 10^{-8}$ bis 5 Gewichts-Prozent bezogen auf aktives Protein. Die Proteinkonzentration kann mit Hilfe bekannter Methoden, zum Beispiel dem BCA-Verfahren (Bicinchoninsäure; 2,2'-Bichinolyl-4,4'-dicarbonsäure) oder dem Biuret-Verfahren (A. G. Gornall, C. S. Bardawill und M.M. David, J. Biol. Chem., 177 (1948), S. 751-766) bestimmt werden.

Unter den Proteasen sind solche vom Subtilisin-Typ bevorzugt. Beispiele hierfür sind die Subtilisine BPN' und Carlsberg, die Protease PB92, die Subtilisine 147 und 309, die Alkalische Protease aus *Bacillus lentus,* Subtilisin DY und die den Subtilasen, nicht mehr jedoch den Subtilisinen im engeren Sinne zuzuordnenden Enzyme Thermitase, Proteinase K und die Proteasen TW3 und TW7. Subtilisin Carlsberg ist in weiterentwickelter Form unter dem Handelsnamen Alcalase® von der Firma Novozymes A/S, Bagsvaerd, Dänemark, erhältlich. Die Subtilisine 147 und 309 werden unter den Handelsnamen Esperase®, beziehungsweise Savinase® von der Firma Novozymes vertrieben. Von der Protease aus *Bacillus lentus* DSM 5483 (WO 91/02792 A1) leiten sich die unter der Bezeichnung BLAP® geführten Varianten ab, die insbesondere in WO 92/21760 A1, WO 95/23221 A1, WO 02/088340 A2 und WO 03/038082 A2 beschrieben werden. Weitere verwendbare Proteasen aus verschiedenen *Bacillus sp.* und *B. gibsonii* gehen aus den Patentanmeldungen WO 03/054185 A1, WO 03/056017 A2, WO 03/055974 A2 und WO 03/054184 A1 hervor.

Weitere brauchbare Proteasen sind beispielsweise die unter den Handelsnamen Durazym®, Relase®, Everlase®, Nafizym, Natalase®, Kannase® und Ovozymes® von der Firma Novozymes, die unter den Handelsnamen, Purafect®, Purafect® OxP und Properase® von der Firma Genencor, das unter dem Handelsnamen Protosol® von der Firma Advanced Biochemicals Ltd., Thane, Indien, das unter dem Handelsnamen Wuxi® von der Firma Wuxi Snyder Bioproducts Ltd., China, die unter den Handelsnamen Proleather® und Protease P® von der Firma Amano Pharmaceuticals Ltd., Nagoya, Japan, und das unter der Bezeichnung Proteinase K-16 von der Firma Kao Corp., Tokyo, Japan, erhältlichen Enzyme.

Beispiele für erfindungsgemäß zusätzlich einsetzbare Amylasen sind die α-Amylasen aus *Bacillus licheniformis,* aus *B. amyloliquefaciens* oder aus *B. stearothermophilus* sowie deren für den Einsatz in Wasch- und Reinigungsmitteln verbesserte Weiterentwicklungen. Das Enzym aus *B.licheniformis* ist von der Firma Novozymes unter dem Namen Termamyl® und von der Firma Genencor unter dem Namen Purastar®ST erhältlich. Weiterentwicklungsprodukte dieser α-Amylase sind von der Firma Novozymes unter den Handelsnamen Duramyl® und Termamyl®ultra, von der Firma Genencor unter dem Namen Purastar®OxAm und von der Firma Daiwa Seiko Inc., Tokyo, Japan, als Keistase® erhältlich. Die α-Amylase von *B. amyloliquefaciens* wird von der Firma Novozymes unter dem Namen BAN® vertrieben, und abgeleitete Varianten von der α-Amylase aus *B. stearothermophilus* unter den Namen BSG® und Novamyl®, ebenfalls von der Firma Novozymes.

Desweiteren sind für diesen Zweck die in der Anmeldung WO 02/10356 A2 offenbarte α-Amylase aus *Bacillus sp.* A 7-7 (DSM 12368) und die in der Anmeldung WO 02/44350 A2 beschriebene Cyclodextrin-Glucanotransferase (CGTase) aus *B. agaradherens* (DSM 9948) hervorzuheben. Ferner sind die amylolytischen Enzyme einsetzbar, die dem Sequenzraum von α-Amylasen angehören, der in der Anmeldung WO 03/002711 A2 definiert wird, und die, die in der Anmeldung WO 03/054177 A2 beschrieben werden. Ebenso sind Fusionsprodukte der genannten Moleküle einsetzbar, beispielsweise die aus der Anmeldung DE 10138753 A1 oder Punktmutationen davon.

Darüber hinaus sind die unter den Handelsnamen Fungamyl® von der Firma Novozymes erhältlichen Weiterentwicklungen der α-Amylase aus *Aspergillus* niger und A. oryzae geeignet. Weitere einsetzbare Handelsprodukte sind

beispielsweise die Amylase-LT® und Stainzyme®, letztere ebenfalls von der Firma Novozymes.

Erfindungsgemäße Mittel können Lipasen oder Cutinasen, insbesondere wegen ihrer Triglycerid-spaltenden Aktivitäten enthalten, aber auch, um aus geeigneten Vorstufen in situ Persäuren zu erzeugen. Hierzu gehören beispielsweise die ursprünglich aus Humicola lanuginosa (Thermomyces lanuginosus) erhältlichen, beziehungsweise weiterentwickelten Lipasen, insbesondere solche mit dem Aminosäureaustausch D96L. Sie werden beispielsweise von der Firma Novozymes unter den Handelsnamen Lipolase®, Lipolase®Ultra, LipoPrime®, Lipozyme® und Lipex® vertrieben. Desweiteren sind beispielsweise die Cutinasen einsetzbar, die ursprünglich aus Fusarium solani pisi und Humicola insolens isoliert worden sind. Ebenso brauchbare Lipasen sind von der Firma Amano unter den Bezeichnungen Lipase CE®, Lipase P®, Lipase B®, beziehungsweise Lipase CES®, Lipase AKG®, Bacillis sp. Lipase®, Lipase AP®, Lipase M-AP® und Lipase AML® erhältlich. Von der Firma Genencor sind beispielsweise die Lipasen, beziehungsweise Cutinasen einsetzbar, deren Ausgangsenzyme ursprünglich aus *Pseudomonas* mendocina und Fusarium solanii isoliert worden sind. Als weitere wichtige Handelsprodukte sind die ursprünglich von der Firma Gist-Brocades vertriebenen Präparationen M1 Lipase® und Lipomax® und die von der Firma Meito Sangyo KK, Japan, unter den Namen Lipase MY-30®, Lipase OF® und Lipase PL® vertriebenen Enzyme zu erwähnen, ferner das Produkt Lumafast® von der Firma Genencor.

Erfindungsgemäße Mittel können, insbesondere wenn sie für die Behandlung von Textilien gedacht sind, Cellulasen enthalten, je nach Zweck als reine Enzyme, als Enzympräparationen oder in Form von Mischungen, in denen sich die einzelnen Komponenten vorteilhafterweise hinsichtlich ihrer verschiedenen Leistungsaspekte ergänzen. Zu diesen Leistungsaspekten zählen insbesondere Beiträge zur Primärwaschleistung, zur Sekundärwaschleistung des Mittels (Antiredepositionswirkung oder Vergrauungsinhibition) und Avivage (Gewebewirkung), bis hin zum Ausüben eines "stone washed"-Effekts.

Eine brauchbare pilzliche, Endoglucanase(EG)-reiche Cellulase-Präparation, beziehungsweise deren Weiterentwicklungen werden von der Firma Novozymes unter dem Handelsnamen Celluzyme® angeboten. Die ebenfalls von der Firma Novozymes erhältlichen Produkte Endolase® und Carezyme® basieren auf der 50 kD-EG, beziehungsweise der 43 kD-EG aus H. insolens DSM 1800. Weitere einsetzbare Handelsprodukte dieser Firma sind Cellusoft® und Renozyme®. Letzteres basiert auf der Anmeldung WO 96/29397 A1. Leistungsverbesserte Cellulase-Varianten gehen beispielsweise aus der Anmeldung WO 98/12307 A1 hervor. Ebenso sind die in der Anmeldung WO 97/14804 A1 offenbarten Cellulasen einsetzbar; beispielsweise die darin offenbarte 20 kD-EG aus Melanocarpus, die von der Firma AB Enzymes, Finnland, unter den Handelsnamen Ecostone® und Biotouch® erhältlich ist. Weitere Handelsprodukte der Firma AB Enzymes sind Econase® und Ecopulp®. Weitere geeignete Cellulasen aus *Bacillus sp.* CBS 670.93 und CBS 669.93 werden in WO 96/34092 A2 offenbart, wobei die aus *Bacillus sp.* CBS 670.93 von der Firma Genencor unter dem Handelsnamen Puradax® erhältlich ist. Weitere Handelsprodukte der Firma Genencor sind "Genencor detergent cellulase L" und IndiAge®Neutra.

Erfindungsgemäße Mittel können insbesondere zur Entfernung bestimmter Problemanschmutzungen weitere Enzyme enthalten, die unter dem Begriff Hemicellulasen zusammengefaßt werden. Hierzu gehören beispielsweise Mannanasen, Xanthanlyasen, Pektinlyasen (=Pektinasen), Pektinesterasen, Pektatlyasen, Xyloglucanasen (=Xylanasen), Pullulanasen und β-Glucanasen. Geeignete Mannanasen sind beispielsweise unter den Namen Gamanase® und Pektinex AR® von der Firma Novozymes, unter dem Namen Rohapec® B1L von der Firma AB Enzymes, unter dem Namen Pyrolase® von der Firma Diversa Corp., San Diego, CA, USA, und unter dem Namen Purabrite® von der Firma Genencor Int., Inc., Palo Alto, CA, USA, erhältlich. Eine geeignete β-Glucanase aus einem *B. alcalophilus* geht beispielsweise aus der Anmeldung WO 99/06573 A1 hervor. Die aus *B. subtilis* gewonnene β-Glucanase ist unter dem Namen Cereflo® von der Firma Novozymes erhältlich.

Zur Erhöhung der bleichenden Wirkung können erfindungsgemäße Wasch- und Reinigungsmittel Oxidoreduktasen, beispielsweise Oxidasen, Oxygenasen, Katalasen, Peroxidasen, wie Halo-, Chloro-, Bromo-, Lignin-, Glucose- oder Mangan-peroxidasen, Dioxygenasen oder Laccasen (Phenoloxidasen, Polyphenoloxidasen) enthalten. Als geeignete Handelsprodukte sind Denilite® 1 und 2 der Firma Novozymes zu nennen. Als vorteilhaft einsetzbare Beispielsysteme für eine enzymatische Perhydrolyse sei auf die Anmeldung WO 98/45398 A1 verwiesen. Insbesondere für solch ein System brauchbare Cholinoxidasen offenbart beispielsweise WO 2004/058955 A2. Modifizierte Proteasen mit ausgeprägter, an dieser Stelle ebenfalls vorteilhaft einsetzbarer Perhydrolaseaktivität, insbesondere zum Erzielen einer milden Bleiche in Textilwaschmitteln gehen aus der Anmeldung WO 2004/058961 A1 hervor. Ein kombiniertes enzymatisches Bleichsystem, umfassend eine Oxidase und eine Perhydrolase beschreibt die Anmeldung DE 102004029475.5. Weitere erfindungsgemäß einsetzbare Perhydrolasen offenbart auch WO 2005/056782 A2. Vorteilhafterweise werden zusätzlich vorzugsweise organische, besonders bevorzugt aromatische, mit den Enzymen wechselwirkende Verbindungen zugegeben, um die Aktivität der betreffenden Oxidoreduktasen zu verstärken (Enhancer) oder um bei stark unterschiedlichen Redoxpotentialen zwischen den oxidierenden Enzymen und den Anschmutzungen den Elektronenfluß zu gewährleisten (Mediatoren).

Die in erfindungsgemäßen Mitteln eingesetzten Enzyme stammen entweder ursprünglich aus Mikroorganismen, etwa der Gattungen *Bacillus, Streptomyces, Humicola,* oder *Pseudomonas,* und/oder werden nach an sich bekannten biotechnologischen Verfahren durch geeignete Mikroorganismen produziert, etwa durch transgene Expressionswirte

der Gattungen *Bacillus* oder durch filamentöse Fungi.

Die Aufreinigung der betreffenden Enzyme erfolgt günstigerweise über an sich etablierte Verfahren, beispielsweise über Ausfällung, Sedimentation, Konzentrierung, Filtration der flüssigen Phasen, Mikrofiltration, Ultrafiltration, Einwirken von Chemikalien, Desodorierung oder geeignete Kombinationen dieser Schritte.

Erfindungsgemäßen Mitteln können die Enzyme in jeder nach dem Stand der Technik etablierten Form zugesetzt werden. Hierzu gehören beispielsweise die durch Granulation, Extrusion oder Lyophilisierung erhaltenen festen Präparationen oder, insbesondere bei flüssigen oder gelförmigen Mitteln, Lösungen der Enzyme, vorteilhafterweise möglichst konzentriert, wasserarm und/oder mit Stabilisatoren versetzt.

Alternativ können die Enzyme sowohl für die feste als auch für die flüssige Darreichungsform verkapselt werden, beispielsweise durch Sprühtrocknung oder Extrusion der Enzymlösung zusammen mit einem, vorzugsweise natürlichen Polymer oder in Form von Kapseln, beispielsweise solchen, bei denen die Enzyme wie in einem erstarrten Gel eingeschlossen sind oder in solchen vom Kern-Schale-Typ, bei dem ein enzymhaltiger Kern mit einer Wasser-, Luft- und/oder Chemikalien-undurchlässigen Schutzschicht überzogen ist. In aufgelagerten Schichten können zusätzlich weitere Wirkstoffe, beispielsweise Stabilisatoren, Emulgatoren, Pigmente, Bleich- oder Farbstoffe aufgebracht werden. Derartige Kapseln werden nach an sich bekannten Methoden, beispielsweise durch Schüttel- oder Rollgranulation oder in Fluidbed-Prozessen aufgebracht. Vorteilhafterweise sind derartige Granulate, beispielsweise durch Aufbringen polymerer Filmbildner, staubarm und aufgrund der Beschichtung lagerstabil.

Weiterhin ist es möglich, zwei oder mehrere Enzyme zusammen zu konfektionieren, so daß ein einzelnes Granulat mehrere Enzymaktivitäten aufweist.

Ein in einem erfindungsgemäßen Mittel enthaltenes Protein und/oder Enzym kann besonders während der Lagerung gegen Schädigungen wie beispielsweise Inaktivierung, Denaturierung oder Zerfall etwa durch physikalische Einflüsse, Oxidation oder proteolytische Spaltung geschützt werden. Bei mikrobieller Gewinnung der Proteine und/oder Enzyme ist eine Inhibierung der Proteolyse besonders bevorzugt, insbesondere wenn auch die Mittel Proteasen enthalten. Bevorzugte erfindungsgemäße Mittel enthalten zu diesem Zweck Stabilisatoren.

Eine Gruppe von Stabilisatoren sind reversible Proteaseinhibitoren. Häufig werden hierfür Benzamidin-Hydrochlorid, Borax, Borsäuren, Boronsäuren oder deren Salze oder Ester eingesetzt, darunter vor allem Derivate mit aromatischen Gruppen, etwa ortho-, meta-oder para-substituierte Phenylboronsäuren, insbesondere 4-Formylphenyl-Boronsäure, beziehungsweise die Salze oder Ester der genannten Verbindungen. Auch Peptidaldehyde, das heißt Oligopeptide mit reduziertem C-Terminus, insbesondere solche aus 2 bis 50 Monomeren werden zu diesem Zweck eingesetzt. Zu den peptidischen reversiblen Proteaseinhibitoren gehören unter anderem Ovomucoid und Leupeptin. Auch spezifische, reversible Peptid-Inhibitoren für die Protease Subtilisin sowie Fusionsproteine aus Proteasen und spezifischen Peptid-Inhibitoren sind hierfür geeignet.

Weitere Enzymstabilisatoren sind Aminoalkohole wie Mono-, Di-, Triethanol- und - Propanolamin und deren Mischungen, aliphatische Carbonsäuren bis zu $C_{12}$, wie beispielsweise Bernsteinsäure, andere Dicarbonsäuren oder Salze der genannten Säuren. Auch endgruppenverschlossene Fettsäureamidalkoxylate sind für diesen Zweck geeignet. Bestimmte als Builder eingesetzte organische Säuren vermögen, wie in WO 97/18287 offenbart, zusätzlich ein enthaltenes Enzym zu stabilisieren.

Niedere aliphatische Alkohole, vor allem aber Polyole, wie beispielsweise Glycerin, Ethylenglykol, Propylenglykol oder Sorbit sind weitere häufig eingesetzte Enzymstabilisatoren. Auch Di-Glycerinphosphat schützt gegen Denaturierung durch physikalische Einflüsse. Ebenso werden Calcium- und/oder Magnesiumsalze eingesetzt, wie beispielsweise Calciumacetat oder Calcium-Formiat.

Polyamid-Oligomere oder polymere Verbindungen wie Lignin, wasserlösliche Vinyl-Copolymere oder Cellulose-Ether, Acryl-Polymere und/oder Polyamide stabilisieren die Enzym-Präparation unter anderem gegenüber physikalischen Einflüssen oder pH-Wert-Schwankungen. Polyamin-N-Oxid-enthaltende Polymere wirken gleichzeitig als Enzymstabilisatoren und als Farbübertragungsinhibitoren. Andere polymere Stabilisatoren sind lineare $C_8$-$C_{18}$ Polyoxyalkylene. Auch Alkylpolyglycoside können die enzymatischen Komponenten des erfindungsgemäßen Mittels stabilisieren und vermögen vorzugsweise, diese zusätzlich in ihrer Leistung zu steigern. Vernetzte N-haltige Verbindungen erfüllen vorzugsweise eine Doppelfunktion als Soil-release-Agentien und als EnzymStabilisatoren. Hydrophobes, nichtionisches Polymer stabilisiert insbesondere eine gegebenenfalls enthaltene Cellulase.

Reduktionsmittel und Antioxidantien erhöhen die Stabilität der Enzyme gegenüber oxidativem Zerfall; hierfür sind beispielsweise schwefelhaltige Reduktionsmittel geläufig. Andere Beispiele sind Natrium-Sulfit und reduzierende Zucker.

Besonders bevorzugt werden Kombinatonen von Stabilisatoren eingesetzt, beispielsweise aus Polyolen, Borsäure und/oder Borax, die Kombination von Borsäure oder Borat, reduzierenden Salzen und Bernsteinsäure oder anderen Dicarbonsäuren oder die Kombination von Borsäure oder Borat mit Polyolen oder Polyaminoverbindungen und mit reduzierenden Salzen. Die Wirkung von Peptid-Aldehyd-Stabilisatoren wird günstigerweise durch die Kombination mit Borsäure und/oder Borsäurederivaten und Polyolen gesteigert und noch weiter durch die zusätzliche Wirkung von zweiwertigen Kationen, wie zum Beispiel Calcium-Ionen.

Erfindungsgemäße Mittel sind in einer bevorzugten Ausführungsform dadurch gekennzeichnet, daß sie, beispielsweise um die enthaltenen Wirkstoffe zeitlich oder räumlich voneinander getrennt freizusetzen, aus mehr als einer Phase bestehen. Dabei kann es sich um Phasen in verschiedenen, insbesondere aber um Phasen in denselben Aggregatzuständen handeln.

Erfindungsgemäße Mittel, die aus mehr als einer festen Komponente zusammengesetzt sind, können auf einfache Weise dadurch hergestellt werden, daß verschiedene feste Komponenten, insbesondere Pulver, Granulate oder Extrudate mit verschiedenen Inhaltsstoffen und/oder unterschiedlichem Freisetzungsverhalten in insgesamt loser Form miteinander vermischt werden. Die Herstellung erfindungsgemäßer fester Mittel aus einer oder mehreren Phasen kann auf bekannte Weise, zum Beispiel durch Sprühtrocknen oder Granulation erfolgen, wobei die Enzyme und eventuelle weitere thermisch empfindliche Inhaltsstoffe wie zum Beispiel Bleichmittel gegebenenfalls später separat zugesetzt werden. Zur Herstellung erfindungsgemäßer Mittel mit erhöhtem Schüttgewicht, insbesondere im Bereich von 650 g/l bis 950 g/l, ist ein aus der europäischen Patentschrift EP 0 486 592 bekanntes, einen Extrusionschritt aufweisendes Verfahren bevorzugt. Eine weitere bevorzugte Herstellung mit Hilfe eines Granulationsverfahrens ist in der europäischen Patentschrift EP 0 642 576 beschrieben.

Proteine können für feste Mittel beispielsweise in getrockneter, granulierter, verkapselter oder verkapselter und zusätzlich getrockneter Form eingesetzt werden. Sie können separat, das heißt als eigene Phase, oder mit anderen Bestandteilen zusammen in derselben Phase, mit oder ohne Kompaktierung zugesetzt werden. Sollen mikroverkapselte Enzyme in fester Form verarbeitet werden, so kann das Wasser mit aus dem Stand der Technik bekannten Verfahren aus den sich aus der Aufarbeitung ergebenden wäßrigen Lösungen entfernt werden, wie Sprühtrocknung, Abzentrifugieren oder durch Umsolubilisieren. Die auf diese Weise erhaltenen Teilchen haben üblicherweise eine Teilchengröße zwischen 50 und 200 $\mu$m.

Die verkapselte Form bietet sich an, um die Enzyme vor anderen Bestandteilen, wie beispielsweise Bleichmitteln, zu schützen oder um eine kontrollierte Freisetzung (controlled release) zu ermöglichen. Je nach der Größe dieser Kapseln wird nach Milli-, Mikro- und Nanokapseln unterschieden, wobei Mikrokapseln für Enzyme besonders bevorzugt sind. Eine weitere mögliche Verkapselungsmethode besteht darin, daß die für die Verwendung in Wasch- oder Reinigungsmitteln geeigneten Enzyme, ausgehend von einer Mischung der Enzymlösung mit einer Lösung oder Suspension von Stärke oder einem Stärkederivat, in Stärke, beziehungsweise dem Stärkederivat verkapselt werden.

Es können auch mindestens zwei feste Phasen miteinander verbunden vorliegen. So besteht eine Möglichkeit, ein festes erfindungsgemäßes Mittel zur Verfügung zu stellen, in dem Verpressen oder Kompaktieren zu Tabletten. Solche Tabletten können ein- oder mehrphasig sein. Damit bietet auch diese Darreichungsform die Möglichkeit, ein festes erfindungsgemäßes Mittel mit zwei festen Phasen vorzulegen. Zur Herstellung von erfindungsgemäßen Mitteln in Tablettenform, die einphasig oder mehrphasig, einfarbig oder mehrfarbig sein können und/oder aus einer mehreren Schichten bestehen können, werden vorzugsweise alle Bestandteile - gegebenenfalls je einer Schicht - in einem Mischer miteinander vermischt und das Gemisch mittels herkömmlicher Tablettenpressen, beispielsweise Exzenterpressen oder Rundläuferpressen, mit Preßkräften im Bereich von etwa 50 bis 100 $kN/cm^2$, vorzugsweise bei 60 bis 70 $kN/cm^2$ verpreßt. Insbesondere bei mehrschichtigen Tabletten kann es von Vorteil sein, wenn mindestens eine Schicht vorverpreßt wird. Dies wird vorzugsweise bei Preßkräften zwischen 5 und 20 $kN/cm^2$, insbesondere bei 10 bis 15 $kN/cm^2$ durchgeführt. Vorzugsweise weist eine derart hergestellte Tablette ein Gewicht von 10 g bis 50 g, insbesondere von 15 g bis 40 g auf. Die Raumform der Tabletten ist beliebig und kann rund, oval oder eckig sein, wobei auch Zwischenformen möglich sind.

Besonders vorteilhaft ist es, wenn in mehrphasigen Mitteln wenigstens eine der Phasen ein Amylase-sensitives Material, insbesondere Stärke enthält oder von diesem zumindest teilweise umgeben oder beschichtet ist. Auf diese Weise wird diese Phase mechanisch stabilisiert und/oder gegen Einflüsse von außen geschützt und gleichzeitig über eine in der Waschflotte wirksame Amylase angegriffen, so daß die Freisetzung der Inhaltsstoffe erleichtert wird.

Ebenfalls bevorzugte erfindungsgemäße Mittel sind dadurch gekennzeichnet, daß sie insgesamt flüssig, gelförmig oder pastös vorliegen. Die enthaltenen Proteine, vorzugsweise ein erfindungsgemäßes Protein, werden solchen Mitteln bevorzugt ausgehend von einer nach dem Stand der Technik durchgeführten Proteingewinnung und Präparation in konzentrierter wäßriger oder nichtwäßriger Lösung, beispielsweise in flüssiger Form, etwa als Lösung, Suspension oder Emulsion, aber auch in Gelform oder verkapselt oder als getrocknetes Pulver zugesetzt. Derartige erfindungsgemäße Wasch- oder Reinigungsmittel in Form von Lösungen in üblichen Lösungsmitteln werden in der Regel durch einfaches Mischen der Inhaltsstoffe hergestellt, die in Substanz oder als Lösung in einen automatischen Mischer gegeben werden können.

Eine Ausführungsform der vorliegenden Erfindung sind solche flüssigen, gelförmigen oder pastösen Mittel, denen ein erfindungswesentliches Protein und/oder eines der anderen enthaltenen Proteine und/oder einer der anderen enthaltenen Inhaltsstoffe verkapselt, vorzugsweise in Form von Mikrokapseln zugesetzt worden ist. Besonders bevorzugt sind darunter solche mit Kapseln aus amylasesensitivem Material. Solch eine gemeinsame Verwendung von Amylase-sensitiven Materialien und dem erfindungswesentlichen amylolytischen Enzym in einem Wasch- oder Reinigungsmittel kann Synergieeffekte zeigen, etwa dergestalt daß das stärkespaltende Enzym die Spaltung der Mikrokapseln unterstützt und somit den Freisetzungsprozeß der verkapselten Inhaltsstoffe steuert, so daß deren Freisetzung nicht

während der Lagerung und/oder nicht zu Beginn des Reinigungsvorgangs, sondern erst zu einem bestimmten Zeitpunkt erfolgt. Auf diesem Mechanismus können komplexe Wasch- und Reinigungsmittelsysteme mit verschiedensten Inhaltsstoffen und verschiedensten Kapseltypen beruhen, die besonders bevorzugte Ausführungsformen der vorliegenden Erfindung darstellen.

Ein vergleichbarer Effekt ist dann gegeben, wenn sich die Inhaltsstoffe des Wasch- oder Reinigungsmittels auf mindestens zwei unterschiedliche Phasen verteilen, beispielsweise zwei oder mehr feste, miteinander verbundene Phasen eines tablettenförmigen Wasch- oder Reinigungsmittels, oder verschiedene Granulate innerhalb desselben pulverförmigen Mittels. Zwei- oder Mehrphasenreiniger sind für die Anwendung sowohl in maschinellen Geschirrspülern als auch in Waschmitteln Stand der Technik. Die Aktivität eines amylolytischen Enzyms in einer früher aktivierten Phase ist Voraussetzung für die Aktivierung einer späteren Phase, wenn diese von einer Amylase-sensitiven Hülle oder Beschichtung umgeben ist oder das Amylase-sensitive Material einen integralen Bestandteil der festen Phase darstellt, bei dessen teilweiser oder vollständiger Hydrolyse die betreffende Phase desintegriert. Der Einsatz des erfindungswesentlichen Enzyms für diesen Zweck stellt somit eine bevorzugte Ausführungsform der vorliegenden Erfindung dar.

Die Inhaltsstoffe von Wasch- und Reinigungsmitteln vermögen sich geeigneterweise gegenseitig in ihrer Leistung zu unterstützen. Die synergistische Verwendung von Amylase und Farbübertragungsinhibitoren zur Steigerung der Reinigungsleistung wird beispielsweise mit der Anmeldung WO 99/63035 offenbart. Es ist auch bekannt, daß Polymere, die gleichzeitig als Cobuilder eingesetzt werden können, wie beispielsweise Alkyl-Poly-Glykoside, die Aktivität und die Stabilität von enthaltenen Enzymen stabilisieren und steigern können, so aus der Anmeldung WO 98/45396. Somit ist es bevorzugt, wenn eine erfindungsgemäße α-Amylase-Variante durch einen der übrigen, oben aufgeführten Bestandteile modifiziert, insbesondere stabilisiert und/oder in seinem Beitrag zur Wasch-, beziehungsweise Reinigungsleistung des Mittels gesteigert wird. Entsprechend abgestimmte Rezepturen für erfindungsgemäße Mittel stellen somit besonders bevorzugte Ausführungsformen der vorliegenden Erfindung dar.

Im Rahmen entsprechender Mittel können erfindungsgemäße α-Amylase-Varianten zur Aktivierung der eigenen oder anderer Phasen dienen, wenn sie allein oder zusammen mit mindestens einem anderen reinigungsaktiven oder die Reinigungswirkung unterstützenden Wirkstoff in einem aus mehr als einer Phase bestehenden Wasch- oder Reinigungsmittel bereitgestellt werden. In entsprechender Weise können sie auch dazu dienen, Inhaltsstoffe aus Kapseln freizusetzen, wenn sie oder ein anderer Wirkstoff in einem Wasch- oder Reinigungsmittel in verkapselter Form bereitgestellt wird.

Einen weiteren Erfindungsgegenstand stellen Verfahren zur Reinigung von Textilien oder von harten Oberflächen dar, die dadurch gekennzeichnet sind, daß in wenigstens einem der Verfahrensschritte eine oben beschriebene erfindungsgemäße α-Amylase-Variante aktiv wird.

Denn in dieser Ausführungsform wird die Erfindung dadurch realisiert, daß die erfindungsgemäß verbesserten enzymatischen Eigenschaften, insbesondere die erhöhte Stabilität gegen Multimerisierung prinzipiell jedem Reinigungsverfahren zugute kommen, beispielsweise dadurch, daß auch während der Anwendung weniger Enzym durch Aggregation verlorengeht. Jedes Reinigungsverfahren wird um die betreffende Aktivität bereichert, wenn sie in wenigstens einem Verfahrensschritt zugesetzt wird. Derartige Verfahren werden beispielsweise mit Maschinen wie gängigen Haushaltsgeschirrspülmaschinen oder Haushaltswaschmaschinen verwirklicht. Bevorzugte Verfahren werden entsprechend den oben gemachten Angaben entsprechend bevorzugt.

Weiter bevorzugt sind derartige Verfahren, die dadurch gekennzeichnet sind, daß die α-Amylase-Variante über ein oben beschriebenes erfindungsgemäßes Mittel eingesetzt wird.

Besonders bevorzugt ist jedes Verfahren, das dadurch gekennzeichnet ist, daß die α-Amylase in dem betreffenden Verfahrensschritt in einer Menge von 0,01 mg bis 400 mg pro entsprechendem Verfahrensschritt eingesetzt wird, vorzugsweise von 0,02 mg bis 300 mg, besonders bevorzugt von 0,03 mg bis 100 mg.

Günstigenfalls ergeben sich dabei Konzentrationswerte von 0,0005 bis 20 mg pro l, bevorzugt 0,005 bis 10 mg pro l, besonders bevorzugt 0,005 bis 8 mg des amylolytischen Proteins pro l Waschflotte. Die Proteinkonzentration kann mit Hilfe bekannter Methoden, zum Beispiel den oben genannten BCA- oder Biuret-Verfahren bestimmt werden.

Entsprechend den bisherigen Ausführungen wird die vorliegende Erfindung auch durch die Verwendung erfindungsgemäßer α-Amylase-Varianten realisiert. Denn auch hierbei kommen die günstigen Eigenschaften der betreffenden Enzyme zur Wirkung. Diese gelten insbesondere für Reinigungszwecke.

Einen eigenen Erfindungsgegenstand stellt somit die Verwendung einer der oben beschriebenen erfindungsgemäßen α-Amylase-Variante zur Reinigung von Textilien oder von harten Oberflächen dar.

Dabei kann es als einzige wirksame Komponente eingesetzt werden. Vorzugsweise geschieht dies zusammen mit mindestens einem anderen reinigungsaktiven oder die Reinigungswirkung unterstützenden Wirkstoff.

Bevorzugt ist somit eine derartige Verwendung, die dadurch gekennzeichnet ist, daß die α-Amylase-Variante über ein oben beschriebenes erfindungsgemäßes Mittel eingesetzt wird.

Günstigerweise ist eine solche Verwendung dadurch gekennzeichnet, daß pro Anwendung, vorzugsweise pro Anwendung in einer Geschirrspülmaschine oder einer Waschmaschine 0,01 mg bis 400 mg der α-Amylase-Variante eingesetzt werden, bevorzugt 0,02 mg bis 300 mg, besonders bevorzugt 0,03 mg bis 100 mg.

Denn hierdurch ergeben sich in der Waschflotte günstigerweise die oben angegebenen Konzentrationswerte. Diese Dosierung kann, je nach Reinigungsproblem vom Hersteller des Mittels oder vom Endverbraucher vorgenommen werden.

Eine weitere Ausführungsform stellt die Verwendung einer erfindungsgemäßen α-Amylase-Variante zur Behandlung von Rohmaterialien oder Zwischenprodukten in der Textilherstellung dar, insbesondere zum Entschlichten von Baumwolle.

Rohmaterialien und Zwischenprodukte der Textilherstellung, beispielsweise für solche auf Baumwollbasis, werden im Rahmen ihrer Herstellung und Weiterverarbeitung mit Stärke ausgerüstet, um eine bessere Verarbeitung zu ermöglichen. Dieses sowohl auf Garne, auf Zwischenprodukte, als auch auf Textilien angewendete Verfahren nennt man Schlichten (sizing). Zur Entfernung der Schlichte, also der stärkehaltigen Schutzschicht (Entschlichten, desizing) sind erfindungsgemäße amylolytische Proteine geeignet, und zwar insbesondere deshalb, weil sie während des Einwirkens in einem flüssigen Medium gegen Multimerisierung stabilisiert sind.

Die nachfolgenden Beispiele erläutern die vorliegende Erfindung weiter.

## Beispiele

Alle molekularbiologischen Arbeitsschritte folgen Standardmethoden, wie sie beispielsweise in dem Handbuch von Fritsch, Sambrook und Maniatis "Molecular cloning: a laboratory manual", Cold Spring Harbour Laboratory Press, New York, 1989, oder vergleichbaren einschlägigen Werken angegeben sind. Enzyme, Baukästen (Kits) und Geräte werden nach den Angaben der jeweiligen Hersteller eingesetzt.

### Beispiel 1

### Kultivierung von *Bacillus sp.* A 7-7 (DSM 12368)

Der Mikroorganismus *Bacillus sp.* A 7-7 ist unter der Hinterlegungsnummer DSM 12368 bei der Deutschen Sammlung von Mikroorganismen und Zellkulturen GmbH, Mascheroder Weg 1 b, 38124 Braunschweig (http://www.dsmz.de) hinterlegt. Er wird in der Anmeldung WO 02/10356 A2 beschrieben. Die DNA- und Aminosäuresequenzen der von dieser (hinterlegten) Spezies gebildeten α-Amylase unterscheiden in folgenden zwei Positionen von den Sequenzen, die im Sequenzprotokoll von WO 02/10356 A2 dargestellt sind: Die zugehörige DNA verfügt in den Nukleinsäure-Positionen 805-807 gemäß SEQ ID NO. 1 der vorliegenden Anmeldung über das Triplett gat, welches für die Aminosäure D (in Aminosäureposition 236) codiert, und in den Positionen 1156-1158 über das Triplett tat, welches für die Aminosäure Y (in Position 353) codiert. (In SEQ ID NO. 1 beziehungsweise 2 von WO 02/10356 A2 sind an den entsprechenden Stellen die Tripletts ggt für G beziehungsweise tgt für C angegeben.)

Über allgemein bekannte Verfahren zur Punktmutagenese, beispielsweise mit Hilfe des QuickChange®-Kit der Firma Stratagene, La Jolla, USA (siehe unten), und anhand von SEQ ID NO. 1 abgeleiteter Primer, ist es möglich, diese α-Amylase in eine andere zu überführen.

Die Kultivierung von *Bacillus sp.* A 7-7 (DSM 12368) ist in WO 02/10356 A2 beschrieben. Ein geeignetes Medium ist YPSS-Medium, enthaltend 15 g/l lösliche Stärke, 4 g/l Hefeextrakt, 1 g/l $K_2HPO_4$ und 0,5 g/l $MgSO_4$ x 7 $H_2O$, wobei der pH-Wert nach dem Autoklavieren mit 20%iger Natriumcarbonatlösung auf 10,3 eingestellt wird. Hieraus kann, wie ebenfalls in WO 02/10356 A2 dargestellt ist, die betreffende α-Amylase gewonnen werden. Somit sind diese α-Amylase und die davon abgeleiteten Varianten über allgemein bekannte Verfahren zumindest im Labormaßstab herstellbar.

### Beispiel 2

### Homologiemodelling und Auswahl der erfindungsgemäß austauschbaren Aminosäuren

### Homologiemodelling

Das Homologiemodelling wurde für die α-Amylase aus *Bacillus sp.* A 7-7 (DSM 12368) wie in der Beschreibung dargestellt über die RSCB-Protein-Databank (zugänglich über das Max-Delbrück-Zentrum in Berlin, Deutschland) durchgeführt. Dabei wurden bei der Suche mit der Proteinsequenz folgende Strukturen gefunden: die α-Amylase aus *B. licheniformis* (RCSB-PDB-Datenbankeintrag: 1 BLI), eine chimäre α-Amylase aus denen von *B. amyloliquefaciens* und *B. licheniformis* in nativer Struktur(1E3X, 1 E43), ein Acarbose-Komplex derselben chimären α-Amylase (1E3Z), ein Tris/Maltotriose-Komplex derselben chimären α-Amylase (1E40) und eine kinetisch stabilisierte Variante der α-Amylase aus *B. licheniformis* (1OB0).

Durch Übereinanderlagerung (Superimposition) dieser Strukturen im SwissPDB-Viewer wurde eine Leitstruktur

erstellt, auf die dann die Proteinsequenz der ALBA aufmodelliert wurde. Anschließend wurde die Orientierung der Seitenketten in der ALBA-Struktur korrigiert und eine Energieminimierung durchgeführt. Die einzelnen Schritte sind dem erwähnten Benutzerhandbuch zu entnehmen und erfolgten gemäß den Standardeinstellungen des Programms.

Insgesamt liegen auf der Oberfläche des 484 Aminosäuren umfassenden Moleküls folgende 407 Aminosäuren (Sie werden als solche über eine Akzessibilität von mindestens 1 definiert; zur Akzessibilität: siehe Beschreibung und nachfolgenden Abschnitt):

T5, N6, G7, T8, M9, Q11, Y12, E14, W15, Y16, L17, P18, N19, D20, G21, N22, H23, W24, N25, R26, R28, S29, D30, A31, S32, N33, K35, D36, K37, G38, I39, T40, A41, W43, P46, A47, W48, K49, G50, A51, S52, Q53, N54, D55, V56, G57, Y58, G59, A60, Y61, D62, L63, Y64, L66, G67, E68, F69, N70, Q71, K72, G73, T74, V75, R76, T77, K78, Y79, G80, T81, R82, N83, Q84, L85, Q86, A87, V89, T90, A91, K93, S94, N95, G96, Q98, V99, Y100, V103, M105, N106, H107, K108, G110, A111, D112, A113, T114, E115, W116, V117, R118, V120, E121, V122, N123, P124, S125, N126, R127, N128, Q129, E130, V131, S132, G133, D134, Y135, T136, 1137, E138, W140, K142, F143, D144, F145, P146, G147, R148, G149, N150, T151, H152, S153, N154, F155, K156, W157, R158, W159, Y160, H161, D166, W167, D168, Q169, S170, R171, Q172, L173, Q174, N175, R176, I177, Y178, K179, R181, G182, D183, G184, K185, G186, W187, W189, E190, V191, D192, T193, E194, N195, G196, N197, Y198, D199, Y200, L201, M202, Y203, I206, D207, M208, D209, H210, P211, E212, V214, N215, E216, L217, R218, N219, V222, W223, T225, N226, T227, L228, G229, L230, D231, F233, R234, I235, G236, A237, K239, H240, 1241, K242, Y243, S244, F245, T246, R247, D248, W249, L250, T251, H252, V253, R254, N255, T256, T257, G258, K259, N260, M261, F262, A263, E266, F267, W268, K269, N270, D271, I272, G273, A274, I275, E276, N277, S280, K281, N283, W284, N285, H286, S287, V288, F289, P292, L293, Y295, N296, L297, Y298, N299, S301, R302, S303, G304, G305, N306, Y307, D308, M309, R310, Q311, 1312, F313, N314, G315, V318, Q319, R320, H321, P322, T323, H324, T327, F328, V329, D330, N331, H332, D333, Q335, P336, E337, E338, A339, L340, E341, S342, F343, E345, E346, W347, F348, K349, P350, L351, C353, L355, T356, L357, R359, D360, Q361, G362, Y363, S365, V366, F367, Y368, D370, Y371, Y372, G373, I374, P375, T376, H377, G378, P380, A381, M382, K383, S384, K385, I386, D387, P388, L390, E391, R393, Q394, K395, Y396, Y398, G399, K400, Q401, N402, D403, Y404, L405, D406, H407, H408, N409, M410, R415, E416, G417, N418, T419, A420, H421, P422, N423, S424, M430, D432, G433, P434, G435, G436, N437, K438, W439, Y441, G443, R444, N445, K446, A447, G448, Q449, V450, W451, R452, D453, I454, T455, G456, N457, R458, S459, G460, T461, V462, T463, I464, N465, A466, D467, W469, N471, S473, V474, N475, G476, G477, S478, V479, V483, N484, N485.

**Berechnung der Oberflächenaminosäurereste mit einem Beitrag zum elektrostatischen Potential des Gesamtmoleküls**

Auf die Ermittlung der dreidimensionalen Struktur folgte eine Berechnung der jeweiligen Beiträge der an der Oberfläche gelegenen Aminosäuren zum elektrostatischen Potential des Gesamtmoleküls. Auch diese Berechnung erfolgte wie in der Beschreibung dargestellt mit der entsprechenden Funktion des erwähnten SwissPDB-Viewers unter Standardparametern. Derartige Beiträge werden sowohl von neutralen und negativ geladenen Aminosäureresten ausgeübt, als auch von solchen, die selbst neutral sind, aber eine weiter im Inneren des Moleküls gelegene Ladung überdecken. Es handelt sich also um eine Projektion der Ladungen auf die Moleküloberfläche.

Das Ergebnis hiervon ist in Figur 1 gezeigt: dort erkennt man die dreidimensionale Darstellung der Conolly-Oberfläche der $\alpha$-Amylase aus *Bacillus sp.* A 7-7 (DSM 12368), wobei die Ladungs- und Polaritätsverteilung farblich (weiß, grau und schwarz) hervorgehoben ist. Insgesamt liefern folgende 118 Aminosäurereste des $\alpha$-Amylase-Moleküls von *Bacillus sp.* A 7-7 (DSM 12368) einen positiven oder neutralen Beitrag zum elektrostatischen Potential der Oberfläche:

T5, N6, G7, T8, N19, G21, N22, H23, N25, R26, R28, S29, A31, S32, N33, K35, K37, G38, K49, Q53, L66, K72, T74, V75, R76, K78, T81, R82, N83, Q84, L85, Q86, A87, V89, T90, A91, K93, S94, N95, G96, Q98, K108, R118, T136, K142, G149, N150, T151, H152, N154, K156, R158, Y160, H161, R171, Q172, R176, R181, R218, T227, L228, G229, K242, R247, T251, R254, K259, N260, K281, N283, R302, R310, R320, T323, R359, Y368, Y372, T376, K383, K385, R393, Q394, K395, Y398, G399, K400, Y404, M410, R415, G417, N418, T419, A420, H421, P422, G435, G436, K438, W439, R444, N445, K446, Q449, V450, R452, R458, S459, G460, T461, V462, T463, N465, A466, N471, S473, N475, G476, N484.

**Berechnung der Solvenszugänglichkeit (Akzessibilität)**

Aufbauend auf diesen Ergebnissen wurde nun die Berechnung der Solvenszugänglichkeit (Akzessibilität) der zuvor beschriebenen auf der Oberfläche befindlichen Aminosäureresten durchgeführt, die einen positiven oder neutralen Beitrag zur Ladung beziehungsweise Polarität des Gesamtmoleküls leisten. Hierfür wurde wiederum der bereits erwähnte

SwissPdb-Viewer eingesetzt, unter Einhaltung der Standard-Paramter des Programms. Dadurch wurden folgende Aminosäurereste ermittelt, wobei die Werte für die Solvenszugänglichkeit jeweils in % in Klammern hinter den genannten Positionen angegeben sind:

T5 (39), N6 (12), G7 (13), N19 (28), N22 (28), N25 (16), R26 (27), R28 (39), S29 (38), S32 (28), N33 (28), K35 (37), K37 (18), Q53 (12), K72 (27), V75 (30), R76 (24), T81 (16), R82 (22), N83 (44), Q84 (24), Q86 (18), A87 (18), T90 (30), A91 (11), K93 (32), S94 (50), N95 (19), G96 (25), Q98 (29), R118 (41), T136 (22), K142 (30), G149 (14), N150 (39), T151 (22), H152 (27), N154 (41), K156 (30), R158 (33), Y160 (20), R171 (32), Q172 (53), R176 (41), R181 (34), R218 (18), T227 (19), G229 (14), K242 (15), R247 (20), T251 (23), R254 (15), K259 (26), N260 (49), K281 (33), N283 (40), R302 (50), R310 (31), R320 (52), T323 (49), R359 (13), Y368 (12), Y372 (37), T376 (56), K383 (19), K385 (37), Q394 (20), K395 (38), G399 (16), K400 (44), Y404 (11), G417 (11), N418 (25), T419 (59), A420 (37), H421 (16), P422 (46), G435 (25), G436 (17), W439 (47), R444 (49), N445 (41), Q449 (31), V450 (24), R452 (33), R458 (24), S459 (52), G460 (32), T461 (35), T463 (40), N465 (22), A466 (37), N471 (20), S473 (10), N475 (25), G476 (31), N484 (12).

Dies sind also die 97 Aminosäurereste des insgesamt 484 Aminosäuren umfassenden α-Amylase-Moleküls von *Bacillus sp.* A 7-7 (DSM 12368), die einen positiven oder neutralen Beitrag zum elektrostatischen Potential der Oberfläche leisten und zusätzlich eine Akzessibilität von mindestens 10% aufweisen.

Die übrigen 21 Oberflächenaminosäuren, die zuvor als solche mit einem neutralen oder positiven Beitrag ermittelt worden waren, aber eine geringere Akzessibilität als 10% besitzen, sind folgende, wobei der zugehörige Wert wiederum in Klammern angegeben ist:

T8 (2), G21 (4), H23 (2), A31 (2), G38 (6), K49 (2), L66 (2), T74 (6), K78 (6), L85 (2), V89 (1), K108 (9), H161 (1), L228 (1), R393 (5), Y398 (4), M410 (3), R415 (6), K438 (7), K446 (5), V462 (5).

**Gruppierung der neutral oder positiv geladenen oder polarisierten und dem Lösungsmittel besonders zugänglichen Aminosäurereste**

Die identifizierten 97 Reste mit einer Akzessibilität von mindestens 10% können nach ihre Lage auf der Oberfläche dieser α-Amylase verschiedenen Gruppen zugeordnet werden, wobei die der Gruppen A und B jeweils zusammenhängende Bereiche neutraler oder positiver Polarität oder Ladung darstellen. Folgende Gruppe A umfaßt die 63 Aminosäurereste unter ihnen, die eine zusammenhängende Oberfläche mit neutralem oder positiven elektrostatischen Potential bilden (in Klammern wiederum die zuvor bestimmte Akzessibilität):

(A) T5 (39), N6 (12), G7 (13), N19 (28), N22 (28), N25 (16), R26 (27), R28 (39), S29 (38), S32 (28), N33 (28), K35 (37), K37 (18), Q53 (12), K72 (27), V75 (30), R76 (24), T81 (16), R82 (22), N83 (44), Q84 (24), Q86 (18), A87 (18), T90 (30), A91 (11), K93 (32), S94 (50), N95 (19), G96 (25), Q98 (29), R118 (41), T136 (22), K142 (30), G149 (14), N150 (39), T151 (22), H152 (27), N154 (41), K156 (30), R158 (33), Y160 (20), R171 (32), Q172 (53), R181 (34), T227 (19), G229 (14), R247 (20), T251 (23), R254 (15), K259 (26), N260 (49), K281 (33), N283 (40), Q394 (20), K395 (38), G399 (16), K400 (44), G417 (11), N418 (25), T419 (59), A420 (37), H421 (16), P422 (46).

Folgende Gruppe B umfaßt die 20 Aminosäurereste, die eine zweite zusammenhängende Oberfläche mit neutralem oder positiven elektrostatischen Potential bilden:

(B) G435 (25), G436 (17), W439 (47), R444 (49), N445 (41), Q449 (31), V450 (24), R452 (33), R458 (24), S459 (52), G460 (32), T461 (35), T463 (40), N465 (22), A466 (37), N471 (20), S473 (10), N475 (25), G476 (31), N484 (12).

Folgende Gruppe C umfaßt die verbleibenden 14 Aminosäurereste, die keiner dieser beiden größeren Flächen zugeordnet werden können sondern isoliert auftreten:

(C) R176 (41), R218 (18), K242 (15), R302 (50), R310 (31), R320 (52), T323 (49), R359 (13), Y368 (12), Y372 (37), T376 (56), K383 (19), K385 (37), Y404 (11).

Insbesondere von den Aminosäuren, die den Gruppen A und B angehören, kann man annehmen, daß ihre Beiträge zu einer zusammenhängenden Oberflächenladung zu der beobachteten Tendenz zur ladungs- und/oder polaritätsvermittelten Di- und/oder Multimerisierung und damit zur Aggregation beitragen.

**Beispiel 3**

**Ortsgerichtete Mutagenese**

Die im vorangegangenen Beispiel ermittelten Positionen dienen für die α-Amylase aus *Bacillus sp.* A 7-7 (DSM 12368) als Ausgangspunkt für Punktmutationen über ortsgerichtete Mutagenese, das heißt zum Einführen einer anderen Aminosäure für die betreffende(n) Positione(n). Sie wird beispielsweise mit Hilfe des QuikChange-Kit (Fa. Stratagene, Kat.-Nr. 200518) nach dem zugehörigen Protokoll durchgeführt. Die Primer können anhand der in SEQ ID NO. 1 angegebenen DNA- und Aminosäuresequenzen entworfen werden, wobei das jeweilige Codon entsprechend der einzuführenden Aminosäure geändert wird. Hierbei sind zur Erzeugung einer weniger neutralen oder positiven Polarität oder Ladung, das heißt zum Einführen einer eher negativen Polarität oder Ladung folgende Aminosäureaustausche möglich:

| Ausgangsaminosäure | zu |
|---|---|
| Arg (R) | K, Y, C, H, G, A, V, L, I, M, F, W, P, S, T, N, Q, E oder D |
| Lys (K) | Y, C, H, G, A, V, L, I, M, F, W, P, S, T, N, Q, E oder D |
| Tyr (Y) | C, H, G, A, V, L, I, M, F, W, P, S, T, N, Q, E oder D |
| Cys (C) | H, G, A, V, L, I, M, F, W, P, S, T, N, Q, E oder D |
| His (H) | G, A, V, L, I, M, F, W, P, S, T, N, Q, E oder D |
| Gly (G) | A, V, L, I, M, F, W, P, S, T, N, Q, E oder D |
| Ala (A) | V, L, I, M, F, W, P, S, T, N, Q, E oder D |
| Val (V) | L, I, M, F, W, P, S, T, N, Q, E oder D |
| Leu (L) | I, M, F, W, P, S, T, N, Q, E oder D |
| Ile (I) | M, F, W, P, S, T, N, Q, E oder D |
| Met (M) | F, W, P, S, T, N, Q, E oder D |
| Phe (F) | W, P, S, T, N, Q, E oder D |
| Trp (W) | P, S, T, N, Q, E oder D |
| Pro (P) | S, T, N, Q, E oder D |
| Ser (S) | T, N, Q, E oder D |
| Thr (T) | N, Q, E oder D |
| Asn (N) | Q, E oder D |
| Gln (Q) | E oder D |
| Glu (E) | D |

Hierfür wird also das Codon in der Gensequenz der betreffenden α-Amylase durch ein Codon der einzuführenden Aminosäure ersetzt. Nach diesem Prinzip wird geeigneterweise ein die α-Amylase-Sequenz enthaltender Expressionsvektor entsprechend mutagenisiert und nach allgemein bekannten Verfahren in einen Expressionsstamm, im vorliegenden Beispiel in *B. subtilis* transformiert.

**Beispiel 4**

**Gewinnung und Reinigung der Amylasemutanten**

Die Anzucht Amylase-positiver *B. subtilis*-Stämme erfolgt in dem in Beispiel 1 genannten YPSS-Medium. Dieses Vorgehen sowie die Aufreinigung des von diesen Stämmen gebildeten Enzyms erfolgt gemäß der Beschreibung in WO 02/10356 A2. Daraus geht auch die Bestimmung der amylolytischen Aktivität des gereinigten Enzyms gemäß der sogenannten DNS-Methode hervor. Im folgenden dient die auf diese Weise bestimmbare Aktivität als Parameter für die Stabilität des Enzyms unter jeweils verschiedenen Bedingungen.

**Bestimmung der Aggregatbildung**

Die Bildung von Multimeren und Niederschlag wird über die Trübung einer Amylasehaltigen Lösung mit einem Amylase-Gehalt von mindestens 5 mg/ml spektrometrisch bei einer Wellenlänge von 600 nm nachgewiesen. Erfindungsgemäße Mutanten zeigen eine verringerte Neigung zur Niederschlagsbildung nach 16stündiger Inkubation in der Konzentration von mindestens 5 mg/ml Protein in Puffer oder Kultur-Medium bei 25°C, welche sich in einer verringerten Zunahme der Absorption bei 600 nm äußert.

**Beschreibung der Figuren**

Figur 1: Darstellung der Ladungs- und Polaritätsverteilung auf der Conolly-Oberfläche der α-Amylase aus *Bacillus sp.* A 7-7 (DSM 12368).

Die Darstellung wurde mithilfe des Swiss-Pdb-Viewers erstellt (Swiss Institute of Bioinformatics; http://us.expasy.org/spdbv/; Details: siehe Text).

Farbcodierung:

| | |
|---|---|
| grau: | negative Ladung oder Polarisierung |
| weiß: | neutrale Ladung oder Polarisierung |
| schwarz: | positive Ladung oder Polarisierung |

Die in dieser Darstellung nicht zu sehende rückwärtige Oberfläche des Moleküls, die auch das aktive Zentrum enthält, weist ein durchgehend negatives Ladungsmuster auf.

Figur 2: Alignment der α-Amylase aus *Bacillus sp.* A 7-7 (DSM 12368; SEQ ID NO. 2) mit den wichtigsten anderen α-Amylasen aus dem Stand der Technik, jeweils über den Bereich des maturen Proteins, das heißt den Positionen 32 bis 516 gemäß SEQ ID NO. 2.

Die individuelle Zählung kann anhand der zu Beginn jeder Zeile hinter dem jeweiligen Namen angegebenen Positionsnummer für die jeweils erste gezeigte Aminosäure nachvollzogen werden.
Darin bedeuten:

| | |
|---|---|
| A 7-7: | α-Amylase aus *Bacillus sp.* A 7-7(DSM 12368; SEQ ID NO. 2) |
| S707: | α-Amylase aus *Bacillus* sp. #707 |
| LAMY: | α-Amylase aus *Bacillus* sp. KSM-AP1378 |
| BAA: | α-Amylase aus *B. amyloliquefaciens* |
| BLA: | α-Amylase aus *B. licheniformis* |
| BStA: | α-Amylase aus *B. stearothermophilus* |
| MK716: | α-Amylase aus *Bacillus* sp. MK716 |
| TS-23: | α-Amylase aus *Bacillus* sp. TS-23 |
| K38: | α-Amylase aus *Bacillus* KSM-K38 |

## Patentansprüche

1. α-Amylase-Variante mit mindestens einem Aminosäureaustausch gegenüber dem Ausgangsmolekül, wobei bei dem Aminosäureaustausch ein auf der Oberfläche des Moleküls gelegener und einen neutralen oder positiv polaren oder geladenen Beitrag zum elektrostatischen Potential des Moleküls ausübender Aminosäurerest des Ausgangsmoleküls zu einem stärker negativ polaren oder negativ geladenen Aminosäurerest ausgetauscht worden ist, wobei folgende Aminosäureaustausche möglich sind:

| Ausgangsaminosäure | zu |
|---|---|
| Arg (R) | K, Y, C, H, G, A, V, L, I, M, F, W, P, S, T, N, Q, E oder D |
| Lys (K) | Y, C, H, G, A, V, L, I, M, F, W, P, S, T, N, Q, E oder D |
| Tyr (Y) | C, H, G, A, V, L, I, M, F, W, P, S, T, N, Q, E oder D |
| Cys (C) | H, G, A, V, L, I, M, F, W, P, S, T, N, Q, E oder D |
| His (H) | G, A, V, L, I, M, F, W, P, S, T, N, Q, E oder D |
| Gly (G) | A, V, L, I, M, F, W, P, S, T, N, Q, E oder D |
| Ala (A) | V, L, I, M, F, W, P, S, T, N, Q, E oder D |
| Val (V) | L, I, M, F, W, P, S, T, N, Q, E oder D |
| Leu (L) | I, M, F, W, P, S, T, N, Q, E oder D |
| Ile (I) | M, F, W, P, S, T, N, Q, E oder D |
| Met (M) | F, W, P, S, T, N, Q, E oder D |
| Phe (F) | W, P, S, T, N, Q, E oder D |
| Trp (W) | P, S, T, N, Q, E oder D |
| Pro (P) | S, T, N, Q, E oder D |
| Ser (S) | T, N, Q, E oder D |
| Thr (T) | N, Q, E oder D |
| Asn (N) | Q, E oder D |
| Gln (Q) | E oder D |
| Glu (E) | D |

und wobei der genannte Aminosäurerest in einer Position liegt, die einer der beiden folgenden Gruppen angehört:

(A) 5, 6, 7, 19, 22, 25, 26, 28, 29, 32, 33, 35, 37, 53, 72, 75, 76, 81, 82, 83, 84, 86, 87, 90, 91, 93, 94, 95, 96, 98, 118, 136, 142, 149, 150, 151, 152, 154, 156, 158, 160, 171, 172, 181, 227, 229, 247, 251, 254, 259, 260, 281, 283, 394, 395,399,400, 417, 418, 419, 420, 421 und 422; oder

(B) 435, 436, 439, 444, 445, 449, 450, 452, 458, 459, 460, 461, 463, 465, 466, 471, 473, 475, 476 und 484,

jeweils angegeben in der Zählung des maturen Proteins gemäß SEQ ID NO. 2, wobei es sich bei dem Ausgangsmolekül um die α-Amylase aus *Bacillus sp.* A 7-7 (DSM 12368) handelt, deren Aminosäuresequenz zu der in SEQ ID NO. 2 angegebenen Aminosäuresequenz in den Positionen +1 bis 484 100% identisch ist.

**2.** α-Amylase-Variante nach Anspruch 1, in der der genannte Aminosäurerest vor dem Aminosäureaustausch eine Akzessibilität von mindestens 10%, vorzugsweise mindestens 20%, besonders bevorzugt mindestens 30% aufweist, wobei die Akzessibilität des betreffenden Aminosäurerests über eine Skala von 0% bis 100% berechnet wird, wobei ein Wert von 0% Akzessibilität bedeutet, dass der betreffende Rest dem Solvens nicht zugänglich ist, und 100% für die in einem hypothetischen Pentapeptid GGXGG mögliche Zugänglichkeit steht.

**3.** α-Amylase-Variante nach Anspruch 1 oder 2, wobei der genannte Aminosäurerest in einem neutralen oder positiv polarisierten oder geladenen Bereich aus mindestens zwei auf der Oberfläche unmittelbar benachbarten Aminosäureresten liegt.

**4.** α-Amylase-Variante nach einem der Ansprüche 1 bis 3, wobei mehrere der genannten Aminosäureaustausche durchgeführt worden sind, vorzugsweise mindestens 2, 3, 4, 5, 6, 7, 8, 9, 10, 11, 12, 13, 14, 15, 16, 17, 18, 19, 20, 21, 22, 23, 24, 25, 26, 27, 28, 29 oder 30, besonders bevorzugt zwischen 10 und 30 und ganz besonders bevorzugt zwischen 15 und 25.

**5.** α-Amylase-Variante nach einem der Ansprüche 1 bis 4, bei der zur Verringerung der Gesamtladungsänderung an mindestens einer bis höchstens genauso vielen anderen Positionen ein oder mehrere andere auf der Oberfläche des Moleküls gelegene Aminosäurereste des Ausgangsmoleküls als die der Gruppe (A) oder (B) zu weniger negativ polaren oder negativ geladenen Aminosäureresten ausgetauscht worden ist/sind, wobei jeweils folgende Aminosäureaustausche möglich sind:

| Ausgangsaminosäure | zu |
|---|---|
| K, Y, C, H, G, A, V, L, I, M, F, W, P, S, T, N, Q, E oder D | Arg (R) |
| Y, C, H, G, A, V, L, I, M, F, W, P, S, T, N, Q, E oder D | Lys (K) |
| C, H, G, A, V, L, I, M, F, W, P, S, T, N, Q, E oder D | Tyr (Y) |
| H, G, A, V, L, I, M, F, W, P, S, T, N, Q, E oder D | Cys (C) |
| G, A, V, L, I, M, F, W, P, S, T, N, Q, E oder D | His (H) |
| A, V, L, I, M, F, W, P, S, T, N, Q, E oder D | Gly (G) |
| V, L, I, M, F, W, P, S, T, N, Q, E oder D | Ala (A) |
| L, I, M, F, W, P, S, T, N, Q, E oder D | Val (V) |
| I, M, F, W, P, S, T, N, Q, E oder D | Leu (L) |
| M, F, W, P, S, T, N, Q, E oder D | Ile (I) |
| F, W, P, S, T, N, Q, E oder D | Met (M) |
| W, P, S, T, N, Q, E oder D | Phe (F) |
| P, S, T, N, Q, E oder D | Trp (W) |
| S, T, N, Q, E oder D | Pro (P) |
| T, N, Q, E oder D | Ser (S) |
| N, Q, E oder D | Thr (T) |
| Q, E oder D | Asn (N) |

(fortgesetzt)

| Ausgangsaminosäure | zu |
|---|---|
| E oder D | Gln (Q) |
| D | Glu (E) |

**6.** α-Amylase-Variante nach Anspruch 5, wobei der beziehungsweise die zur Verringerung der Gesamtladungsänderung durchgeführte(n) Austausch(e) an Aminosäureposition(en) durchgeführt worden ist/sind, der/die nicht zu den folgenden gehört/en:

(A)5, 6, 7, 19, 22, 25, 26, 28, 29, 32, 33, 35, 37, 53, 72, 75, 76, 81, 82, 83, 84, 86, 87, 90, 91, 93, 94, 95, 96, 98, 118, 136, 142, 149, 150, 151, 152, 154, 156, 158, 160, 171, 172, 181, 227, 229, 247, 251, 254, 259, 260, 281, 283, 394, 395, 399, 400, 417, 418, 419, 420, 421 und 422 oder
(B)435, 436, 439, 444, 445, 449, 450, 452, 458, 459, 460, 461, 463, 465, 466, 471, 473, 475, 476 und 484 oder
(C) 176, 218, 242, 302, 310, 320, 323, 359, 368, 372, 376, 383, 385 und 404,

jeweils angegeben in der Zählung des maturen Proteins gemäß SEQ ID NO. 2.

**7.** α-Amylase-Variante nach einem der Ansprüche 1 bis 6, wobei es sich bei dem Ausgangsmolekül um eine α-Amylase-Variante mit zunehmend bevorzugt 1, 2, 3, 4, 5, 6, 7, 8, 9, 10 oder 11 zusätzlichen Punktmutationen handelt.

**8.** α-Amylase-Variante nach Anspruch 7 mit den zusätzlichen Punktmutationen in einer oder mehreren der folgenden Positionen: 5, 167, 170, 177, 202, 204, 271, 330, 377, 385 und 445, in der Zählung des maturen Proteins gemäß SEQ ID NO. 2.

**9.** α-Amylase-Variante nach Anspruch 8, wobei es sich um folgende Punktmutationen handelt: 5A, 167R, 170P, 177L, 202L, 204V, 271D, 330D, 377R, 385S und/oder 445Q.

**10.** Verfahren zur Erhöhung der Stabilität einer α-Amylase aus *Bacillus sp.* A 7-7 (DSM 12368) gegenüber einer über elektrostatische Wechselwirkungen vermittelten Dimerisierung und/oder Multimerisierung, wodurch mindestens ein auf der Oberfläche des Moleküls gelegener und einen neutralen oder positiv polaren oder geladenen Beitrag zum elektrostatischen Potential des Moleküls ausübender Aminosäurerest des Ausgangsmoleküls zu einem stärker negativ polaren oder negativ geladenen Aminosäurerest ausgetauscht wird ist, wobei folgende Aminosäureaustausche möglich sind:

| Ausgangsaminosäure | zu |
|---|---|
| Arg (R) | K, Y, C, H, G, A, V, L, I, M, F, W, P, S, T, N, Q, E oder D |
| Lys (K) | Y, C, H, G, A, V, L, I, M, F, W, P, S, T, N, Q, E oder D |
| Tyr (Y) | C, H, G, A, V, L, I, M, F, W, P, S, T, N, Q, E oder D |
| Cys (C) | H, G, A, V, L, I, M, F, W, P, S, T, N, Q, E oder D |
| His (H) | G, A, V, L, I, M, F, W, P, S, T, N, Q, E oder D |
| Gly (G) | A, V, L, I, M, F, W, P, S, T, N, Q, E oder D |
| Ala (A) | V, L, I, M, F, W, P, S, T, N, Q, E oder D |
| Val (V) | L, I, M, F, W, P, S, T, N, Q, E oder D |
| Leu (L) | I, M, F, W, P, S, T, N, Q, E oder D |
| Ile (I) | M, F, W, P, S, T, N, Q, E oder D |
| Met (M) | F, W, P, S, T, N, Q, E oder D |
| Phe (F) | W, P, S, T, N, Q, E oder D |
| Trp (W) | P, S, T, N, Q, E oder D |
| Pro (P) | S, T, N, Q, E oder D |

(fortgesetzt)

| Ausgangsaminosäure | zu |
|---|---|
| Ser (S) | T, N, Q, E oder D |
| Thr (T) | N, Q, E oder D |
| Asn (N) | Q, E oder D |
| Gln (Q) | E oder D |
| Glu (E) | D |

und wobei der genannte Aminosäurerest in einer Position liegt, die einer der beiden folgenden Gruppen angehört:

(A) 5, 6, 7, 19, 22, 25, 26, 28, 29, 32, 33, 35, 37, 53, 72, 75, 76, 81, 82, 83, 84, 86, 87, 90, 91, 93, 94, 95, 96, 98, 118, 136, 142, 149, 150, 151, 152, 154, 156, 158, 160, 171, 172, 181, 227, 229, 247, 251, 254, 259, 260, 281, 283, 394, 395, 399, 400, 417, 418, 419, 420, 421 und 422; oder
(B) 435, 436, 439, 444, 445, 449, 450, 452, 458, 459, 460, 461, 463, 465, 466, 471, 473, 475, 476 und 484,

jeweils angegeben in der Zählung des maturen Proteins gemäß SEQ ID NO. 2, wobei es sich bei dem Ausgangsmolekül um die $\alpha$-Amylase aus *Bacillus sp.* A 7-7 (DSM 12368) handelt, deren Aminosäuresequenz zu der in SEQ ID NO. 2 angegebenen Aminosäuresequenz in den Positionen +1 bis 484 100% identisch ist.

**11.** Verfahren nach Anspruch 10, wobei der genannte Aminosäurerest vor dem Aminosäureaustausch eine eine Akzessibilität von mindestens 10%, vorzugsweise mindestens 20%, besonders bevorzugt mindestens 30% aufweist, wobei die Akzessibilität des betreffenden Aminosäurerests über eine Skala von 0% bis 100% berechnet wird, wobei ein Wert von 0% Akzessibilität bedeutet, dass der betreffefnde Rest dem Solvens nicht zugänglich ist, und 100% für die in einem hypothetischen Pentapeptid GGXGG mögliche Zugänglichkeit steht.

**12.** Verfahren nach Anspruch 10 oder 11, wobei der genannte Aminosäurerest in einem neutralen oder positiv polarisierten oder geladenen Bereich aus mindestens zwei auf der Oberfläche unmittelbar benachbarten Aminosäureresten liegt.

**13.** Verfahren nach einem der Ansprüche 10 bis 12, wobei mehrere der genannten Aminosäureaustausche durchgeführt werden, vorzugsweise mindestens 2, 3, 4, 5, 6, 7, 8, 9, 10, 11, 12, 13, 14, 15, 16, 17, 18, 19, 20, 21, 22, 23, 24, 25, 26, 27, 28, 29 oder 30, besonders bevorzugt zwischen 10 und 30 und ganz besonders bevorzugt zwischen 15 und 25.

**14.** Verfahren nach einem der Ansprüche 10 bis 13, wonach zur Verringerung der Gesamtladungsänderung an mindestens einer bis höchstens genauso vielen anderen Positionen ein oder mehrere andere auf der Oberfläche des Moleküls gelegene Aminosäurereste des Ausgangsmoleküls als die der Gruppe (A) oder (B) zu weniger negativ polaren oder negativ geladenen Aminosäureresten ausgetauscht wird ist/sind, wobei jeweils folgende Aminosäureaustausche möglich sind:

| Ausgangsaminosäure | zu |
|---|---|
| K, Y, C, H, G, A, V, L, I, M, F, W, P, S, T, N, Q, E oder D | Arg (R) |
| Y, C, H, G, A, V, L, I, M, F, W, P, S, T, N, Q, E oder D | Lys (K) |
| C, H, G, A, V, L, I, M, F, W, P, S, T, N, Q, E oder D | Tyr (Y) |
| H, G, A, V, L, I, M, F, W, P, S, T, N, Q, E oder D | Cys (C) |
| G, A, V, L, I, M, F, W, P, S, T, N, Q, E oder D | His (H) |
| A, V, L, I, M, F, W, P, S, T, N, Q, E oder D | Gly (G) |
| V, L, I, M, F, W, P, S, T, N, Q, E oder D | Ala (A) |
| L, I, M, F, W, P, S, T, N, Q, E oder D | Val (V) |
| I, M, F, W, P, S, T, N, Q, E oder D | Leu (L) |

(fortgesetzt)

| Ausgangsaminosäure | zu |
|---|---|
| M, F, W, P, S, T, N, Q, E oder D | Ile (I) |
| F, W, P, S, T, N, Q, E oder D | Met (M) |
| W, P, S, T, N, Q, E oder D | Phe (F) |
| P, S, T, N, Q, E oder D | Trp (W) |
| S, T, N, Q, E oder D | Pro (P) |
| T, N, Q, E oder D | Ser (S) |
| N, Q, E oder D | Thr (T) |
| Q, E oder D | Asn (N) |
| E oder D | Gln (Q) |
| D | Glu (E) |

**15.** Verfahren nach Anspruch 14, wobei der beziehungsweise die zur Verringerung der Gesamtladungsänderung durchgeführte(n) Austausch(e) an Aminosäureposition(en) durchgeführt worden ist/sind, der/die nicht zu den folgenden gehört/en:

(A)5, 6, 7, 19, 22, 25, 26, 28, 29, 32, 33, 35, 37, 53, 72, 75, 76, 81, 82, 83, 84, 86, 87, 90, 91, 93, 94, 95, 96, 98, 118, 136, 142, 149, 150, 151, 152, 154, 156, 158, 160, 171, 172, 181, 227, 229, 247, 251, 254, 259, 260, 281, 283, 394, 395, 399, 400, 417, 418, 419, 420, 421 und 422 oder
(B)435, 436, 439, 444, 445, 449, 450, 452, 458, 459, 460, 461, 463, 465, 466, 471, 473, 475, 476 und 484 oder
(C) 176, 218, 242, 302, 310, 320, 323, 359, 368, 372, 376, 383, 385 und 404,

jeweils angegeben in der Zählung des maturen Proteins gemäß SEQ ID NO. 2.

**16.** Verfahren nach einem der Ansprüche 10 bis 15, wobei es sich bei dem Ausgangsmolekül um eine α-Amylase-Variante mit zunehmend bevorzugt 1, 2, 3, 4, 5, 6, 7, 8, 9, 10 oder 11 zusätzlichen Punktmutationen handelt.

**17.** Verfahren nach Anspruch 16, wobei die eingebrachte α-Amylase eine α-Amylase-Variante mit den zusätzlichen Punktmutationen in einer oder mehreren der folgenden Positionen ist: 5, 167, 170, 177, 202, 204, 271, 330, 377, 385 und 445, in der Zählung des maturen Proteins gemäß SEQ ID NO. 2.

**18.** Verfahren nach Anspruch 17, wobei es sich um folgende Punktmutationen handelt: 5A, 167R, 170P, 177L, 202L, 204V, 271D, 330D, 377R, 385S und/oder 445Q.

**19.** Nukleinsäure, codierend für eine α-Amylase-Variante nach einem der Ansprüche 1 bis 9.

**20.** Nukleinsäure nach Anspruch 19, welche sich von einer Nukleinsäure gemäß SEQ ID NO. 1 ableitet, oder von einer Variante hiervon mit zunehmend bevorzugt 1, 2, 3, 4, 5, 6, 7, 8, 9, 10 oder 11 zusätzlichen Punktmutationen zu solchen Mutationen, die zu einem der mit den Ansprüchen 1 oder 6 bezeichneten Aminosäureaustausche führen.

**21.** Vektor, enthaltend eine Nukleinsäure nach Anspruch 19 oder 20, bevorzugt ein Klonierungsvektor oder ein Expressionsvektor.

**22.** Zelle, die nach gentechnischer Modifizierung eine der in den Ansprüchen 19 oder 20 bezeichneten Nukleinsäuren enthält.

**23.** Zelle nach Anspruch 22, wobei die genannte Nukleinsäure Teil eines Vektors ist, insbesondere eines Vektors nach Anspruch 21.

**24.** Zelle nach Anspruch 22 oder 23, bei der es sich um ein Bakterium handelt, insbesondere eines, das die gebildete α-Amylase-Variante ins umgebende Medium sekretiert.

**25.** Zelle nach Anspruch 24, wobei es sich um ein gramnegatives Bakterium handelt, insbesondere eines der Gattungen *Escherichia coli, Klebsiella, Pseudomonas* oder *Xanthomonas,* insbesondere um Stämme von *E. coli* K12, *E. coli* B oder *Klebsiella planticola,* und ganz besonders um Derivate der Stämme *Escherichia coli* BL21 (DE3), *E. coli* RV308, *E. coli* DH5$\alpha$, *E.coli* JM109, *E. coli* XL-1 oder *Klebsiella planticola* (Rf).

**26.** Zelle nach Anspruch 24, wobei es sich um ein grampositives Bakterium handelt, insbesondere eines der Gattungen *Bacillus, Staphylococcus* oder *Corynebacterium,* ganz besonders der Species *Bacillus lentus, B. licheniformis*, *B. amyloliquefaciens, B. subtilis, B. globigii* oder *B. alcalophilus, Staphylococcus carnosus* oder *Corynebacterium glutamicum,* und hierunter wiederum ganz besonders bevorzugt um ein Derivat von *B. licheniformis* DSM 13.

**27.** Biotechnologisches Produktionsverfahren zur Herstellung einer $\alpha$-Amylase-Variante nach einem der Ansprüche 1 bis 9.

**28.** Verfahren nach Anspruch 27, unter Verwendung einer Nukleinsäure nach einem der Ansprüche 19 oder 20, besonders bevorzugt unter Verwendung eines Vektors nach Anspruch 21, ganz besonders bevorzugt unter Verwendung einer Zelle nach einem der Ansprüche 22 bis 26.

**29.** Mittel, enthaltend eine $\alpha$-Amylase-Variante nach einem der Ansprüche 1 bis 9.

**30.** Mittel nach Anspruch 29, wobei es sich um ein Wasch- oder Reinigungsmittel handelt.

**31.** Wasch- oder Reinigungsmittel nach Anspruch 30, enthaltend 0,000001 Gewichts-Prozent bis 5 Gew.-%, insbesondere 0,00001 bis 3 Gew.-% der $\alpha$-Amylase-Variante.

**32.** Wasch- oder Reinigungsmittel nach Anspruch 30 oder 31, welches zusätzlich andere Enzyme enthält, insbesondere hydrolytische Enzyme oder Oxidoreduktasen, besonders bevorzugt weitere Amylasen, Proteasen, Lipasen, Cutinasen, Hemicellulasen, Cellulasen, $\beta$-Glucanasen, Oxidasen, Peroxidasen, Perhydrolasen und/oder Laccasen.

**33.** Wasch- oder Reinigungsmittel nach einem der Ansprüche 30 bis 32, wobei die $\alpha$-Amylase-Variante durch einen der sonstigen Bestandteile des Mittels stabilisiert und/oder in ihrem Beitrag zur Wasch-, beziehungsweise Reinigungsleistung des Mittels gesteigert wird.

**34.** Wasch- oder Reinigungsmittel nach einem der Ansprüche 30 bis 33, welches insgesamt fest ist, vorzugsweise nach einem Kompaktierungsschritt für mindestens eine der enthaltenen Komponenten, besonders bevorzugt, daß es insgesamt kompaktiert ist.

**35.** Wasch- oder Reinigungsmittel nach einem der Ansprüche 30 bis 34, welches insgesamt flüssig, gelförmig oder pastös ist, vorzugsweise unter Verkapselung für mindestens eine der enthaltenen Komponenten, besonders bevorzugt unter Verkapselung mindestens eines der enthaltenen Enzyme, ganz besonders bevorzugt unter Verkapselung der $\alpha$-Amylase-Variante.

**36.** Verfahren zur Reinigung von Textilien oder von harten Oberflächen, in welchem wenigstens einem der Verfahrensschritte eine $\alpha$-Amylase-Variante nach einem der Ansprüche 1 bis 9 zugesetzt und aktiv wird.

**37.** Verfahren nach Anspruch 36, wobei die $\alpha$-Amylase-Variante über ein Mittel nach einem der Ansprüche 30 bis 35 eingesetzt wird.

**38.** Verfahren nach Anspruch 36 oder 37, wobei die $\alpha$-Amylase in dem betreffenden Verfahrensschritt in einer Menge von 0,01 mg bis 400 mg pro entsprechendem Verfahrensschritt eingesetzt wird, vorzugsweise von 0,02 mg bis 300 mg, besonders bevorzugt von 0,03 mg bis 100 mg.

**39.** Verwendung einer $\alpha$-Amylase-Variante nach einem der Ansprüche 1 bis 9 zur Reinigung von Textilien oder von harten Oberflächen.

**40.** Verwendung nach Anspruch 39, wobei die $\alpha$-Amylase-Variante über ein Mittel nach einem der Ansprüche 30 bis 35 eingesetzt wird.

**41.** Verwendung nach Anspruch 39 oder 40, wobei pro Anwendung, vorzugsweise pro Anwendung in einer Geschirrspülmaschine oder einer Waschmaschine 0,01 mg bis 400 mg der α-Amylase-Variante eingesetzt werden, bevorzugt 0,02 mg bis 300 mg, besonders bevorzugt 0,03 mg bis 100 mg.

**42.** Verwendung einer α-Amylase-Variante nach einem der Ansprüche 1 bis 9 zur Behandlung von Rohmaterialien oder Zwischenprodukten in der Textilherstellung, insbesondere zum Entschlichten von Baumwolle.

## Claims

**1.** An α-amylase variant having at least one amino acid substitution over the starting molecule, wherein, in the case of the amino acid substitution, an amino acid residue on the surface of said starting molecule, which makes a neutral or positively polar or charged contribution to the electrostatic potential of said molecule, has been replaced with a more negatively polar or negatively charged amino acid residue, with the following amino acid substitutions being possible:

| Starting amino acid | to give |
|---|---|
| Arg (R) | K, Y, C, H, G, A, V, L, I, M, F, W, P, S, T, N, Q, E or D |
| Lys (K) | Y, C, H, G, A, V, L, I, M, F, W, P, S, T, N, Q, E or D |
| Tyr (Y) | C, H, G, A, V, L, I, M, F, W, P, S, T, N, Q, E or D |
| Cys (C) | H, G, A, V, L, I, M, F, W, P, S, T, N, Q, E or D |
| His (H) | G, A, V, L, I, M, F, W, P, S, T, N, Q, E or D |
| Gly (G) | A, V, L, I, M, F, W, P, S, T, N, Q, E or D |
| Ala (A) | V, L, I, M, F, W, P, S, T, N, Q, E or D |
| Val (V) | L, I, M, F, W, P, S, T, N, Q, E or D |
| Leu (L) | I, M, F, W, P, S, T, N, Q, E or D |
| Ile (I) | M, F, W, P, S, T, N, Q, E or D |
| Met (M) | F, W, P, S, T, N, Q, E or D |
| Phe (F) | W, P, S, T, N, Q, E or D |
| Trp (W) | P, S, T, N, Q, E or D |
| Pro (P) | S, T, N, Q, E or D |
| Ser (S) | T, N, Q, E or D |
| Thr (T) | N, Q, E or D |
| Asn (N) | Q, E or D |
| Gln (Q) | E or D |
| Glu (E) | D |

and wherein said amino acid residue is in a position belonging to either of the following two groups:

(A) 5, 6, 7, 19, 22, 25, 26, 28, 29, 32, 33, 35, 37, 53, 72, 75, 76, 81, 82, 83, 84, 86, 87, 90, 91, 93, 94, 95, 96, 98, 118, 136, 142, 149, 150, 151, 152, 154, 156, 158, 160, 171, 172, 181, 227, 229, 247, 251, 254, 259, 260, 281, 283, 394, 395, 399, 400, 417, 418, 419, 420, 421 and 422; or
(B) 435, 436, 439, 444, 445, 449, 450, 452, 458, 459, 460, 461, 463, 465, 466, 471, 473, 475, 476 and 484,

in each case indicated in the numbering of the mature protein according to SEQ ID NO. 2,
wherein the starting molecule is the α-amylase from *Bacillus sp.* A 7-7 (DSM 12368), whose amino acid sequence is 100% identical to the amino acid sequence indicated in SEQ ID NO. 2 in positions +1 to 484.

**2.** The α-amylase variant according to claim 1, in which said amino acid residue has an accessibility of at least 10%,

preferably at least 20%, particularly preferably at least 30%, prior to amino acid substitution, wherein said accessibility of the amino acid residue in question is calculated on a scale from 0% to 100%, wherein a value of 0% accessibility means that the relevant residue is not accessible to the solvent, and 100% represents the accessibility possible in a hypothetical pentapeptide, GGXGG.

**3.** The α-amylase variant according to claim 1 or 2, in which said amino acid residue is located in a neutral or positively polarized or charged region of at least two directly adjacent amino acid residues on the surface.

**4.** The α-amylase variant according to any of claims 1 to 3, wherein a plurality of said amino acid substitutions have been carried out, preferably at least 2, 3, 4, 5, 6, 7, 8, 9, 10, 11, 12, 13, 14, 15, 16, 17, 18, 19, 20, 21, 22, 23, 24, 25, 26, 27, 28, 29 or 30, particularly preferably between 10 and 30 and very particularly preferably between 15 and 25.

**5.** The α-amylase variant according to any of claims 1 to 4, in which, in order to reduce the change in the overall charge, in from at least one to no more than exactly as many other positions one or more other amino acid residues on the surface of the starting molecule than those of group (A) or (B) have been replaced with less negatively polar or negatively charged amino acid residues, with in each case the following amino acid substitutions being possible:

| Starting amino acid | to give |
|---|---|
| K, Y, C, H, G, A, V, L, I, M, F, W, P, S, T, N, Q, E or D | Arg (R) |
| Y, C, H, G, A, V, L, I, M, F, W, P, S, T, N, Q, E or D | Lys (K) |
| C, H, G, A, V, L, I, M, F, W, P, S, T, N, Q, E or D | Tyr (Y) |
| H, G, A, V, L, I, M, F, W, P, S, T, N, Q, E or D | Cys (C) |
| G, A, V, L, I, M, F, W, P, S, T, N, Q, E | His |
| or D | (H) |
| A, V, L, I, M, F, W, P, S, T, N, Q, E or D | Gly (G) |
| V, L, I, M, F, W, P, S, T, N, Q, E or D | Ala (A) |
| L, I, M, F, W, P, S, T, N, Q, E or D | Val (V) |
| I, M, F, W, P, S, T, N, Q, E or D | Leu (L) |
| M, F, W, P, S, T, N, Q, E or D | Ile (I) |
| F, W, P, S, T, N, Q, E or D | Met (M) |
| W, P, S, T, N, Q, E or D | Phe (F) |
| P, S, T, N, Q, E or D | Trp (W) |
| S, T, N, Q, E or D | Pro (P) |
| T, N, Q, E or D | Ser (S) |
| N, Q, E or D | Thr (T) |
| Q, E or D | Asn (N) |
| E or D | Gln (Q) |
| D | Glu (E) |

**6.** The α-amylase variant according to claim 5, wherein the substitution(s) carried out in order to reduce the change in the overall charge have been carried out at amino acid position(s) which does/do not belong to the following:

(A) 5, 6, 7, 19, 22, 25, 26, 28, 29, 32, 33, 35, 37, 53, 72, 75, 76, 81, 82, 83, 84, 86, 87, 90, 91, 93, 94, 95, 96, 98, 118, 136, 142, 149, 150, 151, 152, 154, 156, 158, 160, 171, 172, 181, 227, 229, 247, 251, 254, 259, 260, 281, 283, 394, 395, 399, 400, 417, 418, 419, 420, 421 and 422; or
(B) 435, 436, 439, 444, 445, 449, 450, 452, 458, 459, 460, 461, 463, 465, 466, 471, 473, 475, 476 and 484 or
(C) 176, 218, 242, 302, 310, 320, 323, 359, 368, 372, 376, 383, 385 and 404,

in each case indicated in the numbering of the mature protein according to SEQ ID NO. 2.

7. The α-amylase variant according to any of claims 1 to 6, wherein the starting molecule is an α-amylase variant containing, with increasing preference, 1, 2, 3, 4, 5, 6, 7, 8, 9, 10 or 11 additional point mutations.

8. The α-amylase variant according to claim 7, having the additional point mutations in one or more of the following positions: 5, 167, 170, 177, 202, 204, 271, 330, 377, 385 and 445, according to the numbering of the mature protein according to SEQ ID NO. 2.

9. The α-amylase variant according to claim 8, wherein the point mutations are as follows: 5A, 167R, 170P, 177L, 202L, 204V, 271D, 330D, 377R, 385S and/or 445Q.

10. A method of increasing the stability of an α-amylase from Bacillus sp. A7-7 (DSM 12368) to a dimerization and/or multimerization brought about by electrostatic interactions, whereby at least one amino acid residue on the surface of the starting molecule, which makes a neutral or positively polar or charged contribution to the electrostatic potential of said molecule, is replaced with a more negatively polar or negatively charged amino acid residue, with the following amino acid substitutions being possible:

| Starting amino acid | to give |
|---|---|
| Arg (R) | K, Y, C, H, G, A, V, L, I, M, F, W, P, S, T, N, Q, E or D |
| Lys (K) | Y, C, H, G, A, V, L, I, M, F, W, P, S, T, N, Q, E or D |
| Tyr (Y) | C, H, G, A, V, L, I, M, F, W, P, S, T, N, Q, E or D |
| Cys (C) | H, G, A, V, L, I, M, F, W, P, S, T, N, Q, E or D |
| His (H) | G, A, V, L, I, M, F, W, P, S, T, N, Q, E or D |
| Gly (G) | A, V, L, I, M, F, W, P, S, T, N, Q, E or D |
| Ala (A) | V, L, I, M, F, W, P, S, T, N, Q, E or D |
| Val (V) | L, I, M, F, W, P, S, T, N, Q, E or D |
| Leu (L) | I, M, F, W, P, S, T, N, Q, E or D |
| Ile (I) | M, F, W, P, S, T, N, Q, E or D |
| Met (M) | F, W, P, S, T, N, Q, E or D |
| Phe (F) | W, P, S, T, N, Q, E or D |
| Trp (W) | P, S, T, N, Q, E or D |
| Pro (P) | S, T, N, Q, E or D |
| Ser (S) | T, N, Q, E or D |
| Thr (T) | N, Q, E or D |
| Asn (N) | Q, E or D |
| Gln (Q) | E or D |
| Glu (E) | D |

and wherein said amino acid residue is in a position belonging to either of the following two groups:

(A) 5, 6, 7, 19, 22, 25, 26, 28, 29, 32, 33, 35, 37, 53, 72, 75, 76, 81, 82, 83, 84, 86, 87, 90, 91, 93, 94, 95, 96, 98, 118, 136, 142, 149, 150, 151, 152, 154, 156, 158, 160, 171, 172, 181, 227, 229, 247, 251, 254, 259, 260, 281, 283, 394, 395, 399, 400, 417, 418, 419, 420, 421 and 422; or
(B) 435, 436, 439, 444, 445, 449, 450, 452, 458, 459, 460, 461, 463, 465, 466, 471, 473, 475, 476 and 484,

in each case indicated in the numbering of the mature protein according to SEQ ID NO. 2,
wherein the starting molecule is the α-amylase from *Bacillus sp.* A 7-7 (DSM 12368), whose amino acid sequence

is 100% identical to the amino acid sequence indicated in SEQ ID NO. 2 in positions +1 to 484.

**11.** The method according to claim 10, wherein said amino acid residue has an accessibility of at least 10%, preferably at least 20%, particularly preferably at least 30%, prior to amino acid substitution, wherein said accessibility of the amino acid residue in question is calculated on a scale from 0% to 100%, wherein a value of 0% accessibility means that the relevant residue is not accessible to the solvent, and 100% represents the accessibility possible in a hypothetical pentapeptide, GGXGG.

**12.** The method according to claim 10 or 11, in which said amino acid residue is located in a neutral or positively polarized or charged region of at least two directly adjacent amino acid residues on the surface.

**13.** The method according to any of claims 10 to 12, wherein a plurality of said amino acid substitutions have been carried out, preferably at least 2, 3, 4, 5, 6, 7, 8, 9, 10, 11, 12, 13, 14, 15, 16, 17, 18, 19, 20, 21, 22, 23, 24, 25, 26, 27, 28, 29 or 30, particularly preferably between 10 and 30 and very particularly preferably between 15 and 25.

**14.** The method according to any of claims 10 to 13, whereby, in order to reduce the change in the overall charge, in from at least one to no more than exactly as many other positions one or more other amino acid residues on the surface of the starting molecule than those of groupb (A) or (B) have been replaced with less negatively polar or negatively charged amino acid residues, with in each case the following amino acid substitutions being possible:

| Starting amino acid | to give |
|---|---|
| K, Y, C, H, G, A, V, L, I, M, F, W, P, S, T, N, Q, E or D | Arg (R) |
| Y, C, H, G, A, V, L, I, M, F, W, P, S, T, N, Q, E or D | Lys (K) |
| C, H, G, A, V, L, I, M, F, W, P, S, T, N, Q, E or D | Tyr (Y) |
| H, G, A, V, L, I, M, F, W, P, S, T, N, Q, E or D | Cys (C) |
| G, A, V, L, I, M, F, W, P, S, T, N, Q, E or D | His (H) |
| A, V, L, I, M, F, W, P, SF, T, N, Q, E or D | Gly (G) |
| V, L, I, M, F, W, P, S, T, N, Q, E or D | Ala (A) |
| L, I, M, F, W, P, S, T, N, Q, E or D | Val (V) |
| I, M, F, W, P, S, T, N, Q, E or D | Leu (L) |
| M, F, W, P, S, T, N, Q, E or D | Ile (I) |
| F, W, P, S, T, N, Q, E or D | Met (M) |
| W, P, S, T, N, Q, E or D | Phe (F) |
| P, S, T, N, Q, E or D | Trp (W) |
| S, T, N, Q, E or D | Pro |
| | (P) |
| T, N, Q, E or D | Ser (S) |
| N, Q, E or D | Thr (T) |
| Q, E or D | Asn (N) |
| E or D | Gln (Q) |
| D | Glu (E) |

**15.** The method according to claim 14, wherein the substitution(s) carried out in order to reduce the change in the overall charge have been carried out at amino acid position(s) which does/do not belong to the following:

(A) 5, 6, 7, 19, 22, 25, 26, 28, 29, 32, 33, 35, 37, 53, 72, 75, 76, 81, 82, 83, 84, 86, 87, 90, 91, 93, 94, 95, 96, 98, 118, 136, 142, 149, 150, 151, 152, 154, 156, 158, 160, 171, 172, 181, 227, 229, 247, 251, 254, 259, 260,

281, 283, 394, 395, 399, 400, 417, 418, 419, 420, 421 and 422; or
(B) 435, 436, 439, 444, 445, 449, 450, 452, 458, 459, 460, 461, 463, 465, 466, 471, 473, 475, 476 and 484,
(C) 176, 218, 242, 302, 310, 320, 323, 359, 368, 372, 376, 383, 385 and 404,

in each case indicated in the numbering of the mature protein according to SEQ ID NO. 2.

**16.** The method according to any of claims 10 to 15, wherein the starting molecule is an α-amylase variant containing, with increasing preference, 1, 2, 3, 4, 5, 6, 7, 8, 9, 10 or 11 additional point mutations.

**17.** The method according to claim 16, wherein the introduced α-amylase is an α-amylase variant having the additional point mutations in one or more of the following positions: 5, 167, 170, 177, 202, 204, 271, 330, 377, 385 and 445, according to the numbering of the mature protein according to SEQ ID NO. 2.

**18.** The method according to claim 17, wherein the point mutations are as follows: 5A, 167R, 170P, 177L, 202L, 204V, 271D, 330D, 377R, 385S and/or 445Q.

**19.** A nucleic acid coding for an α-amylase variant according to any of claims 1 to 9.

**20.** The nucleic acid according to claim 19, which is derived from a nucleic acid according to SEQ ID NO. 1 or from a variant thereof having, with increasing preference, 1, 2, 3, 4, 5, 6, 7, 8, 9, 10 or 11 additional point mutations to give those mutations resulting in any of the amino acid substitutions as defined in claim 1 or 6.

**21.** A vector comprising a nucleic acid according to claim 19 or 20, preferably a cloning vector or an expression vector.

**22.** A cell comprising, after genetic modification, any of the nucleic acids as defined in claim 19 or 20.

**23.** The cell according to claim 22, wherein said nucleic acid is part of a vector, in particular part of a vector according to claim 21.

**24.** The cell according to claim 22 or 23, which is a bacterium, in particular one that secretes the α-amylase variant formed into the surrounding medium.

**25.** The cell according to claim 24, which is a Gram-negative bacterium, in particular one of the genera *Escherichia coli, Klebsiella, Pseudomonas* or *Xanthomonas,* in particular *E. coli* K12, *E. coli* B or *Klebsiella planticola* strains, and very particular derivatives of the strains *Escherichia coli* BL21 (DE3), *E. coli* RV308, *E. coli* DH5α, *E. coli* JM109, *E. coli* XL-1 or *Klebsiella planticola* (Rf).

**26.** The cell according to claim 24, which is a Gram-positive bacterium, in particular one of the genera *Bacillus, Staphylococcus* or *Corynebacterium,* very particularly of the species *Bacillus lentus, B. licheniformis, B. amyloliquefaciens, B. subtilis, B. globigii* or *B. alcalophilus, Staphylococcus carnosus* or *Corynebacterium glutamicum,* and among these in turn, very particularly preferably a *B. licheniformis* DSM 13 derivative.

**27.** A bio-technological production method for producing an α-amylase variant according to any of claims 1 to 9.

**28.** The method according to claim 27, using a nucleic acid according to either of claims 19 and 20, particularly preferably using a vector according to claim 21, very particularly preferably using a cell according to any of claims 22 to 26.

**29.** An agent comprising an α-amylase variant according to any of claims 1 to 9.

**30.** The agent according to claim 29, which is a detergent or cleanser.

**31.** A detergent or cleanser according to claim 30, comprising from 0.000001 percent by weight to 5% by weight, in particular from 0.00001 to 3% by weight, of the α-amylase variant.

**32.** The detergent or cleanser according to claim 30 or 31, which additionally includes other enzymes, in particular hydrolytic enzymes or oxidoreductases, particularly preferably further amylases, proteases, lipases, cutinases, hemicellulases, cellulases, β-glucanases, oxidases, peroxidases, perhydrolases and/or laccases.

**33.** The detergent or cleanser according to any of claims 30 to 32, wherein the α-amylase variant is stabilized and/or its contribution to the washing or cleaning performance of the agent is increased by any of the other components of said agent.

**34.** The detergent or cleanser according to any of claims 30 to 33, which is overall solid, preferably after a compacting step for at least one of the included components, particularly preferably that it is overall compacted.

**35.** The detergent or cleanser according to any of claims 30 to 34, which is overall liquid, gel-like or paste-like, preferably with encapsulation of at least one of the included components, particularly preferably with encapsulation of at least one of the included enzymes, very particularly preferably with encapsulation of the α-amylase variant.

**36.** A method of cleaning textiles or hard surfaces, which comprises at least one step in which an α-amylase variant according to any of claims 1 to 9 is added and becomes active.

**37.** The method according to claim 36, wherein the α-amylase variant is employed by way of an agent according to any of claims 30 to 35.

**38.** The method according to claim 36 or 37, wherein the α-amylase is employed in the step in question in an amount of from 0.01 mg to 400 mg per corresponding step, preferably from 0.02 mg to 300 mg, particularly preferably from 0.03 mg to 100 mg.

**39.** The use of an α-amylase variant according to any of claims 1 to 9 for cleaning textiles or hard surfaces.

**40.** The use according to claim 39, wherein the α-amylase variant is employed by way of an agent according to any of claims 30 to 35.

**41.** The use according to claim 39 or 40, wherein from 0.01 mg to 400 mg of the α-amylase variant, preferably from 0.02 mg to 300 mg, particularly preferably from 0.03 mg to 100 mg, are employed per application, preferably per application in a dishwasher or a washing machine.

**42.** The use of an α-amylase variant according to any of claims 1 to 9 for the treatment of raw materials or intermediate products in textile manufacture, in particular for desizing cotton.

## Revendications

**1.** Variant d'α-amylase comportant au moins un échange d'acides aminés par rapport à la molécule de départ, un résidu d'acide aminé de la molécule de départ, se trouvant sur la surface de la molécule et exerçant une contribution neutre ou positivement polaire ou chargée au potentiel électrostatique de la molécule, ayant été, lors de l'échange d'acides aminés, remplacé par un résidu d'acide aminé plus fortement négativement polaire ou négativement chargé, les échanges d'acides aminés suivants étant possibles :

| Acide aminé de départ | en |
|---|---|
| Arg (R) | K, Y, C, H, G, A, V, L, I, M, F, W, P, S, T, N, Q, E ou D |
| Lys (K) | Y, C, H, G, A, V, L, I, L, F, W, P, S, T, N, Q, E ou D |
| Tyr (Y) | C, H, G, A, V, L, I, M, F, W, P, S, T, N, Q, E ou D |
| Cys (C) | H, G, A, V, L, I, M, F, W, P, S, T, N, Q, E ou D |
| His (H) | G, A, V, L, I, M, F, W, P, S, T, N, Q, E ou D |
| Gly (G) | A, V, L, I, M, F, W, P, S, T, N, Q, E ou D |
| Ala (A) | V, L, I, M, F, W, P, S, T, N, Q, E ou D |
| Val (V) | L, I, M, F, W, P, S, T, N, Q, E ou D |
| Leu (L) | I, M, F, W, P, S, T, N, Q, E ou D |
| Ile (I) | M, F, W, P, S, T, N, Q, E ou D |

(suite)

| Acide aminé de départ | en |
|---|---|
| Met (M) | F, W, P, S, T, N, Q, E ou D |
| Phe (F) | W, P, S, T, N, Q, E ou D |
| Trp (W) | P, S, T, N, Q, E ou D |
| Pro (P) | S, T, N, Q, E ou D |
| Ser (S) | T, N, Q, E ou D |
| Thr (T) | N, Q, E ou D |
| Asn (N) | Q, E ou D |
| Gln (Q) | E ou D |
| Glu (E) | D |

et le résidu d'acide aminé mentionné se trouvant sur une position qui appartient à l'un des deux groupes suivants :

(A) 5, 6, 7, 19, 22, 25, 26, 28, 29, 32, 33, 35, 37, 53, 72, 75, 76, 81, 82, 83, 84, 86, 87, 90, 91, 93, 94, 95, 96, 98, 118, 136, 142, 149, 150, 151, 152, 154, 156, 158, 160, 171, 172, 181, 227, 229, 247, 251, 254, 259, 260, 281, 283, 394, 395, 399, 400, 417, 418, 419, 420, 421, et 422, ou
(B) 435, 436, 439, 444, 445, 449, 450, 452, 458, 459, 460, 461, 463, 465, 466, 471, 473, 475, 476 et 484,

chacune de ces indications correspondant à la numérotation de la protéine mature selon SEQ ID NO:2, où, pour ce qui concerne la molécule de départ, il s'agit de l'$\alpha$-amylase de *Bacillus sp.* A 7-7 (DSM 12368), dont la séquence d'acides aminés a une identité de 100 % avec la séquence d'acides aminés présentée dans SEQ ID NO:2 sur les positions +1 à 484.

**2.** Variant d'$\alpha$-amylase selon la revendication 1, dans lequel le résidu d'acide aminé mentionné présente, avant le remplacement de l'acide aminé, une accessibilité d'au moins 10 %, de préférence d'au moins 20 %, d'une manière particulièrement préférée d'au moins 30 %, l'accessibilité du résidu d'acide aminé considéré étant calculée sur une échelle de 0 % à 100 %, une valeur de 0 % désignant une accessibilité telle que le résidu considéré ne soit pas accessible au solvant, et 100 % désignant l'accessibilité possible dans un pentapeptide hypothétique GGXGG.

**3.** Variant d'$\alpha$-amylase selon la revendication 1 ou 2, dans lequel le résidu d'acide aminé mentionné se trouve dans un domaine neutre ou positivement polarisé ou chargé, constitué d'au moins deux résidus d'acides aminés immédiatement voisins sur la surface.

**4.** Variant d'$\alpha$-amylase selon l'une des revendications 1 à 3, dans lequel plusieurs des échanges d'acides aminés mentionnés ont été réalisés, de préférence au moins 2, 3, 4, 5, 6, 7, 8, 9, 10, 11, 12, 13, 14, 15, 16, 17, 18, 19, 20, 21, 22, 23, 24, 25, 26, 27, 28, 29 ou 30, d'une manière particulièrement préférée de 10 à 30 et d'une manière tout particulièrement préférée de 15 à 25.

**5.** Variant d'$\alpha$-amylase selon l'une des revendications 1 à 4, dans lequel, pour diminuer la modification de la charge totale, un ou plusieurs résidus d'acides aminés de la molécule de départ, situés sur la surface de la molécule, autres que ceux du groupe (A) et du groupe (B), ont été, sur au moins une à au plus exactement le même nombre d'autres positions, remplacés par des résidus d'acides aminés moins négativement polaires ou négativement chargés, les échanges d'acides aminés suivants étant possibles dans chaque cas :

| Acide aminé de départ | en |
|---|---|
| K, Y, C, H, G, A, V, L, I, M, F, W, P, S, T, N, Q, E ou D | Arg (R) |
| Y, C, H, G, A, V, L, I, L, F, W, P, S, T, N, Q, E ou D | Lys (K) |
| C, H, G, A, V, L, I, M, F, W, P, S, T, N, Q, E ou D | Tyr (Y) |

(suite)

| Acide aminé de départ | en |
|---|---|
| H, G, A, V, L, I, M, F, W, P, S, T, N, Q, E ou D | Cys (C) |
| G, A, V, L, I, M, F, W, P, S, T, N, Q, E ou D | His (H) |
| A, V, L, I, M, F, W, P, S, T, N, Q, E ou D | Gly (G) |
| V, L, I, M, F, W, P, S, T, N, Q, E ou D | Ala (A) |
| L, I, M, F, W, P, S, T, N, Q, E ou D | Val (V) |
| I, M, F, W, P, S, T, N, Q, E ou D | Leu (L) |
| M, F, W, P, S, T, N, Q, E ou D | Ile (I) |
| F, W, P, S, T, N, Q, E ou D | Met (M) |
| W, P, S, T, N, Q, E ou D | Phe (F) |
| P, S, T, N, Q, E ou D | Trp (W) |
| S, T, N, Q, E ou D | Pro (P) |
| T, N, Q, E ou D | Ser (S) |
| N, Q, E ou D | Thr (T) |
| Q, E ou D | Asn (N) |
| E ou D | Gln (Q) |
| D | Glu (E) |

**6.** Variant d'α-amylase selon la revendication 5, dans lequel le ou les échange(s) réalisé(s) pour diminuer la modification de la charge totale a été/ont été réalisé (s) sur la ou les position (s) d'acides aminés qui n'appartient pas/n'appartiennent pas aux suivantes :

(A) 5, 6, 7, 19, 22, 25, 26, 28, 29, 32, 33, 35, 37, 53, 72, 75, 76, 81, 82, 83, 84, 86, 87, 90, 91, 93, 94, 95, 96, 98, 118, 136, 142, 149, 150, 151, 152, 154, 156, 158, 160, 171, 172, 181, 227, 229, 247, 251, 254, 259, 260, 281, 283, 394, 395, 399, 400, 417, 418, 419, 420, 421, et 422, ou
(B) 435, 436, 439, 444, 445, 449, 450, 452, 458, 459, 460, 461, 463, 465, 466, 471, 473, 475, 476 et 484,
(C) 176, 218, 242, 302, 310, 320, 323, 359, 368, 372, 376, 383, 385 et 404,

chacune correspondant à la numérotation de la protéine mature selon SEQ ID NO:2.

**7.** Variant d'α-amylase selon l'une des revendications 1 à 6, dans lequel, pour ce qui concerne la molécule de départ, il s'agit d'un variant d'α-amylase ayant, dans l'ordre croissant de préférence, 1, 2, 3, 4, 5, 6, 7, 8, 9, 10 ou 11 mutations ponctuelles additionnelles.

**8.** Variant d'α-amylase selon la revendication 7, ayant les mutations ponctuelles additionnelles sur une ou plusieurs des positions suivantes : 5, 167, 170, 177, 202, 204, 271, 330, 377, 385 et 445, selon la numérotation de la protéine mature selon SEQ ID NO:2.

**9.** Variant d'α-amylase selon la revendication 8, dans lequel il s'agit des mutations ponctuelles suivantes : 5A, 167R, 170P, 177L, 202L, 204V, 271D, 330D, 377R, 385S et/ou 445Q.

**10.** Procédé pour augmenter la stabilité d'une α-amylase de *Bacillus sp.* A 7-7 (DSM 12368) vis-à-vis d'une dimérisation et/ou d'une multimérisation médiées par des interactions électrostatiques, grâce auquel au moins un résidu d'acide aminé de la molécule de départ, situé sur la surface de la molécule et exerçant une contribution neutre ou positivement polaire ou chargée au potentiel électrostatique de la molécule, est remplacé par un résidu d'acide aminé plus fortement négativement polaire ou négativement chargé, les échanges d'acides aminés suivants étant possibles :

| Acide aminé de départ | en |
|---|---|
| Arg (R) | K, Y, C, H, G, A, V, L, I, M, F, W, P, S, T, N, Q, E ou D |
| Lys (K) | Y, C, H, G, A, V, L, I, L, F, W, P, S, T, N, Q, E ou D |
| Tyr (Y) | C, H, G, A, V, L, I, M, F, W, P, S, T, N, Q, E ou D |
| Cys (C) | H, G, A, V, L, I, M, F, W, P, S, T, N, Q, E ou D |
| His (H) | G, A, V, L, I, M, F, W, P, S, T, N, Q, E ou D |
| Gly (G) | A, V, L, I, M, F, W, P, S, T, N, Q, E ou D |
| Ala (A) | V, L, I, M, F, W, P, S, T, N, Q, E ou D |
| Val (V) | L, I, M, F, W, P, S, T, N, Q, E ou D |
| Leu (L) | I, M, F, W, P, S, T, N, Q, E ou D |
| Ile (I) | M, F, W, P, S, T, N, Q, E ou D |
| Met (M) | F, W, P, S, T, N, Q, E ou D |
| Phe (F) | W, P, S, T, N, Q, E ou D |
| Trp (W) | P, S, T, N, Q, E ou D |
| Pro (P) | S, T, N, Q, E ou D |
| Ser (S) | T, N, Q, E ou D |
| Thr (T) | N, Q, E ou D |
| Asn (N) | Q, E ou D |
| Gln (Q) | E ou D |
| Glu (E) | D |

et dans lequel le résidu d'acide aminé mentionné se trouve sur une position qui appartient à l'un des deux groupes suivants :

(A) 5, 6, 7, 19, 22, 25, 26, 28, 29, 32, 33, 35, 37, 53, 72, 75, 76, 81, 82, 83, 84, 86, 87, 90, 91, 93, 94, 95, 96, 98, 118, 136, 142, 149, 150, 151, 152, 154, 156, 158, 160, 171, 172, 181, 227, 229, 247, 251, 254, 259, 260, 281, 283, 394, 395, 399, 400, 417, 418, 419, 420, 421, et 422, ou
(B) 435, 436, 439, 444, 445, 449, 450, 452, 458, 459, 460, 461, 463, 465, 466, 471, 473, 475, 476 et 484,

chacune de ces indications correspondant à la numérotation de la protéine mature selon SEQ ID NO:2, où, pour ce qui concerne la molécule de départ, il s'agit de l'$\alpha$-amylase de *Bacillus sp.* A 7-7 (DSM 12368), dont la séquence d'acides aminés a une identité de 100 % avec la séquence d'acides aminés présentée dans SEQ ID NO:2 sur les positions +1 à 484.

**11.** Procédé selon la revendication 10, dans lequel le résidu d'acide aminé mentionné présente, avant l'échange d'acide aminé, une accessibilité d'au moins 10 %, de préférence d'au moins 20 %, d'une manière particulièrement préférée d'au moins 30 %, l'accessibilité du résidu d'acide aminé considéré étant calculée sur une échelle de 0 % à 100 %, une valeur de 0 % désignant une accessibilité telle que le résidu considéré ne soit pas accessible au solvant, et 100 % désignant l'accessibilité possible dans un pentapeptide hypothétique GGXGG.

**12.** Procédé selon la revendication 10 ou 11, dans lequel le résidu d'acide aminé mentionné se trouve dans un domaine neutre ou positivement polarisé ou chargé constitué d'au moins deux résidus d'acides aminés immédiatement voisins sur la surface.

**13.** Procédé selon l'une des revendications 10 à 12, dans lequel plusieurs des échanges d'acides aminés mentionnés sont réalisés, de préférence au moins 2, 3, 4, 5, 6, 7, 8, 9, 10, 11, 12, 13, 14, 15, 16, 17, 18, 19, 20, 21, 22, 23, 24, 25, 26, 27, 28, 29 ou 30, d'une manière particulièrement préférée de 10 à 30 et d'une manière tout particulièrement préférée de 15 à 25.

**14.** Procédé selon l'une des revendications 10 à 13, dans lequel, pour diminuer la modification de la charge totale, un ou plusieurs résidus d'acides aminés de la molécule de départ, se trouvant sur la surface de la molécule, autres que ceux du groupe (A) ou du groupe (B), sont, sur au moins l'une à au plus exactement le même nombre d'autres positions, remplacé(s) par des résidus d'acides aminés moins négativement polaires ou négativement chargés, les échanges d'acides aminés suivants étant possibles dans chaque cas :

| Acide aminé de départ | en |
|---|---|
| K, Y, C, H, G, A, V, L, I, M, F, W, P, S, T, N, Q, E ou D | Arg (R) |
| Y, C, H, G, A, V, L, I, L, F, W, P, S, T, N, Q, E ou D | Lys (K) |
| C, H, G, A, V, L, I, M, F, W, P, S, T, N, Q, E ou D | Tyr (Y) |
| H, G, A, V, L, I, M, F, W, P, S, T, N, Q, E ou D | Cys (C) |
| G, A, V, L, I, M, F, W, P, S, T, N, Q, E ou D | His (H) |
| A, V, L, I, M, F, W, P, S, T, N, Q, E ou D | Gly (G) |
| V, L, I, M, F, W, P, S, T, N, Q, E ou D | Ala (A) |
| L, I, M, F, W, P, S, T, N, Q, E ou D | Val (V) |
| I, M, F, W, P, S, T, N, Q, E ou D | Leu (L) |
| M, F, W, P, S, T, N, Q, E ou D | Ile (I) |
| F, W, P, S, T, N, Q, E ou D | Met (M) |
| W, P, S, T, N, Q, E ou D | Phe (F) |
| P, S, T, N, Q, E ou D | Trp (W) |
| S, T, N, Q, E ou D | Pro (P) |
| T, N, Q, E ou D | Ser (S) |
| N, Q, E ou D | Thr (T) |
| Q, E ou D | Asn (N) |
| E ou D | Gln (Q) |
| D | Glu (E) |

**15.** Procédé selon la revendication 14, dans lequel le ou les échange(s) réalisé (s) pour diminuer la modification de la charge totale a été/ont été réalisé(s) sur une ou plusieurs position (s) d'acides aminés qui n'appartient/n'appartiennent pas aux suivantes :

(A) 5, 6, 7, 19, 22, 25, 26, 28, 29, 32, 33, 35, 37, 53, 72, 75, 76, 81, 82, 83, 84, 86, 87, 90, 91, 93, 94, 95, 96, 98, 118, 136, 142, 149, 150, 151, 152, 154, 156, 158, 160, 171, 172, 181, 227, 229, 247, 251, 254, 259, 260, 281, 283, 394, 395, 399, 400, 417, 418, 419, 420, 421, et 422, ou
(B) 435, 436, 439, 444, 445, 449, 450, 452, 458, 459, 460, 461, 463, 465, 466, 471, 473, 475, 476 et 484, ou
(C) 176, 218, 242, 302, 310, 320, 323, 359, 368, 372, 376, 383, 385 et 404,

chacune correspondant à la numérotation de la protéine mature selon SEQ ID NO:2.

**16.** Procédé selon l'une des revendications 10 à 15, dans lequel, pour ce qui concerne la molécule de départ, il s'agit d'un variant d'α-amylase comportant, dans l'ordre croissant de préférence, 1, 2, 3, 4, 5, 6, 7, 8, 9, 10 ou 11 mutations ponctuelles additionnelles.

**17.** Procédé selon la revendication 16, dans lequel l'α-amylase introduite est un variant d'α-amylase comportant les mutations ponctuelles additionnelles sur une ou plusieurs des positions suivantes : 5, 167, 170, 177, 202, 204, 271, 330, 377, 385 et 445, selon la numérotation de la protéine mature selon SEQ ID NO:2.

**18.** Procédé selon la revendication 17, dans lequel il s'agit des mutations ponctuelles suivantes : 5A, 167R, 170P, 177L, 202L, 204V, 271D, 330D, 377R, 385S et/ou 445Q.

**19.** Acide nucléique codant pour un variant d'α-amylase selon l'une des revendications 1 à 9.

**20.** Acide nucléique selon la revendication 19, qui dérive d'un acide nucléique selon SEQ ID NO:1, ou d'un variant de ce dernier comportant, dans l'ordre croissant de préférence, 1, 2, 3, 4, 5, 6, 7, 8, 9, 10 ou 11 mutations ponctuelles additionnelles, vers des mutations qui conduisent à l'un des échanges d'acides aminés désignés dans les revendications 1 à 6.

**21.** Vecteur contenant un acide nucléique selon la revendication 19 ou 20, de préférence un vecteur de clonage ou un vecteur d'expression.

**22.** Cellule qui après modification génétique contient l'un des acides nucléiques désigné dans les revendications 19 ou 20.

**23.** Cellule selon la revendication 22, dans laquelle l'acide nucléique mentionné est une partie d'un vecteur, en particulier d'un vecteur selon la revendication 21.

**24.** Cellule selon la revendication 22 ou 23, pour ce qui concerne laquelle il s'agit d'une bactérie, en particulier d'une bactérie qui sécrète dans le milieu environnant le variant d'α-amylase formé.

**25.** Cellule selon la revendication 24, pour ce qui concerne laquelle il s'agit d'une bactérie gram-négative, en particulier une bactérie des genres *Escherichia coli, Klebsiella, Pseudomonas ou Xanthomonas,* en particulier des souches de *E. coli* K12, *E. coli* B ou *Klebsiella planticola,* et tout particulièrement de dérivés des souches *Escherichia coli* BL21 (DE3), *E. coli* RV308, *E. coli* DH5α, *E. coli* JM109, *E. coli* XL-1 ou *Klebsiella planticola* (Rf).

**26.** Cellule selon la revendication 24, pour ce qui concerne laquelle il s'agit d'une bactérie gram-positive, en particulier d'une bactérie des genres *Bacillus, Staphylococcus* ou *Corynebacterium,* tout particulièrement des espèces *Bacillus lentus, B. licheniformis, B. amyloliquefaciens, B. subtilis, B. globigii* ou *B. alcalophilus, Staphylococcus carnosus* ou *Corynebacterieum glutamicum* et, parmi ces dernières, de nouveau d'une manière tout particulièrement préférée d'un dérivé de *B. licheniformis* DSM 13.

**27.** Procédé de production biotechnologique pour la fabrication d'un variant d'α-amylase selon l'une des revendications 1 à 9.

**28.** Procédé selon la revendication 27, par utilisation d'un acide nucléique selon l'une des revendications 19 ou 20, d'une manière particulièrement préférée par utilisation d'un vecteur selon la revendication 21, d'une manière tout particulièrement préférée par utilisation d'une cellule selon l'une des revendications 22 à 26.

**29.** Produit contenant un variant d'α-amylase selon l'une des revendications 1 à 9.

**30.** Produit selon la revendication 29, pour ce qui concerne lequel il s'agit d'un produit lavant ou nettoyant

**31.** Produit lavant ou nettoyant selon la revendication 30, contenant de 0,000001 pourcent en poids à 5 % en poids, en particulier de 0,00001 à 3 % en poids du variant d'α-amylase.

**32.** Produit lavant ou nettoyant selon la revendication 30 ou 31, qui contient en outre d'autres enzymes, en particulier des enzymes hydrolytiques ou des oxydoréductases, d'une manière particulièrement préférée des amylases, protéases, lipases, cutinases, hémicellulases, cellulases, β-glucanases, oxydases, peroxydases, perhydrolases et/ou laccases supplémentaires.

**33.** Produit lavant ou nettoyant selon l'une des revendications 30 à 32, dans lequel le variant d'α-amylase est stabilisé par l'un des autres constituants du produit, et/ou voit augmentée sa contribution aux performances de lavage ou de nettoyage du produit.

**34.** Produit lavant ou nettoyant selon l'une des revendications 30 à 33, qui globalement est solide, de préférence après une étape de compactage pour au moins l'un des composants contenus, et d'une manière particulièrement préférée

est globalement compacté.

**35.** Produit lavant ou nettoyant selon l'une des revendications 30 à 34, qui est globalement liquide, en forme de gel ou pâteux, de préférence par encapsulation d'au moins l'un des composants contenus, d'une manière particulièrement préférée par encapsulation d'au moins l'une des enzymes contenues, d'une manière tout particulièrement préférée par encapsulation du variant d'α-amylase.

**36.** Procédé pour le nettoyage de textiles ou de surfaces dures, dans lequel un variant d'α-amylase selon l'une des revendications 1 à 9 est ajouté à au moins l'une des étapes du procédé, et est actif.

**37.** Procédé selon la revendication 36, dans lequel le variant d'α-amylase est utilisé par l'intermédiaire d'un produit selon l'une des revendications 30 à 35.

**38.** Procédé selon la revendication 36 ou 37, dans lequel l'α-amylase est, dans l'étape de procédé considérée, utilisée en une quantité de 0,01 mg à 400 mg par étape de procédé correspondante, de préférence de 0,02 mg à 300 mg, d'une manière particulièrement préférée de 0,03 mg à 100 mg.

**39.** Utilisation d'un variant d'α-amylase selon l'une des revendications 1 à 9 pour le nettoyage de textiles ou de surfaces dures.

**40.** Utilisation selon la revendication 39, pour laquelle le variant d'α-amylase est utilisé par l'intermédiaire d'un produit selon l'une des revendications 30 à 35.

**41.** Utilisation selon la revendication 39 ou 40, pour laquelle on utilise pour chaque utilisation, de préférence pour chaque utilisation dans un lave-vaisselle ou une machine à laver, de 0,01 mg à 400 mg du variant d'α-amylase, de préférence de 0,02 mg à 300 mg, d'une manière particulièrement préférée de 0,03 mg à 100 mg.

**42.** Utilisation d'un variant d'α-amylase selon l'une des revendications 1 à 9 pour le traitement de matières premières ou de produits intermédiaires lors de la fabrication de textiles, en particulier pour le désencollage du coton.

Figur 1

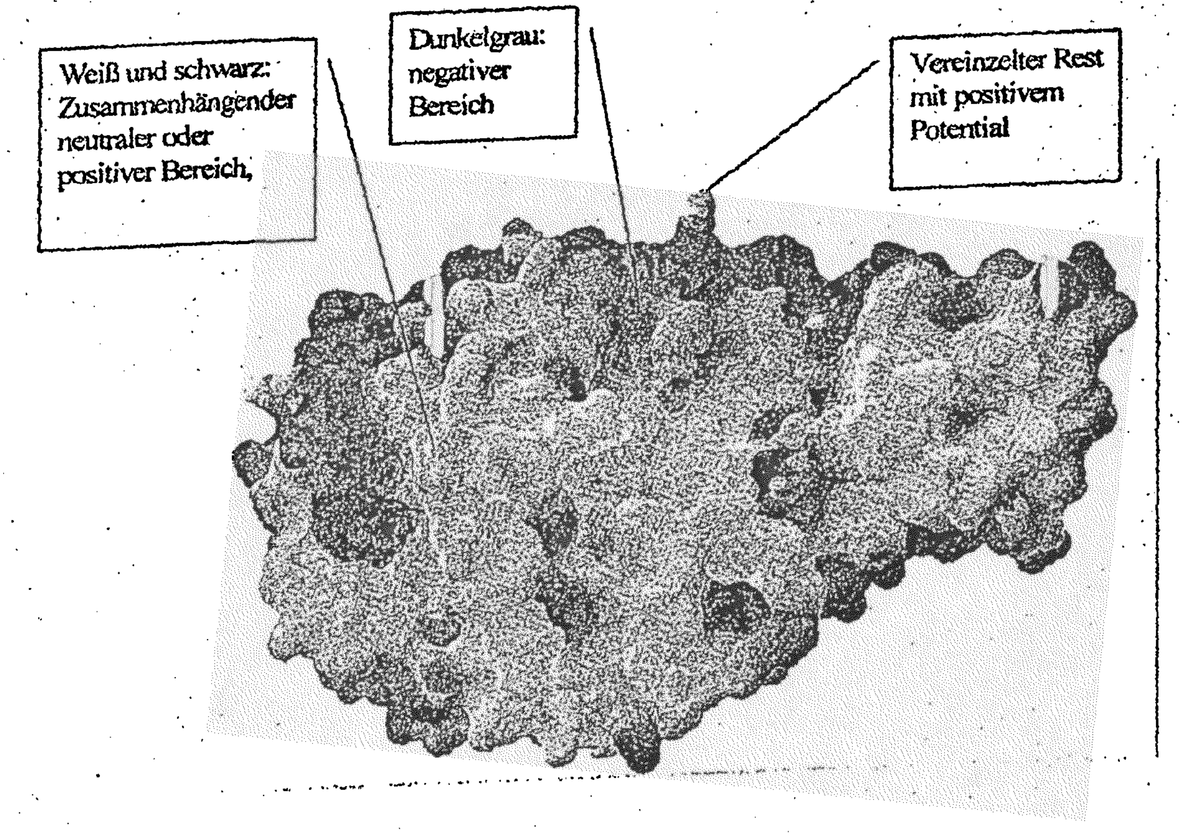
Weiß und schwarz:
Zusammenhängender
neutraler oder
positiver Bereich,
Dunkelgrau:
negativer
Bereich
Vereinzelter Rest
mit positivem
Potential

Figur 2 / Teil 1

```
                1                                                50
A 7-7     (1)   -HHNGTNGTMMQYFEWYLPNDGNHWNRLRSDASNLKDKGITAVWIPPAWK
 S707     (1)   -HHNGTNGTMMQYFEWYLPNDGNHWNRLNSDASNLKSKGITAVWIPPAWK
 LAMY     (1)   -HHNGTNGTMMQYFEWHLPNDGNHWNRLRDDAANLKSKGITAVWIPPAWK
  BAA     (1)   -----VNGTLMQYFEWYTPNDGQHWKRLQNDAEHLSDIGITAVWIPPAYK
  BLA     (1)   ---ANLNGTLMQYFEWYMPNDGQHWKRLQNDSAYLAEHGITAVWIPPAYK
 BStA     (1)   --AAPFNGTMMQYFEWYLPDDGTLWTKVANEANNLSSLGITALWLPPAYK
MK716     (1)   --AAPFNGTMMQYFEWYLPDDGTLWTKVANEANNLSSLGITALWLPPAYK
TS-23     (1)   ANTAPINETMMQYFEWDLPNDGTLWTKVKNEAANLSSLGITALWLPPAYK
  K38     (1)   --ADGLNGTMMQYYEWHLENDGQHWNRLHDDAAALSDAGITAIWIPPAYK

                51                                              100
A 7-7    (50)   GASQNDVGYGAYDLYDLGEFNQKGTVRTKYGTRNQLQAAVTALKSNGIQV
 S707    (50)   GASQNDVGYGAYDLYDLGEFNQKGTVRTKYGTRSQLQAAVTSLKNNGIQV
 LAMY    (50)   GTSQNDVGYGAYDLYDLGEFNQKGTVRTKYGTRSQLQGAVTSLKNNGIQV
  BAA    (46)   GLSQSDNGYGPYDLYDLGEFQQKGTVRTKYGTKSELQDAIGSLHSRNVQV
  BLA    (48)   GTSQADVGYGAYDLYDLGEFHQKGTVRTKYGTKGELQSAIKSLHSRDINV
 BStA    (49)   GTSRSDVGYGVYDLYDLGEFNQKGAVRTKYGTKAQYLQAIQAAHAAGMQV
MK716    (49)   GTSRSDVGYGVYDLYDLGEFNQKGAVRTKYGTKAQYLQAIQAAHAAGMQV
TS-23    (51)   GTSQSDVGYGVYDLYDLGEFNQKGTIRTKYGTKTQYIQAIQAAKAAGMQV
  K38    (49)   GNSQADVGYGAYDLYDLGEFNQKGTVRTKYGTKAQLERAIGSLKSNDINV

                101                                             150
A 7-7   (100)   YGDVVMNHKGGADATEWVRAVEVNPSNRNQEVSGDYTIEAWTKFDFPGRG
 S707   (100)   YGDVVMNHKGGADATEMVRAVEVNPNNRNQEVTGEYTIEAWTRFDFPGRG
 LAMY   (100)   YGDVVMNHKGGADGTEMVNAVEVNRSNRNQEISGEYTIEAWTKFDFPGRG
  BAA    (96)   YGDVVLNHKAGADATEDVTAVEVNPANRNQETSEEYQIKAWTDFRFPGRG
  BLA    (98)   YGDVVINHKGGADATEDVTAVEVDPADRNRVISGEHRIKAWTHFHFPGRG
 BStA    (99)   YADVVFDHKGGADGTEWVDAVEVNPSDRNQEISGTYQIQAWTKFDFPGRG
MK716    (99)   YADVVFDHKGGADGTEWVDAVEVNPSDRNQEISGTYQIQAWTKFDFPGRG
TS-23   (101)   YADVVFNHKAGADGTEFVDAVEVDPSNRNQETSGTYQIQAWTKFDFPGRG
  K38    (99)   YGDVVMNHKMGADFTEAVQAVQVNPTNRWQDISGAYTIDAWTGFDFSGRN

                151                                             200
A 7-7   (150)   NTHSNFKWRWYHFDGVDWDQSRQLQNRIYKFRGDGKGWDWEVDTENGNYD
 S707   (150)   NTHSSFKWRWYHFDGVDWDQSRRLNNRIYKFRGHGKAWDWEVDTENGNYD
 LAMY   (150)   NTHSNFKWRWYHFDGTDWDQSRQLQNKIYKFRGTGKAWDWEVDIENGNYD
  BAA   (146)   NTYSDFKWHWYHFDGADWDESRK-ISRIFKFRGEGKAWDWEVSSENGNYD
  BLA   (148)   STYSDFKWHWYHFDGTDWDESRK-LNRIYKFQG--KAWDWEVSNENGNYD
 BStA   (149)   NTYSSFKWRWYHFDGVDWDESRK-LSRIYKFRGIGKAWDWEVDTENGNYD
MK716   (149)   NTYSSFKWRWYHFDGVDWDESRK-LSRIYKFRGIGKAWDWEVDTENGNYD
TS-23   (151)   NTYSSFKWRWYHFDGTDWDESRK-LNRIYKFRSTGKAWDWEVDTENGNYD
  K38   (149)   NAYSDFKWRWFHFNGVDWDQRYQ-ENHIFRFAN--TNWNWRVDEENGNYD
```

Figur 2 / Teil 2

```
                 201                                               250
A 7-7    (200)  YLMYADIDMDHPEVVNELRNWGVWYTNTLGLDGFRIDAVKHIKYSFTRDW
 S707    (200)  YLMYADIDMDHPEVVNELRNWGVWYTNTLGLDGFRIDAVKHIKYSFTRDW
 LAMY    (200)  YLMYADIDMDHPEVINELRNWGVWYTNTLNLDGFRIDAVKHIKYSYTRDW
  BAA    (195)  YLMYADVDYDHPDVVAETKKWGIWYANELSLDGFRIDAAKHIKFSFLRDW
  BLA    (195)  YLMYADIDYDHPDVAAEIKRWGTWYANELQLDGFRLDAVKHIKFSFLRDW
 BStA    (198)  YLMYADLDMDHPEVVTELKSWGKWYVNTTNIDGFRLDAVKHIKFSFFPDW
MK716    (198)  YLMYADLDMDHPEVVTELKNWGKWYVNTTNIDGFRLDAVKHIKFSFFPDW
TS-23    (200)  YLMFADLDMDHPEVVTELKNWGTWYVNTTNIDGFRLDAVKHIKYSFFPDW
  K38    (196)  YLLGSNIDFSHPEVQDELKDWGSWFTDELDLDGYRLDAIKHIPFWYTSDW

                 251                                               300
A 7-7    (250)  LTHVRNTTGKNMFAVAEFWKNDIGAIENYLSKTNWNHSVFDVPLHYNLYN
 S707    (250)  INHVRSATGKNMFAVAEFWKNDLGAIENYLQKTNWNHSVFDVPLHYNLYN
 LAMY    (250)  LTHVRNTTGKPMFAVAEFWKNDLAAIENYLNKTSWNHSVFDVPLHYNLYN
  BAA    (245)  VQAVRQATGKEMFTVAEYWQNNAGKLENYLNKTSFNQSVFDVPLHFNLQA
  BLA    (245)  VNHVREKTGKEMFTVAEYWQNDLGALENYLNKTNFNHSVFDVPLHYQFHA
 BStA    (248)  LSDVRSQTGKPLFTVGEYWSYDINKLHNYIMKTNGTMSLFDAPLHNKFYT
MK716    (248)  LSYVRSQTGKPLFTVGEYWSYDINKLHNYITKTNGTMSLFDAPLHNKFYT
TS-23    (250)  LTYVRNQTGKNLFAVGEFWSYDVNKLHNYITKTNGSMSLFDAPLHNNFYT
  K38    (246)  VRHQRNEADQDLFVVGEYWKDDVGALEFYLDEMNWEMSLFDVPLNYNFYR

                 301                                               350
A 7-7    (300)  ASRSGGNYDMRQIFNGTVVQRHPTHAVTFVDNHDSQPEEALESFVEEWFK
 S707    (300)  ASKSGGNYDMRNIFNGTVVQRHPSHAVTFVDNHDSQPEEALESFVEEWFK
 LAMY    (300)  ASNSGGYFDMRNILNGSVVQKHPIHAVTFVDNHDSQPGEALESFVQSWFK
  BAA    (295)  ASSQGGGYDMRRLLDGTVVSRHPEKAVTFVENHDTQPGQSLESTVQTWFK
  BLA    (295)  ASTQGGGYDMRKLLNSTVVSKHPLKAVTFVDNHDTQPGQSLESTVQTWFK
 BStA    (298)  ASKSGGTFDMRTLMTNTLMKDQPTLAVTFVDNHDTEPGQALQSWVDPWFK
MK716    (298)  ASKSGGAFDMRTLMTNTLMKDQPTLAVTFVDNHDTEPGQALQSWVDPWFK
TS-23    (300)  ASKSSGYFDMRYLLNNTLMKDQPSLAVTLVDNHDTQPGQSLQSWVEPWFK
  K38    (296)  ASQQGGSYDMRNILRGSLVEAHPMHAVTFVDNHDTQPGESLESWVADWFK

                 351                                               400
A 7-7    (350)  PLAYALTLTRDQGYPSVFYGDYYGIPTHG---VPAMKSKIDPILEARQKY
 S707    (350)  PLAYALTLTREQGYPSVFYGDYYGIPTHG---VPAMRSKIDPILEARQKY
 LAMY    (350)  PLAYALILTREQGYPSVFYGDYYGIPTHG---VPSMKSKIDPLLQARQTY
  BAA    (345)  PLAYAFILTRESGYPQVFYGDMYGTKGTSPKEIPSLKDNIEPILKARKEY
  BLA    (345)  PLAYAFILTRESGYPQVFYGDMYGTKGDSQREIPALKHKIEPILKARKQY
 BStA    (348)  PLAYAFILTRQEGYPCVFYGDYYGIPQYN---IPSLKSKIDPLLIARRDY
MK716    (348)  PLAYAFILTRQEGYPCVFYGDYYGIPQYN---IPSLKSKIDPLLIARRDY
TS-23    (350)  PLAYAFILTRQEGYPCVFYGDYYGIPKYN---IPGLKSKIDPLLIARRDY
  K38    (346)  PLAYATILTREGGYPNVFYGDYYGIPNDN---ISAKKDMIDELLDARQNY
```

Figur 2 / Teil 3

```
                   401                                               450
 A 7-7   (397) AYGKQNDYLDHHNMIGWTREGNTAHPNSGLATIMSDGPGGNKWMYVGRNK
  S707   (397) AYGKQNDYLDHHNIIGWTREGNTAHPNSGLATIMSDGAGGSKWMFVGRNK
  LAMY   (397) AYGTQHDYFDHHDIIGWTREGDSSHPNSGLATIMSDGPGGNKWMYVGKHK
   BAA   (395) AYGPQHDYIDHPDVIGWTREGDSSAAKSGLAALITDGPGGSKRMYAGLKN
   BLA   (395) AYGAQHDYFDHHDIVGWTREGDSSVANSGLAALITDGPGGAKRMYVGRQN
  BStA   (395) AYGTQHDYLDHSDIIGWTREGVTEKPGSGLAALITDGPGGSKWMYVGKQH
 MK716   (395) AYGTQHDYLDHSDIIGWTREGVTEKPGSGLAALITDGPGGSKWMYVGKQH
 TS-23   (397) AYGTQRDYIDHQDIIGWTREGIDTKPNSGLAALITDGPGGSKWMYVGKKH
   K38   (393) AYGTQHDYFDHWDVVGWTREGSSSRPNSGLATIMSNGPGGSKWMYVGRQN

               451                                               500
 A 7-7   (447) AGQVWRDITGNRSGTVTINADGWGNFSVNGGSVSIWVNN-----------
  S707   (447) AGQVWSDITGNRTGTVTINADGWGNFSVNGGSVSIWVNK-----------
  LAMY   (447) AGQVWRDITGNRSGTVTINADGWGNFTVNGGAVSVWVKQ-----------
   BAA   (445) AGETWYDITGNRSDTVKIGSDGWGEFHVNDGSVSIYVQK-----------
   BLA   (445) AGETWHDITGNRSEPVVINSEGWGEFHVNGGSVSIYVQR-----------
  BStA   (445) AGKVFYDLTGNRSDTVTINSDGWGEFKVNGGSVSVWVPRKTTVSTIAWSI
 MK716   (445) AGKVFYDLTGNRSDTVTINSDGWGEFKVNGGSVSVWVPRRPVN-------
 TS-23   (447) AGKVFYDLTGNRSDTVTINADGWGEFKVNGGSVSIWVAKTSNVTFTVNNA
   K38   (443) AGQTWTDLTGNNGASVTINGDGWGEFFTNGGSVSVYVNQ-----------

               501                                               550
 A 7-7   (486) --------------------------------------------------
  S707   (486) --------------------------------------------------
  LAMY   (486) --------------------------------------------------
   BAA   (484) --------------------------------------------------
   BLA   (484) --------------------------------------------------
  BStA   (495) TTRPWTDEFVRWTEPRLVAWP-----------------------------
 MK716   (488) --------------------------------------------------
 TS-23   (497) TTTSGQNVYVVANIPELGNWNTANAIKMNPSSYPTWKATIALPQGKAIEF
   K38   (482) --------------------------------------------------

               551                                 588
 A 7-7   (486) --------------------------------------
  S707   (486) --------------------------------------
  LAMY   (486) --------------------------------------
   BAA   (484) --------------------------------------
   BLA   (484) --------------------------------------
  BStA   (516) --------------------------------------
 MK716   (488) --------------------------------------
 TS-23   (547) KFIKKDQAGNVIWESTSNRTYTVPFSSTGSYTASWNVP
   K38   (482) --------------------------------------
```

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- WO 0210356 A2
- WO 0244350 A2
- WO 03002711 A2
- WO 03054177 A2
- DE 10309803
- WO 0022103 A1
- WO 9623874 A1
- WO 9741213 A1
- WO 0060059 A2
- WO 03014358 A2
- WO 9623873 A1
- WO 0060060 A2
- WO 0166712 A2
- WO 9418314 A1
- WO 0181597 A1
- WO 2004067557 A1
- DE 10360841
- DE 102004021384
- DE 102004019528
- DE 102004020430
- WO 9963036 A
- WO 9963037 A
- DE 19616693
- DE 19616767
- EP 0525239 A
- WO 9963038 A
- WO 9963041 A
- WO 9102792 A1
- WO 9221760 A1
- WO 9523221 A1
- WO 02088340 A2
- WO 03038082 A2
- WO 03054185 A1
- WO 03056017 A2
- WO 03055974 A2
- WO 03054184 A1
- DE 10138753 A1
- WO 9629397 A1
- WO 9812307 A1
- WO 9714804 A1
- WO 9634092 A2
- WO 9906573 A1
- WO 9845398 A1
- WO 2004058955 A2
- WO 2004058961 A1
- DE 102004029475
- WO 2005056782 A2
- WO 9718287 A
- EP 0486592 A
- EP 0642576 A
- WO 9963035 A
- WO 9845396 A
- DE 102004047776


### In der Beschreibung aufgeführte Nicht-Patentliteratur


- **N. GUEX ; M. C. PEITSCH.** SWISS-Model and the Swiss-PdbViewer: An environment for comparative protein modeling. *Electrophoresis,* 1997, vol. 18, 2714-2723
- **M.L. CONNOLLY.** Solvent-Accessible Surfaces of Proteins and Nucleic Acids. *Science,* 1983, vol. 221, 709-713
- **S. A. HASSA et al.** A Critical Analysis of Continuum Electrostatics: The Screened Coulomb Potential-Implicit Solvent Model and the Study of the Alanine Dipeptide and Discrimination of Misfolded Structures of Proteins. *Proteins,* 2002, vol. 45, 47
- **E. L. MEHLER et al.** Electrostatic effects in proteins: comparison of dielectric and charge models. *Protein Eng.,* 1991, vol. 8, 903-910
- **A. JAKALIAN et al.** Fast, efficient generation of high-quality atomic charges. AM1-BCC model: II. Parameterization and validation. *J. Comput. Chem.,* 2002, vol. 16, 1623-1641
- **A. G. GORNALL ; C. S. BARDAWILL ; M.M. DAVID.** *J. Biol. Chem.,* 1948, vol. 177, 751-766
- **FRITSCH ; SAMBROOK ; MANIATIS.** Molecular cloning: a laboratory manual. Cold Spring Harbour Laboratory Press, 1989

SEQUENCE LISTING

<110> Henkel Kommanditgesellschaft auf Aktien

<120> Gegen Di- und/oder Multimerisierung stabilisierte Alpha-Amylase-Varianten, Verfahren zu deren Herstellung sowie Wasch- und Reinigungsmittel mit diesen Alpha-Amylase-Varianten

<130> H 06466 PCT

<150> DE102004047776.0
<151> 2004-10-01

<160> 2

<170> PatentIn version 3.1

<210> 1
<211> 1554
<212> DNA
<213> Bacillus sp. A 7-7 (DSM 12368)

<220>
<221> gene
<222> (1)..(1554)
<223> Alpha-Amylase

<220>
<221> CDS
<222> (1)..(1551)
<223>

<220>
<221> mat_peptide
<222> (100)..()
<223>

<400> 1

```
atg acg atg aga aaa cgt aaa aat gga tta atc agt att cta ttg gca        48
Met Thr Met Arg Lys Arg Lys Asn Gly Leu Ile Ser Ile Leu Leu Ala
            -30                 -25                 -20

ttt ttg ttg gta ctt aca tca ata cct ttt act tca gca aac gta gaa        96
Phe Leu Leu Val Leu Thr Ser Ile Pro Phe Thr Ser Ala Asn Val Glu
        -15                 -10                 -5

gca cac cat aat ggc aca aat gga aca atg atg caa tat ttt gaa tgg       144
Ala His His Asn Gly Thr Asn Gly Thr Met Met Gln Tyr Phe Glu Trp
-1  1               5                   10                  15

tat ttg cca aat gac ggt aat cat tgg aat aga tta aga tca gat gca       192
Tyr Leu Pro Asn Asp Gly Asn His Trp Asn Arg Leu Arg Ser Asp Ala
                20                  25                  30

agt aat ctt aaa gat aaa ggg att aca gcg gtt tgg att cca cct gct       240
Ser Asn Leu Lys Asp Lys Gly Ile Thr Ala Val Trp Ile Pro Pro Ala
```

```
                35                      40                      45

tgg aaa ggg gct tct caa aat gat gta ggg tat gga gcc tat gat ctg      288
Trp Lys Gly Ala Ser Gln Asn Asp Val Gly Tyr Gly Ala Tyr Asp Leu
        50                  55                  60

tat gat tta gga gaa ttc aat caa aaa gga acc gta cgt aca aag tac      336
Tyr Asp Leu Gly Glu Phe Asn Gln Lys Gly Thr Val Arg Thr Lys Tyr
    65                  70                  75

gga acc cgt aat caa tta caa gct gca gta acc gcc tta aaa agt aat      384
Gly Thr Arg Asn Gln Leu Gln Ala Ala Val Thr Ala Leu Lys Ser Asn
80                  85                  90                  95

ggt att caa gta tac gga gat gtc gta atg aat cat aag ggt gga gcg      432
Gly Ile Gln Val Tyr Gly Asp Val Val Met Asn His Lys Gly Gly Ala
                100                 105                 110

gat gcc act gag tgg gtt cga gcg gtt gaa gtg aac cca agt aat cgt      480
Asp Ala Thr Glu Trp Val Arg Ala Val Glu Val Asn Pro Ser Asn Arg
            115                 120                 125

aat caa gaa gtc tct ggt gat tat acg att gag gct tgg act aag ttt      528
Asn Gln Glu Val Ser Gly Asp Tyr Thr Ile Glu Ala Trp Thr Lys Phe
        130                 135                 140

gat ttt cct ggt cga ggt aat acc cac tct aac ttt aaa tgg aga tgg      576
Asp Phe Pro Gly Arg Gly Asn Thr His Ser Asn Phe Lys Trp Arg Trp
    145                 150                 155

tat cat ttc gat ggt gta gat tgg gat cag tca cgt caa ttg cag aat      624
Tyr His Phe Asp Gly Val Asp Trp Asp Gln Ser Arg Gln Leu Gln Asn
160                 165                 170                 175

cga atc tat aaa ttc aga gga gat gga aaa ggt tgg gac tgg gaa gtt      672
Arg Ile Tyr Lys Phe Arg Gly Asp Gly Lys Gly Trp Asp Trp Glu Val
                180                 185                 190

gat aca gag aac gga aac tat gac tat cta atg tac gcg gat att gat      720
Asp Thr Glu Asn Gly Asn Tyr Asp Tyr Leu Met Tyr Ala Asp Ile Asp
            195                 200                 205

atg gat cac cct gaa gta gtg aat gaa ctc aga aac tgg ggt gta tgg      768
Met Asp His Pro Glu Val Val Asn Glu Leu Arg Asn Trp Gly Val Trp
        210                 215                 220

tat acc aat aca ctg ggg cta gac ggg ttc aga ata gat gcg gta aaa      816
Tyr Thr Asn Thr Leu Gly Leu Asp Gly Phe Arg Ile Asp Ala Val Lys
    225                 230                 235

cat ata aaa tat agc ttt act cgt gat tgg ctt act cac gtt aga aat      864
His Ile Lys Tyr Ser Phe Thr Arg Asp Trp Leu Thr His Val Arg Asn
240                 245                 250                 255

acg aca ggt aaa aat atg ttt gca gtt gca gag ttc tgg aag aat gac      912
Thr Thr Gly Lys Asn Met Phe Ala Val Ala Glu Phe Trp Lys Asn Asp
                260                 265                 270

ata ggt gca att gaa aat tac tta agt aaa aca aat tgg aat cat tca      960
```

```
Ile Gly Ala Ile Glu Asn Tyr Leu Ser Lys Thr Asn Trp Asn His Ser
            275                 280                 285

gtt ttt gat gtg ccc ctg cat tat aac ctt tat aat gca tcg aga agt     1008
Val Phe Asp Val Pro Leu His Tyr Asn Leu Tyr Asn Ala Ser Arg Ser
        290                 295                 300

ggt ggc aat tat gat atg agg caa ata ttt aat gga aca gtt gtt cag     1056
Gly Gly Asn Tyr Asp Met Arg Gln Ile Phe Asn Gly Thr Val Val Gln
    305                 310                 315

aga cat cct aca cat gct gta aca ttt gtt gat aac cat gat tca cag     1104
Arg His Pro Thr His Ala Val Thr Phe Val Asp Asn His Asp Ser Gln
320                 325                 330                 335

ccg gaa gaa gcc cta gag tca ttt gtt gaa gag tgg ttc aaa ccg tta     1152
Pro Glu Glu Ala Leu Glu Ser Phe Val Glu Glu Trp Phe Lys Pro Leu
                340                 345                 350

gcg tat gct ctc aca cta aca cgt gat caa gga tat cct tcc gtt ttt     1200
Ala Tyr Ala Leu Thr Leu Thr Arg Asp Gln Gly Tyr Pro Ser Val Phe
            355                 360                 365

tat gga gat tat tat ggg att ccg acg cat ggt gta cca gca atg aaa     1248
Tyr Gly Asp Tyr Tyr Gly Ile Pro Thr His Gly Val Pro Ala Met Lys
        370                 375                 380

tct aag att gat ccg att tta gaa gca cgt caa aag tat gcg tac gga     1296
Ser Lys Ile Asp Pro Ile Leu Glu Ala Arg Gln Lys Tyr Ala Tyr Gly
    385                 390                 395

aaa caa aat gat tat ttg gat cac cat aat atg att ggc tgg acg cgt     1344
Lys Gln Asn Asp Tyr Leu Asp His His Asn Met Ile Gly Trp Thr Arg
400                 405                 410                 415

gaa ggt aat aca gca cat ccc aac tca gga cta gca act att atg tcg     1392
Glu Gly Asn Thr Ala His Pro Asn Ser Gly Leu Ala Thr Ile Met Ser
                420                 425                 430

gat ggc cca gga gga aat aaa tgg atg tat gtt ggg cgt aat aag gct     1440
Asp Gly Pro Gly Gly Asn Lys Trp Met Tyr Val Gly Arg Asn Lys Ala
            435                 440                 445

gga caa gtt tgg aga gat att aca gga aat cgc tca ggt acg gtg acg     1488
Gly Gln Val Trp Arg Asp Ile Thr Gly Asn Arg Ser Gly Thr Val Thr
        450                 455                 460

att aac gca gat ggg tgg ggt aat ttt tct gta aat ggt ggg tct gta     1536
Ile Asn Ala Asp Gly Trp Gly Asn Phe Ser Val Asn Gly Gly Ser Val
    465                 470                 475

tct ata tgg gta aat aat                                             1554
Ser Ile Trp Val Asn
480
```

<210> 2
<211> 517
<212> PRT
<213> Bacillus sp. A 7-7 (DSM 12368)

<400> 2

Met Thr Met Arg Lys Arg Lys Asn Gly Leu Ile Ser Ile Leu Leu Ala
-30 -25 -20

Phe Leu Leu Val Leu Thr Ser Ile Pro Phe Thr Ser Ala Asn Val Glu
-15 -10 -5

Ala His His Asn Gly Thr Asn Gly Thr Met Met Gln Tyr Phe Glu Trp
-1 1 5 10 15

Tyr Leu Pro Asn Asp Gly Asn His Trp Asn Arg Leu Arg Ser Asp Ala
20 25 30

Ser Asn Leu Lys Asp Lys Gly Ile Thr Ala Val Trp Ile Pro Pro Ala
35 40 45

Trp Lys Gly Ala Ser Gln Asn Asp Val Gly Tyr Gly Ala Tyr Asp Leu
50 55 60

Tyr Asp Leu Gly Glu Phe Asn Gln Lys Gly Thr Val Arg Thr Lys Tyr
65 70 75

Gly Thr Arg Asn Gln Leu Gln Ala Ala Val Thr Ala Leu Lys Ser Asn
80 85 90 95

Gly Ile Gln Val Tyr Gly Asp Val Val Met Asn His Lys Gly Gly Ala
100 105 110

Asp Ala Thr Glu Trp Val Arg Ala Val Glu Val Asn Pro Ser Asn Arg
115 120 125

Asn Gln Glu Val Ser Gly Asp Tyr Thr Ile Glu Ala Trp Thr Lys Phe
130 135 140

Asp Phe Pro Gly Arg Gly Asn Thr His Ser Asn Phe Lys Trp Arg Trp
145 150 155

Tyr His Phe Asp Gly Val Asp Trp Asp Gln Ser Arg Gln Leu Gln Asn
160 165 170 175

Arg Ile Tyr Lys Phe Arg Gly Asp Gly Lys Gly Trp Asp Trp Glu Val
180 185 190

Asp Thr Glu Asn Gly Asn Tyr Asp Tyr Leu Met Tyr Ala Asp Ile Asp
195 200 205

Met Asp His Pro Glu Val Val Asn Glu Leu Arg Asn Trp Gly Val Trp
210 215 220

Tyr Thr Asn Thr Leu Gly Leu Asp Gly Phe Arg Ile Asp Ala Val Lys
225 230 235

His Ile Lys Tyr Ser Phe Thr Arg Asp Trp Leu Thr His Val Arg Asn
240 245 250 255

Thr Thr Gly Lys Asn Met Phe Ala Val Ala Glu Phe Trp Lys Asn Asp
260 265 270

Ile Gly Ala Ile Glu Asn Tyr Leu Ser Lys Thr Asn Trp Asn His Ser
275 280 285

Val Phe Asp Val Pro Leu His Tyr Asn Leu Tyr Asn Ala Ser Arg Ser
290 295 300

Gly Gly Asn Tyr Asp Met Arg Gln Ile Phe Asn Gly Thr Val Val Gln
305 310 315

Arg His Pro Thr His Ala Val Thr Phe Val Asp Asn His Asp Ser Gln
320 325 330 335

Pro Glu Glu Ala Leu Glu Ser Phe Val Glu Glu Trp Phe Lys Pro Leu
340 345 350

Ala Tyr Ala Leu Thr Leu Thr Arg Asp Gln Gly Tyr Pro Ser Val Phe
355 360 365

Tyr Gly Asp Tyr Tyr Gly Ile Pro Thr His Gly Val Pro Ala Met Lys
370 375 380

Ser Lys Ile Asp Pro Ile Leu Glu Ala Arg Gln Lys Tyr Ala Tyr Gly
385 390 395

Lys Gln Asn Asp Tyr Leu Asp His His Asn Met Ile Gly Trp Thr Arg
400 405 410 415

Glu Gly Asn Thr Ala His Pro Asn Ser Gly Leu Ala Thr Ile Met Ser
420 425 430

Asp Gly Pro Gly Gly Asn Lys Trp Met Tyr Val Gly Arg Asn Lys Ala
435 440 445

Gly Gln Val Trp Arg Asp Ile Thr Gly Asn Arg Ser Gly Thr Val Thr
450 455 460

Ile Asn Ala Asp Gly Trp Gly Asn Phe Ser Val Asn Gly Gly Ser Val
465 470 475

Ser Ile Trp Val Asn
480